# EUROPEAN PATENT SPECIFICATION

(11) **EP 4 003 963 B1**
(45) Date of publication and mention of the grant of the patent: **16.08.2023**
(21) Application number: 20747384.4
(22) Date of filing: 27.07.2020
(51) Int. Cl.: C07D 231/12, C07D 403/04, A01N 43/56, A61K 31/415, A61K 31/4155

(54) **PYRAZOLE DERIVATIVES FOR CONTROLLING ARTHROPODS**
PYRAZOLDERIVATE FÜR DIE KONTROLLE VON ARTHROPODEN
DÉRIVÉS DE PYRAZOLE POUR LE CONTRÔLE DES ARTHROPODES

(30) Priority: 29.07.2019 EP 19188857
(43) Date of publication of application: 01.06.2022
(73) Proprietor: Elanco Animal Health GmbH, 40789 Monheim am Rhein (DE)
(72) Inventor: BÖRNGEN, Kirsten, 50859 Köln (DE); TURBERG, Andreas, 42781 Haan (DE); JANSSEN, Isa, Jana, Irina, 51107 Köln (DE); TELSER, Joachim, 42115 Wuppertal (DE); SCHOHE-LOOP, Rudolf, 42327 Wuppertal (DE)
(74) Representative: Cohausz & Florack
(86) International application number: PCT/EP2020/071158
(87) International publication number: WO 2021/018849

(56) References cited:
- WO-A1-2016/020441
- WO-A1-2016/174049
- WO-A1-2019/030358
- WO-A1-2019/030359
- WO-A1-2019/068819

## Description

### Introduction and Background of the Invention

The present invention relates to novel halogen-substituted compounds, to processes for their preparation and to their use for controlling animal pests, in particular arthropods and especially insects and arachnids.

### State of the Art

### Ectoparasiticides

Halogenated carboxamides with insecticidal and ectoparasiticidal activity are described in EP 1911751, WO 2012/069366, WO 2012/080376, WO 2012/107434, WO 2012/175474, WO2014/122083 and WO2015/067647. Combinations of halogenated carboxamides for the treatment of animal pests have been described in WO 2016/174052. Further reference is made to Wo2019/030359, WO2019/030358, WO2019/068819, WO2016/174049, WO2016/020441.

Besides the aforementioned classes, also other compounds with ectoparasiticidal activity are well-known in the art. Common to these molecules of a number of different molecular modes of action is that none of them exerts a broad arthropodicidal effect over a period of more than three months. Commercial use of prolonged broad arthropodicidal activity can only be found in constant release approaches as used in collars (e.g. deltamethrin in Scalibor^{®} or flumethrin/imidacloprid in Seresto^{®}). A convenient one-time parenteral application with broad arthropod control is not marketed or shown to work in practice to date. Reasons for failure in that area can be found in short halflife, low solubility, low bioavailability, insufficient stability, incomplete arthropod parasite spectrum, local and systemic side effects. A multitude of these parameters need to be improved to lead to a successful approach to fulfil the need of a systemic treatment of ectoparasites with an extended term of efficacy.

### Prodrugs

In cases where the efficacy of a drug is limited by its physicochemical properties, a prodrug concept may be used. Prodrugs are defined as bioreversible derivatives of the corresponding parent drugs. This means that the prodrug carries a cleavable group, a so called pro-moiety. This group facilitates administration, absorption into the body and distribution in the treated animal or human.The pro-moiety is cleaved by biological or chemical transformations in the patient's body liberating the parent drug once the prodrug has been absorbed. A general overview of prodrug concepts can be found, for instance, in a review article by J. Rautio, H. Kumpulainen, T. Heimbach, R. Oliyai, D. Oh, T. Järvinen, J. Savolainen, Nature Reviews Drug Discovery 2008, 255-270. In many cases, the administration of a drug is limited by its poor aqueous solubility which can impact both oral and, even more so, parenteral administration. Prodrugs have been described for both oral and intravenous applications of drugs whereas little is known about prodrugs for subcutaneous use (see V.J. Stella, K.W. Nti-Addae, Advanced Drug Delivery Reviews 2007, 59, 677-694). Many prodrugs use an attachment group of the parent drug to link the pro-moiety via a linker group and optionally spacer groups. Attachment points are usually functional groups of the parent drug which allow the bioreversible chemical modification, for instance hydroxyl groups, carboxylic acid groups, amino groups, amides or other. A recent example of carbamate linkers attached to an amide group can be found in C. Liu, J. Lin, G. Everlof, C. Gesenberg, H. Zhang, P.H. Marathe, M. Malley, M.A. Gallella, M. McKinnon, J.H. Dodd, J.C. Barrish, G.L. Schieven, K. Leftheris, Bioorg. Med. Chem. Lett. 2013, 23, 3028-3033.

Besides carbamates, also carbonate esters are occasionally described as linkers in the prodrug literature, e.g. for the modification of a pyridyl group (see E. Binderup, F. Björkling, P.V. Hjranaa, S. Latini, B. Balther, M. Carlsen, L. Binderup, Bioorg. Med. Chem. Lett. 2005, 15, 2491-2494). *N,O*-acetals have been used to join carboxylic acid groups onto some heterocycles, e.g. hydantoins (see S.A..Varia, S. Schuller, K.B. Sloan, V. Stella, J.Pharm. Sci 1984, 73, 1068-1073)., pyrimidinediones (see WO 2018/121678), pyrrolidinediones (see, WO 2001/027106, WO 2019/079721, piperidinedione (WO 9737988), 3-(pyrrol-2-ylmethylidene)-2-indolinones (WO 2001/090103), fluoroxindoles (US 2005/0203089, WO 2011/031979).

WO 2020/007704 (PCT application PCT/EP2019/067165 claiming priority from European patent application no. 18181950.9) describes prodrugs of halogen-substituted compounds and their use for controlling animal pests, in particular arthropods and especially insects and arachnids.

It was an object of the present invention to provide novel compounds with high insecticidal and ectoparasiticidal systemic activity and enhanced solubility. A further object of the present invention was to provide novel compounds with high ectoparasiticidal, in particular insecticidal and/or acaricidal, systemic activity and improved bioavailability compared to known compounds. It was a further object of the present invention to provide novel compounds with high ectoparasiticidal, in particular insecticidal and/or acaricidal, systemic activity having sufficient stability in formulations for pharmaceutical administration. It was a further object of the present invention to provide novel compounds with high insecticidal and ectoparasiticidal systemic activity with optimum balance of stability in formulations for pharmaceutical administration and systemic release properties to release the active principle of the novel compounds under systemic conditions in the body. In a further object of the present invention the systemic release of the active principle of the novel compounds should occur at a suitable point of time or over a suitable time period to achieve improved systemic activity in the treatment. A further object of the present invention was to provide novel compounds with high insecticidal and ectoparasiticidal systemic activity and enhanced solubility in formulations for use as a medicament for subcutaneous administration. The novel compounds should also not be toxic or release toxic groups upon administration.

The inventors of the present invention now surprisingly found that with the new compounds of the present invention the disadvantages of the prior art can be avoided and the objects described above can be solved.

Accordingly, the new compounds further exhibit and can therefore be employed particularly well in the animal health sector.

### Detailed Description of the Invention

The present invention can be described in particular by the following embodiments:
[1] In a first aspect the invention relates to compounds of formula (I) 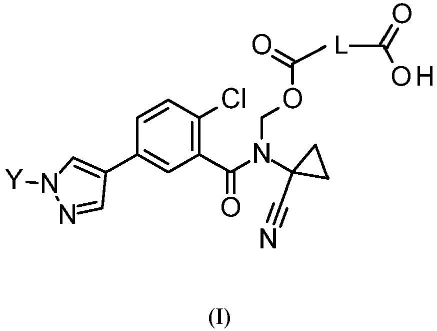 wherein
   L is linear C₁-C₄ alkanediyl, C₂-C₄ alkenediyl or C₂-C₄ alkynediyl, each of which may be substituted with one or more groups independently selected from halogen, C₁-C₄ alkyl and C₃-C₆ cycloalkyl, wherein two C₁-C₄-alkyl substituents may form a ring together with the carbon atom to which they are bonded; or
   a moiety (CH₂)ₙ-X-(CH₂)ₘ wherein
   n and m are independently 0, 1 or 2 and
   X is selected from a group X¹, X², X³ or X⁴, wherein
   X¹ is C₃-C₇-cycloalkanediyl, which is optionally substituted with 1 to 3 substituents selected from halogen, cyano, and C₁-C₄ alkyl, wherein (CH₂)ₙ- and (CH₂)ₘ- may be attached either to the same or to different carbon atoms of X¹; or
   X² is phenylene, which is optionally substituted with 1 to 4 substituents selected from halogen, cyano, and C₁-C₄ alkyl; or
   X³ is C₅-C₆-heteroarenediyl, which is optionally substituted with 1, 2 or 3 substituents selected from halogen, cyano, and C₁-C₄ alkyl; or
   X⁴ is C₅-C₈ bicycloalkanediyl, which is optionally substituted with 1 to 4 substituents selected from halogen, cyano, and C₁-C₄ alkyl wherein (CH₂)ₙ- and (CH₂)ₘ- may be attached either to the same or to different carbon atoms of X⁴; and
   Y is selected from a group (T¹) 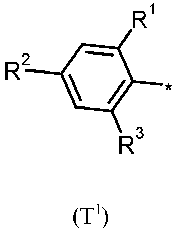
   wherein
   R¹, R² and R³ are each independently selected from hydrogen, halogen, cyano, nitro, linear or branched C₁-C₆-alkyl, C₃-C₆-cycloalkyl, C₁-C₆-alkoxy, halogen-substituted linear or branched C₁-C₆-alkyl, halogen-substituted C₁-C₆-alkoxy, halogen substituted C₃-C₆-cycloalkyl, C₁-C₆-alkylsulphanyl, C₁-C₆-alkylsulphinyl, C₁-C₆-alkylsulphonyl, *N-*C₁-C₆-alkylamino, *N,N-*di-C₁-C₆-alkylamino, *N-*C₁-C₃-alkoxy-C₁-C₄-alkylamino and 1-pyrrolidinyl
      or from a group (T²) 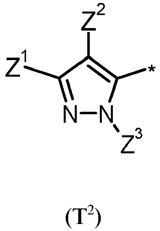
   wherein
   Z¹ and Z² are each independently selected from hydrogen, halogen, cyano, nitro, linear or branched C₁-C₆-alkyl, C₃-C₆-cycloalkyl, C₁-C₆-alkoxy, C₁-C₆-alkylcarbonyl, halogen-substituted linear or branched C₁-C₆-alkyl, halogen-substituted C₁-C₆-alkoxy, halogen substituted C₃-C₆-cycloalkyl, C₁-C₆-alkylsulphanyl, C₁-C₆-alkylsulphinyl, C₁-C₆-alkylsulphonyl, *N-*C₁-C₆-alkylamino, *N,N-*di-C₁*-*C₆-alkylamino, *N*-C₁-C₃-alkoxy-C₁-C₄-alkylamino and 1-pyrrolidinyl; and
   Z³ represents hydrogen, linear or branched C₁-C₆-alkyl, C₃-C₆-cycloalkyl, C₂-C₆-alkenyl, C₂-C₆-alkynyl, aryl or hetaryl, each of which may be substituted with 1 to 5 substituents selected from hydroxy, halogen, cyano, nitro, amino, C₁-C₃-alkyl, C₁-C₃-alkoxy, hydroxycarbonyl, alkoxycarbonyl, alkylcarbamoyl, cycloalkylcarbamoyl and phenyl;
   and to the salts thereof.
[2] In a further aspect the invention relates to compounds of formula (I) as defined in [1], wherein
   L is linear C₁-C₄ alkanediyl or C₂-C₄ alkenediyl, each of which may be substituted with one or more groups independently selected from halogen, C₁-C₄ alkyl and C₃-C₆ cycloalkyl, wherein two C₁-C₄-alkyl substituents may form a ring together with the carbon atom to which they are bonded; or
   a moiety (CH₂)ₙ-X-(CH₂)ₘ wherein
   n and m are independently 0, 1 or 2 and
   X is selected from a group X¹, X², X³ or X⁴ as defined in [1]; and
   Y is selected from a group (T¹) or (T²) as defined in [1];
   and the salts thereof.
[3] In a further aspect the invention relates to compounds of formula (I) as defined in [1] or [2], wherein
   L is linear C₁-C₄ alkanediyl or C₂-C₄ alkenediyl, each of which may be substituted with one or more groups independently selected from halogen, C₁-C₄ alkyl and C₃-C₆ cycloalkyl, wherein two C₁-C₄-alkyl substituents may form a ring together with the carbon atom to which they are bonded; or
   a moiety (CH₂)ₙ-X-(CH₂)ₘ wherein
   n and m are independently 0, 1 or 2 and
   X is selected from a group X¹ or X² as defined in [1]; and
   Y is selected from a group (T¹) or (T²) as defined in [1];
   and the salts thereof.
[4] In a further aspect the invention relates to compounds of formula (I) as defined in [1] to [3], wherein
   Y is selected from a group (T¹) 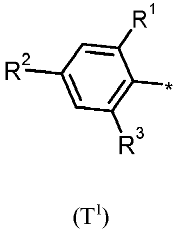 wherein
   R¹, R² and R³ are each independently selected from hydrogen, halogen, linear or branched C₁-C₃-alkyl, C₃-C₆-cycloalkyl, C₁-C₃-alkoxy, linear or branched halogen-substituted C₁-C₃-alkyl, halogen-substituted C₁-C₃-alkoxy, halogen substituted C₃-C₆-cycloalkyl, and 1-pyrrolidinyl,
   preferably R¹, R² and R³ are each independently selected from halogen, linear or branched halogen-substituted C₁-C₃-alkyl, and halogen-substituted C₁-C₃-alkoxy;
      or from a group (T²) 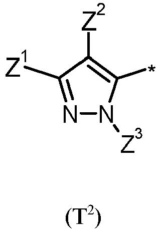
   wherein
   Z¹ represents linear or branched C₁-C₃-alkyl, C₃-C₆-cycloalkyl, C₁-C₃-alkoxy,
   which may independently of one another be substituted with 1 to 5 substituents selected from hydroxy, halogen, cyano, nitro, C₁-C₃-alkyl, and C₁-C₃-alkoxy;
   preferably Z¹ represents linear or branched C₁-C₃-alkyl or C₃-C₆-cycloalkyl, which may independently of one another be substituted with 1 to 5 halogen substituents;
   Z² represents halogen, cyano, nitro, amino, or linear or branched C₁-C₆-alkyl, C₁-C₆-alkylcarbonyl, C₁-C₆-alkylsulphanyl, C₁-C₆-alkylsulphinyl, C₁-C₆-alkylsulphonyl, which may independently of one another be substituted with 1 to 5 substituents selected from hydroxy, halogen, cyano, nitro, C₁-C₃-alkyl, and C₁-C₃-alkoxy;
   preferably Z² represents linear or branched C₁-C₃-alkyl, which may be substituted with 1 to 5 halogen substituents, preferably with 1 to 3 halogen substituents, more preferably trifluoromethyl or Z² represents nitro, methylsulphanyl, methylsulphinyl, methylsulphonyl, fluorine, chlorine, bromine, iodine; and
   Z³ represents hydrogen or linear or branched C₁-C₆-alkyl, C₃-C₆-cycloalkyl, C₂-C₆-alkenyl, C₂-C₆-alkynyl, aryl or hetaryl, which may independently of one another be substituted with 1 to 5 substituents selected from hydroxy, halogen, cyano, nitro, C₁-C₃-alkyl, and C₁-C₃-alkoxy;
   preferably Z³ represents hydrogen or linear or branched C₁-C₆-alkyl which may be substituted with 1 to 5 substituents selected from hydroxy, halogen, C₁-C₃-alkyl, and C₁-C₃-alkoxy;
   and the salts thereof.
[5] In a further aspect the invention relates to compounds of formula (I) as defined in [1] to [4], wherein
   Y is selected from a group (T¹) 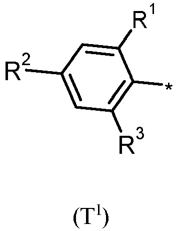 wherein
   R¹ is halogen, preferably fluorine, bromine or chlorine, more preferably chlorine;
   R² is linear or branched C₁-C₃-alkyl substituted with 1 to 7 halogen, preferably linear or branched C₁-C₃-alkyl substituted with 1 to 7 fluorine, more preferably CF₃, C₂F₅ or C₃F₇; and
   R³ is C₁-C₃-alkoxy substituted with 1 to 3 halogen, preferably C₁-C₃-alkoxy substituted with 1 to 3 fluorine, more preferably OCF₃, OC₂F₅ or OC₃F₇;
   or from a group (T²) 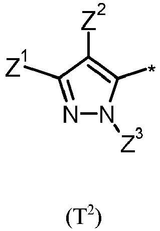
   wherein
   Z¹ represents linear or branched C₁-C₃-alkyl or C₃-C₆-cycloalkyl, substituted with 1 to 5 halogen substituents, preferably trifluoromethyl, 1-chlorocyclopropyl, 1-fluorocyclopropyl or pentafluoroethyl, more preferably trifluoromethyl or pentafluoroethyl;
   Z² represents linear or branched C₁-C₃-alkyl substituted with 1 to 3 halogen substituents, nitro, methylsulphanyl, methylsulphinyl, methylsulphonyl, fluorine, chlorine, bromine, or iodine;
   preferably Z² represents linear or branched C₁-C₃-alkyl substituted with 1 to 3 fluorine, more preferably trifluoromethyl; and
   Z³ represents hydrogen or linear or branched C₁-C₆-alkyl, preferably linear or branched C₁-C₆-alkyl, more preferably hydrogen, methyl, ethyl, or n-propyl;
   and the salts thereof.
[6] In a further aspect the invention relates to compounds of formula (I) as defined in any one of the preceding aspects [1] to [5], wherein
   L is linear C₂-C₄ alkanediyl or C₂-C₄ alkenediyl, each of which may be substituted with one or more groups independently selected from halogen and C₁-C₄ alkyl;
   and the salts thereof.
[7] In a further aspect the invention relates to compounds of formula (I) as defined in any one of the preceding aspects [1] to [6], wherein.
   L is a moiety (CH₂)ₙ-X¹-(CH₂)ₘ wherein
   n and m are independently 0, 1 or 2 and
   X¹ is C₃-C₆-cycloalkanediyl, which is optionally substituted with 1 to 3 substituents selected from halogen, cyano, and C₁-C₄ alkyl, wherein (CH₂)ₙ- and (CH₂)ₘ- may be attached either to the same or to different carbon atoms of X¹;
   and the salts thereof.
[8] In a further aspect the invention relates to compounds of formula (I) as defined in any one of the preceding aspects [1] to [7], wherein
   L is a moiety (CH₂)ₙ-X²-(CH₂)ₘ wherein
   n and m are independently 0, 1 or 2 and
   X² is phenylene which is optionally substituted with 1 to 4 substituents selected from halogen, cyano, and C₁-C₄ alkyl;
   and the salts thereof.
[9] In a further aspect the invention relates to compounds of formula (I) as defined in any one of the preceding aspects [1] to [8], wherein
   L is linear C₂-C₃ alkanediyl, which may be substituted with one or more groups independently selected from halogen, C₁-C₄ alkyl and C₃-C₆ cycloalkyl, wherein two C₁-C₄-alkyl substituents may form a ring together with the carbon atom to which they are bonded
   and the salts thereof.
[10] In a further aspect the invention relates to compounds of formula (I) as defined in any one of the preceding aspects [1] to [8], wherein
   L is linear C₂-C₃ alkanediyl, which may be substituted with one or two C₁-C₄ alkyl groups, preferably with one or two methyl groups;
   and the salts thereof.
[11] In a further aspect the invention relates to compounds of formula (I) as defined in any one of the preceding aspects [1] to [8], wherein
   L is linear C₂-C₃ alkenediyl, preferably ethenediyl, which may be substituted with one or more groups independently selected from halogen, C₁-C₄ alkyl and C₃-C₆ cycloalkyl, wherein two C₁-C₄-alkyl substituents may form a ring together with the carbon atom to which they are bonded
   and the salts thereof.
[12] In a further aspect the invention relates to compounds of formula (I) as defined in any one of the preceding aspects [1] to [8] and [11], wherein
   L is linear C₂-C₃ alkenediyl, preferably ethenediyl;
   and the salts thereof.
[13] In a further aspect the invention relates to compounds of formula (I) as defined in any one of the preceding aspects [1] to [8], wherein
   L is a moiety (CH₂)ₙ-X-(CH₂)ₘ wherein n and m are independently 0 or 1;
   and the salts thereof.
[14] In a further aspect the invention relates to compounds of formula (I) as defined in any one of the preceding aspects [1] to [8] and [13], wherein
   L is a moiety (CH₂)ₙ-X¹-(CH₂)ₘ
   wherein n and m are independently 0, 1 or 2; and
   X¹ is C₃- or C₆-cycloalkanediyl, which is optionally substituted with 1 to 3 substituents selected from halogen, cyano, and C₁-C₄ alkyl, wherein (CH₂)ₙ- and (CH₂)ₘ- may be attached either to the same or to different carbon atoms of X¹;
   and the salts thereof.
[15] In a further aspect the invention relates to compounds of formula (I) as defined in any one of the preceding aspects [1] to [8] and [13], wherein
   L is a moiety (CH₂)ₙ-X²-(CH₂)ₘ
   wherein n and m are independently 0, 1 or 2; and
   X² is phenylene, which is optionally substituted with 1, 2 or 3 substituents selected from halogen, cyano, and C₁-C₄ alkyl;
   and the salts thereof.
[16] In a further aspect the invention relates to compounds of formula (I) as defined in any one of the preceding aspects [1] to [15], wherein
   Y is selected from a group (T¹) 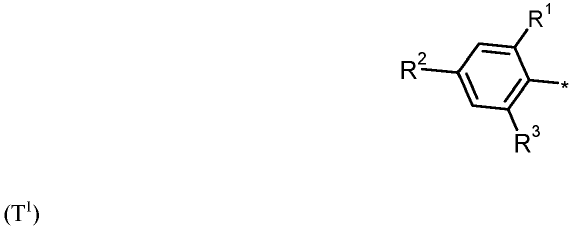
   wherein
   R¹, R² and R³ are each independently selected from hydrogen, halogen, linear or branched C₁-C₆-alkyl, C₁-C₆-alkoxy, halogen-substituted linear or branched C₁-C₆-alkyl, and halogen-substituted C₁-C₆-alkoxy;
   preferably
   R¹ is halogen, preferably fluorine, bromine or chlorine, more preferably chlorine;
   R² is linear or branched C₁-C₃-alkyl substituted with 1 to 7 halogen, preferably linear or branched C₁-C₃-alkyl substituted with 1 to 7 fluorine, more preferably CF₃, C₂F₅ or C₃F₇; and
   R³ is C₁-C₃-alkoxy substituted with 1 to 3 halogen, preferably C₁-C₃-alkoxy substituted with 1 to 3 fluorine, more preferably OCF₃, OC₂F₅ or OC₃F₇and the salts thereof.
[17] In a further aspect the invention relates to compounds of formula (I) as defined in any one of the preceding aspects [1] to [15], wherein
   Y is selected from a group (T²) 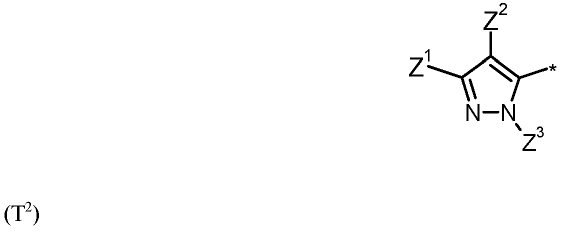
   wherein
   Z¹ and Z² are each independently selected from hydrogen, halogen, linear or branched C₁-C₆-alkyl, and halogen-substituted linear or branched C₁-C₆-alkyl; and
   Z³ represents hydrogen and linear or branched C₁-C₆-alkyl, preferably methyl, ethyl, or n-propyl;
   preferably
   Z¹ represents linear or branched C₁-C₃-alkyl or C₃-C₆-cycloalkyl, substituted with 1 to 5 halogen substituents, preferably trifluoromethyl, 1-chlorocyclopropyl, 1-fluorocyclopropyl or pentafluoroethyl, more preferably trifluoromethyl or pentafluoroethyl;
   Z² represents linear or branched C₁-C₃-alkyl substituted with 1 to 3 halogen substituents, nitro, methylsulphanyl, methylsulphinyl, methylsulphonyl, fluorine, chlorine, bromine, or iodine;
   more preferably Z² represents linear or branched C₁-C₃-alkyl substituted with 1 to 3 fluorine, more preferably trifluoromethyl; and
   Z³ represents hydrogen or linear or branched C₁-C₆-alkyl, preferably linear or branched C₁-C₆-alkyl, more preferably hydrogen, methyl, ethyl, or n-propy
   and the salts thereof.
[18] In a further aspect the invention relates to compounds of formula (I) as defined in any one of the preceding aspects [1] to [17]
   wherein
   Y is selected from a group (T¹) 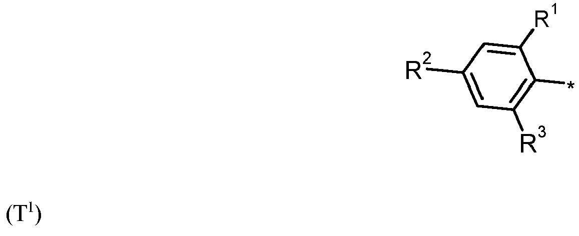
   wherein
      - R¹: is chlorine;
      - R²: is CF₃, C₂F₅ or C₃F₇ and
      - R³: is OCF₃, OC₂F₅ or OC₃F₇;
   or from a group (T²) 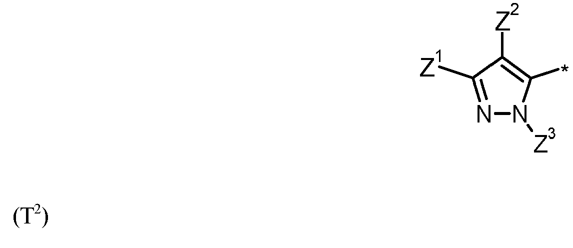
   wherein
      - Z¹: represents trifluoromethyl or pentafluoroethyl;
      - Z²: represents trifluoromethyl; and
      - Z³: represents hydrogen, methyl, ethyl, or n-propyl;
   and the salts thereof.
[19] In a further aspect the invention relates to compounds of formula (I) as defined in [1] to [18], wherein the group (T¹) is represented by one of the following groups (T1-1), (T1-2) or (T2-1): 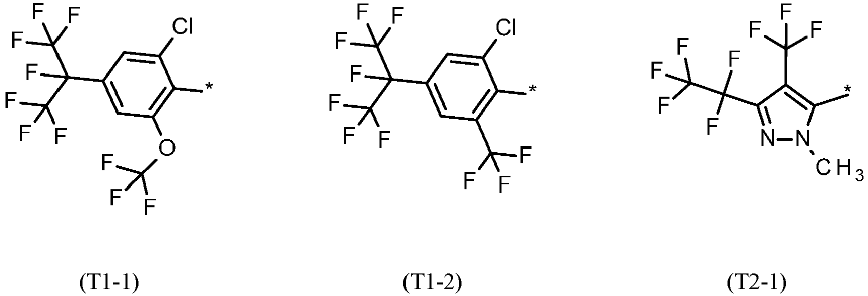 and the salts thereof.
[20] In a further aspect the invention relates to compounds of formula (I) as defined in any one of the preceding aspects [1] to [19],
   wherein (T¹) is represented by the following group (T1-1): 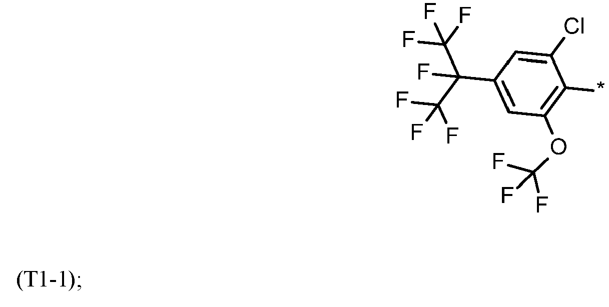
   and the salts thereof.
[21] In a further aspect the invention relates to compounds of formula (I) as defined in any one of the preceding aspects [1] to [19],
   wherein (T¹) is represented by the following group (T1-2): 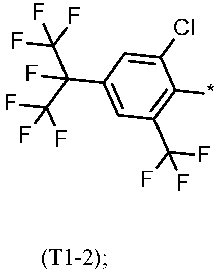
   and the salts thereof.
[22] In a further aspect the invention relates to compounds of formula (I) as defined in any one of the preceding aspects [1] to [19],
   wherein (T²) is represented by the following group (T2-1): 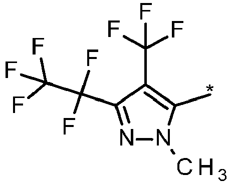 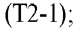
   and the salts thereof.
[23] In a further aspect the invention relates to compounds of formula (I) as defined in any one of the preceding aspects [1] to [22], which are in the form of salts, solvates, N-oxides and tautomeric forms thereof.
[24] In a further aspect the invention relates to compounds of formula (I) as defined in any one of the preceding aspects [1] to [23], which are selected from
   2-({[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}carbonyl)cyclopropane-1-carboxylic acid;
   4-{[{2-Chloro-5-[2'-methyl-5'-(pentafluoroethyl)-4'-(trifluoromethyl)-2'H-[1,3'-bipyrazol]-4-yl]benzoyl} (1-cyanocyclopropyl)amino]methoxy} -2,2-dimethyl-4-oxobutanoic acid;
   4-({[(2-Chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}carbonyl)benzoic acid;
   5-{[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}-5-oxopentanoic acid;
   1-({[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}carbonyl)cyclopropane-1-carboxylic acid;
   4-{[{2-Chloro-5-[2'-methyl-5'-(pentafluoroethyl)-4'-(trifluoromethyl)-2'H-[1,3'-bipyrazol]-4-yl]benzoyl} (1-cyanocyclopropyl)amino]methoxy} -4-oxobutanoic acid;
   (2E)-4-{[{2-Chloro-5-[2'-methyl-5'-(pentafluoroethyl)-4'-(trifluoromethyl)-2'H-[1,3'-bipyrazol]-4-yl]benzoyl} (1-cyanocyclopropyl)amino]methoxy} -4-oxobut-2-enoic acid;
   4-{ [(2-Chloro-5-{ 1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-4-oxobutanoic acid;
   5-{[(2-Chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-5-oxopentanoic acid;
   4-({[{2-Chloro-5-[2'-methyl-5'-(pentafluoroethyl)-4'-(trifluoromethyl)-2'H-[1,3'-bipyrazol]-4-yl]benzoyl} (1-cyanocyclopropyl)amino]methoxy}carbonyl)benzoic acid;
   (trans)-4-({ [(2-Chloro-5-{ 1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}carbonyl)cyclohexane-1-carboxylic acid;
   (trans)-4-({[{2-Chloro-5-[2'-methyl-5'-(pentafluoroethyl)-4'-(trifluoromethyl)-2'H-[l,3'-bipyrazol]-4-yl]benzoyl}(1-cyanocyclopropyl)amino]methoxy}carbonyl)cyclohexane-1-carboxylic acid;
   (2E)-4-{[(2-Chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-4-oxobut-2-enoic acid;
   4-1[(2-Chloro-5-11-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-2,2-dimethyl-4-oxobutanoic acid; and
   4-(2-{ [(2-Chloro-5-{ 1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-2-oxoethyl)benzoic acid;
   and the salts solvates, N-oxides and tautomeric forms thereof.
[25] In a further aspect the invention relates to compounds of formula (I) as defined in any one of the preceding aspects [1] to [24], which are for use as medicaments.
[26] In a further aspect the invention relates to pharmaceutical compositions comprising at least one compound according to any of the preceding aspects [1] to [25].
[27] In a further aspect the invention relates to pharmaceutical compositions according to aspect [26], comprising at least one further component selected from auxiliaries, excipients and/or solvents.
[28] In a further aspect the invention relates to pharmaceutical compositions according to aspect [26] or [27], comprising at least one additional pharmaceutically active agent.
[29] In a further aspect the invention relates to pharmaceutical compositions according to aspect [28], wherein the at least one additional pharmaceutically active agent is selected from the group of active agents with ectoparasiticidal activity, in particular with insecticidal and/or acaricidal activity, or from the group of antigens for vaccination purposes.
[30] In a further aspect the invention relates to pharmaceutical compositions according to any of the preceding aspects [26] to [29], which are in the form of an injectable formulation.
[31] In a further aspect the invention relates to pharmaceutical compositions according to any of the preceding aspects [26] to [29], which are in the form of a formulation for oral administration.
[32] In a further aspect the invention relates to the compounds or the pharmaceutical compositions according to any of the preceding aspects for use as a medicament for subcutaneous application.
[33] In a further aspect the invention relates to the compounds or the pharmaceutical compositions according to any of the preceding aspects for oral application.
[34] In a further aspect the invention relates to the compounds or the pharmaceutical compositions according to any of the preceding aspects for use in treating animals.
[35] In a further aspect the invention relates to the compounds or the pharmaceutical compositions for use according to to aspect [34], wherein the animals to be treated are selected from companion animals.
[36] In a further aspect the invention relates to the compounds or the pharmaceutical compositions for use according to to aspect [34] or [35], wherein the companion animals are selected from cats and dogs, preferably from dogs.
[37] In a further aspect the invention relates to compounds or the pharmaceutical compositions according to any one of the preceding aspects for use in controlling insects and arachnids.
[38] In a further aspect the invention relates to the use according to aspect [37], wherein the arachnids are selected from the group of Chelicerata.
[39] In a further aspect the invention relates to the use according to aspect [37] or [38], wherein the insects are selected from the group consisting of lice, mosquitoes, flies and fleas and arachnids are selected from acari, in particular from the group consisting of ticks, and mites.
[40] In a further aspect the invention relates to the use of the compounds as defined in any one of the preceding aspects for preparing pharmaceutical compositions for controlling parasites on animals.
[41] In a further aspect the invention relates to the use of the compounds or the pharmaceutical compositions according to any one of the preceding aspects with treatment intervals of 3 months to two years, preferably 4 months to one year.
[42] In a further aspect the invention relates to the use of the compounds or the pharmaceutical compositions according to aspect [41], wherein the treatment intervals are 6 months to one year, preferably 9 months to one year.
[43] In a further aspect the invention relates to the use of the compounds or the pharmaceutical compositions according to any of the preceding aspects, wherein the total amount of the compounds as defined in any one of the preceding aspects to be administered is in the range of from 0.01 to 200 mg/kg body weight per application, preferably in the range of from 0.1 to 100 mg/kg body weight per application, more preferably of from 0.5 to 75 mg/kg body weight per application, more preferably in the range of from 1.0 to 50 mg/kg body weight per application, most preferably in the range of from 2.0 to 20 mg/kg body weight per application.
[44] In a further aspect the invention relates to a process for preparing the compounds according to any of the preceding aspects comprising the step of reacting a compound (A) 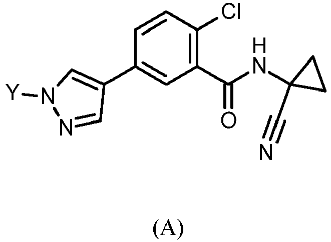 with a group (B) 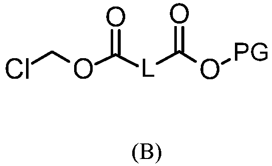 wherein
   Y and L have the meaning as defined in any one of the preceding aspects and wherein PG represents a protecting group or hydrogen to form the compounds according to formula (I),
   and wherein in cases wherein PG is not hydrogen, deprotection is carried out to form the compounds (I).
[45] In a further aspect the invention relates to the process according to aspect [44] further comprising the preliminary step of preparing the group (B) by reacting a compound (b) to form compound (B) 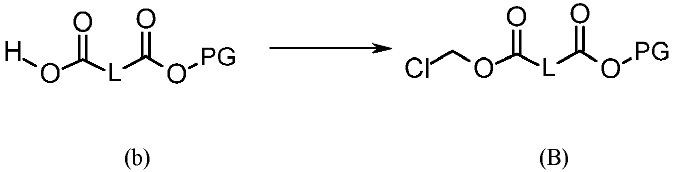
[46] In a further aspect the invention relates to intermediate compounds according to formula (B-1) and (B-2), 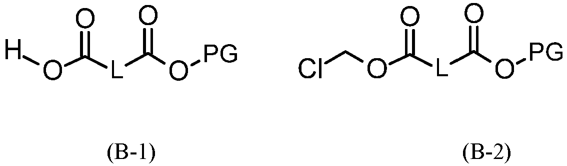 wherein
   - L: has the meaning as defined in any one of the preceding aspects and
   - PG: represents a tert-butyl group.
[47] In a further aspect the invention relates to intermediate compounds according to formula (C), 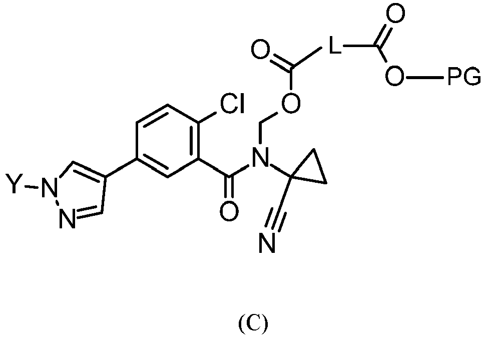 wherein Y and L have the meaning as defined in any one of the preceding aspects and wherein PG represents a tert-butyl group.
   In a further aspect the invention relates to intermediates of formula (A-3) 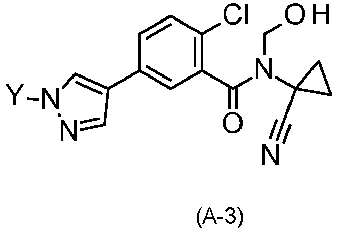 wherein Y has the meaning as defined in any one of the preceding aspects.
[48] In a further aspect the invention relates to intermediate compounds selected from those described in the Examples *infra,* such as in particular intermediate compounds (B-1) selected from intermediate compounds 20A; intermediate compounds (B-2) selected from intermediate compounds 1A, 3A, 5A, 7A, 9A, 11A, 13A, 15A and 21A *infra*; intermediate compounds (C) selected from intermediate compounds 2A, 4A, 6A, 8A, 10A, 12A, 14A, 16A, 17A, 18A, 22A, 23A, 24A, 25A, 26A and 28A *infra;* or intermediate compounds (A-3) selected from intermediate compounds 27A *infra.*

### Definitions

"Arachnids" are a class (Arachnida) of joint-legged invertebrate animals (arthropods), in the subphylum Chelicerata. A preferred subclass of the arachnids are acari (or acarina) which comprise in particular mites and ticks.

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3, 4 or 5, in particular 1, 2 or 3.

As used herein, the term "one or more", e.g. in the definition of the substituents of the compounds of general formula (I) of the present invention, means "1, 2, 3, 4 or 5, particularly 1, 2, 3 or 4, more particularly 1, 2 or 3, even more particularly 1 or 2".

As used herein, the position via which a respective subsituent is connected to the rest of the molecule may in a drawn structure be depicted by a star sign [*] in said substituent.

The term "comprising" when used in the specification includes "consisting of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom, more particularly chlorine and/or fluorine.

The term "C₁-C₄-alkanediyl" represents a divalent straight-chained (linear) or branched alkanediyl radical having from 1 to 4, preferably 1, 2 or 3, or 2, 3 or 4, more preferably 2 or 3, carbon atoms. The term "C₂-C₄-alkanediyl" represents a divalent straight-chained (linear) or branched alkanediyl radical having from 2 to 4, preferably 2 or 3 carbon atoms. The following may be mentioned as preferred examples: methylene, 1,2-ethanediyl, ethane-1,1-diyl, 1,3-propylene (1,3-propanediyl), propane-1,1-diyl, propane-1,2-diyl, propane-2,2-diyl, 1,4-butylene (1,4-butanediyl), butane-1,2-diyl, butane-1,3-diyl, butane-2,3-diyl. Preferred are methylene, 1,2-ethanediyl and 1,3-propylene (1,3-propanediyl), more preferred are 1,2-ethanediyl and 1,3-propylene (1,3-propanediyl).

The term "C₂-C₄-alkenediyl" represents a divalent straight-chained (linear) or branched alkenediyl radical, which contains one double bond, having 2, 3 or 4 carbon atoms, preferably 2 or 3, more preferably 2 carbon atoms. The following may be mentioned as preferred examples: ethenyl (or "vinyl"), prop-2-en-1-yl (or "allyl"), prop-1-en-1-yl, but-3-enyl, but-2-enyl, but-1-enyl, prop-1-en-2-yl (or "isopropenyl"), 2-methylprop-2-enyl, 1-methylprop-2-enyl, 2-methylprop-1-enyl or 1-methylprop-1-enyl. Preferred are ethenyl and allyl, more preferred is ethenyl.

The term "C₂-C₄-alkynediyl" represents a divalent straight-chained (linear) alkenediyl radical, which contains one triple bond, having 2, 3 or 4 carbon atoms. The following may be mentioned as preferred examples: ethynyl, prop-1-ynyl, prop-2-ynyl (or "propargyl"), but-1-ynyl, but-2-ynyl, but-3-ynyl or 1-methylprop-2-ynyl. Particularly, said alkynyl group is prop-1-ynyl or prop-2-ynyl.

In the present invention a linear C₁-C₄-alkanediyl or C₂-C₄-alkanediyl group or a C₂-C₄-alkenediyl or a C₂-C₄-alkynediyl group may be substituted with one or more groups independently selected from halogen (as defined above), C₁-C₄-alkyl and C₃-C₆-cycloalkyl. For example, a linear C₁-C₄-alkanediyl group or a C₂-C₄-alkanediyl group, which is substituted with one or more groups selected from C₁-C₄-alkyl comprises in particular a 1,2-dimethyl-ethanediyl group, a 2,2-dimethyl-ethanediyl group, a 1,1-dimethyl-1,3-propanediyl group, a 2,2-dimethyl-1,3-propanediyl group, a 3,3-dimethyl-1,3-propanediyl group, a 1,2-dimethyl-1,3-propanediyl group, a 1,3-dimethyl-1,3-propanediyl group and a 2,3-dimethyl-1,3-propanediyl group. A 2,2-dimethyl-ethanediyl group is preferred.

In the case of a C₁-C₄-alkanediyl group or a C₂-C₄-alkanediyl group which may be substituted with one or more C₁-C₄-alkyl substituents, it is also possible that two C₁-C₄-alkyl substituents form a ring together with the carbon atom to which they are bonded. Respective groups comprise in particular the following groups:

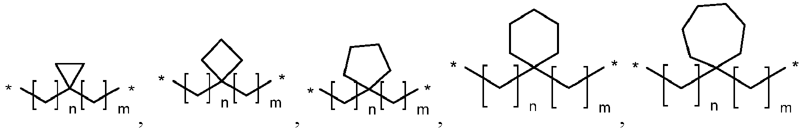

Preferably a respective group is

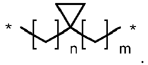

Therein n and m are independently 0, 1, 2 or 3. Preferably n and m are independently 0, 1 or 2. More preferably one of n and m is 0 and the other one is 1.

The term "C₃-C₇-cycloalkanediyl" represents a divalent monocyclic hydrocarbon radical having from 3 to 7, preferably from 3 to 6 ring carbon atoms. Examples are the following groups: 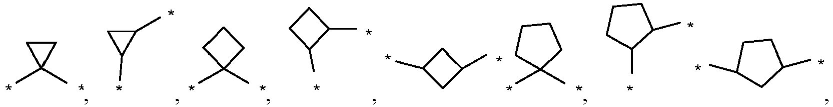 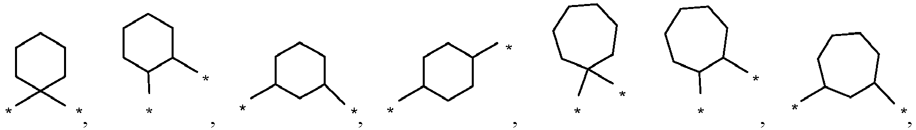 and

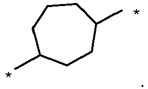

Therein, the following groups are preferred:

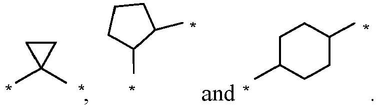

The term "phenylene" represents a divalent monocyclic aromatic radical having 6 ring carbon atoms
The term "C₅-C₆-heteroarenediyl" represents a divalent monocyclic heteroaromatic radical, wherein 1, 2 or 3 ring carbon atoms are replaced by a heteroatom, preferably by a heteroatom from the group consisting of N, O and S.

The term "Cs-Cs bicycloalkanediyl" represents a divalent bicyclic radical having 5 to 8 carbon ring carbon atoms.

Said C₃-C₇-cycloalkanediyl, phenylene, C₅-C₆-heteroarenediyl and C₅-C₈ bicycloalkanediyl groups may be substituted with one or more groups independently selected from halogen, cyano and C₁-C₄-alkyl, each as defined herein.

The term "C₁-C₆-alkyl" comprises linear and branched, saturated, monovalent hydrocarbon group having 1, 2, 3, 4, 5 or 6 carbon atoms. The term "C₁-C₄-alkyl" comprises linear and branched, saturated, monovalent hydrocarbon group having 1, 2, 3, or 4 carbon atoms. The term "C₁-C₃-alkyl" comprises linear and branched, saturated, monovalent hydrocarbon group having 1, 2 or 3 carbon atoms. Examples of the respective alkyl-groups are a methyl, ethyl, *n*-propyl, isopropyl, *n*-butyl, *sec*-butyl, isobutyl, a *tert*-butyl group etc., or an isomer thereof. Most preferred are methyl, ethyl, and n-propyl.

The term "C₃-C₆-cycloalkyl" means a saturated, monovalent, monocyclic hydrocarbon ring which contains 3, 4, 5 or 6 carbon atoms. The term "C₃-C₅-cycloalkyl" means a saturated, monovalent, monocyclic hydrocarbon ring which contains 3, 4 or 5 carbon atoms. Said C₃-C₆-cycloalkyl groups are for example, a monocyclic hydrocarbon ring, *e.g.* a cyclopropyl, cyclobutyl, cyclopentyl or cyclohexyl group. Particularly, said cycloalkyl group contains 3, 5 or 6 carbon atoms and is *e.g*. cyclopropyl, cyclopentyl or cyclohexyl.

The term "C₁-C₆-alkoxy" represents a straight-chain or branched O-alkyl having 1 to 6 carbon atoms, for example methoxy, ethoxy, n-propoxy, isopropoxy, n-butoxy, isobutoxy, s-butoxy and t-butoxy. Preference is also given to alkoxy groups having 1 to 4 carbon atoms. The inventive alkoxy groups may be substituted by one or more identical or different radicals.

The term " C₁-C₆-alkylsulphanyl" represents straight-chain or branched S-alkyl having 1 to 6 carbon atoms, for example methylthio, ethylthio, n-propylthio, isopropylthio, n-butylthio, isobutylthio, s-butylthio and t-butylthio. Preference is also given to alkylsulphanyl groups having 1 to 4 carbon atoms. The inventive alkylsulphanyl groups may be substituted by one or more identical or different radicals.

The term " C₁-C₆-alkylsulphinyl" represents straight-chain or branched alkylsulphinyl having 1 to 6 carbon atoms, for example methylsulphinyl, ethylsulphinyl, n-propylsulphinyl, isopropylsulphinyl, n-butylsulphinyl, isobutylsulphinyl, s-butylsulphinyl and t-butylsulphinyl. Preference is also given to alkylsulphinyl groups having 1 to 4 carbon atoms. The inventive alkylsulphinyl groups may be substituted by one or more identical or different radicals.

The term "alkylsulphonyl" represents straight-chain or branched alkylsulphonyl having 1 to 6 carbon atoms, for example methylsulphonyl, ethylsulphonyl, n-propylsulphonyl, isopropylsulphonyl, n-butylsulphonyl, isobutylsulphonyl, s-butylsulphonyl and t-butylsulphonyl. Preference is also given to alkylsulphonyl groups having 1 to 4 carbon atoms. The inventive alkylsulphonyl groups may be substituted by one or more identical or different radicals.

The terms "halogen-substituted C₁-C₆-alkyl", "halogen-substituted C₁-C₆-alkoxy" and "halogen-substituted C₃-C₆-cycloalkyl" represent C₁-C₆-alkyl, C₁-C₆-alkoxy and C₃-C₆-cycloalkyl groups as defined above, which are mono- or polysubstituted by halogen up to the maximum possible number of substituents. Such groups are also referred to as halo groups (for example haloalkyl). In the case of polysubstitution by halogen, the halogen atoms may be the same or different, and may all be bonded to one carbon atom or may be bonded to a plurality of carbon atoms. Halogen is especially fluorine, chlorine, bromine or iodine, preferably fluorine, chlorine or bromine and more preferably fluorine. More particularly, halogen-substituted groups are monohalocycloalkyl such as 1-fluorocyclopropyl, 2-fluorocyclopropyl or 1-fluorocyclobutyl, monohaloalkyl such as 2-chloroethyl, 2-fluoroethyl, 1-chloroethyl, 1-fluoroethyl, chloromethyl, or fluoromethyl; perhaloalkyl such as trichloromethyl or trifluoromethyl or CF₂CF₃, polyhaloalkyl such as difluoromethyl, 2-fluoro-2-chloroethyl, dichloromethyl, 1,1,2,2-tetrafluoroethyl or 2,2,2-trifluoroethyl. Further examples of haloalkyls are trichloromethyl, chlorodifluoromethyl, dichlorofluoromethyl, chloromethyl, bromomethyl, 1-fluoroethyl, 2-fluoroethyl, 2,2-difluoroethyl, 2,2,2-trifluoroethyl, 2,2,2-trichloroethyl, 2-chloro-2,2-difluoroethyl, pentafluoroethyl, 3,3,3-trifluoropropyl and pentafluoro-t-butyl. Preference is given to haloalkyls having 1 to 4 carbon atoms and 1 to 9, preferably 1 to 5, identical or different halogen atoms selected from fluorine, chlorine and bromine, preferably from fluorine. Particular preference is given to haloalkyls having 1 or 2 carbon atoms and 1 to 5 identical or different halogen atoms selected from fluorine and chlorine, such as, inter alia, difluoromethyl, trifluoromethyl or 2,2-difluoroethyl. Further examples of halogen-substituted compounds are haloalkoxy such as OCF₃, OCHF₂, OCH₂F, OCF₂CF₃, OCH₂CF₃, OCH₂CHF₂ and OCH₂CH₂Cl, haloalkylsulphanyls such as difluoromethylthio, trifluoromethylthio, trichloromethylthio, chlorodifluoromethylthio, 1-fluoroethylthio, 2-fluoroethylthio, 2,2-difluoroethylthio, 1,1,2,2-tetrafluoroethylthio, 2,2,2-trifluoroethylthio or 2-chloro-1,1,2-trifluoroethylthio, haloalkylsulphinyls such as difluoromethylsulphinyl, trifluoromethylsulphinyl, trichloromethylsulphinyl, chlorodifluoromethylsulphinyl, 1-fluoroethylsulphinyl, 2-fluoroethylsulphinyl, 2,2-difluoroethylsulphinyl, 1,1,2,2-tetrafluoroethylsulphinyl, 2,2,2-trifluoroethylsulphinyl and 2-chloro-1,1,2-trifluoroethylsulphinyl, haloalkylsulphinyls such as difluoromethylsulphinyl, trifluoromethylsulphinyl, trichloromethylsulphinyl, chlorodifluoromethylsulphinyl, 1-fluoroethylsulphinyl, 2-fluoroethylsulphinyl, 2,2-difluoroethylsulphinyl, 1,1,2,2-tetrafluoroethylsulphinyl, 2,2,2-trifluoroethylsulphinyl and 2-chloro-1,1,2-trifluoroethylsulphinyl, haloalkylsulphonyl groups such as difluoromethylsulphonyl, trifluoromethylsulphonyl, trichloromethylsulphonyl, chlorodifluoromethylsulphonyl, 1-fluoroethylsulphonyl, 2-fluoroethylsulphonyl, 2,2-difluoroethylsulphonyl, 1,1,2,2-tetrafluoroethylsulphonyl, 2,2,2-trifluoroethylsulphonyl and 2-chloro-1,1,2-trifluoroethylsulphonyl. Most preferred fluorinated alkyl groups are CF₃, C₂F₅ and C₃F₇ and most preferred fluorinated alkoxy groups are OCF₃, OC₂F₅ and OC₃F₇.

The terms "*N-*C₁-C₆-alkylamino", "*N,N-*di-C₁-C₆-alkylamino", and "*N*-C₁-C₃-alkoxy-C₁-C₄-alkylamino" represent an amino group substituted with one or two C₁-C₆-groups or an amino group substituted with one C₁-C₃-alkoxy group and one C₁-C₄-alkyl group, each as defined above.

The term " C₁-C₆-alkylcarbonyl" represents straight-chain or branched alkyl-C(=O) having 2 to 7 carbon atoms such as methylcarbonyl, ethylcarbonyl, n-propylcarbonyl, isopropylcarbonyl, s-butylcarbonyl and t-butylcarbonyl. Preference is also given to alkylcarbonyls having 1 to 4 carbon atoms. The inventive alkylcarbonyls may be substituted by one or more identical or different radicals.

The term "C₂-C₆-alkenyl" represents straight-chain or branched hydrocarbons preferably having 2 to 6 carbon atoms and at least one double bond, for example vinyl, 2-propenyl, 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl, 3-hexenyl, 4-hexenyl, 5-hexenyl, 1-methyl-2-pentenyl, 2-methyl-2-pentenyl, 3-methyl-2-pentenyl, 4-methyl-2-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 2,2-dimethyl-3-butenyl, 2,3-dimethyl-2-butenyl, 2,3-dimethyl-3-butenyl, 1-ethyl-2-butenyl, 1-ethyl-3-butenyl, 2-ethyl-2-butenyl, 2-ethyl-3-butenyl, 1,1,2-trimethyl-2-propenyl, 1-ethyl-1-methyl-2-propenyl and 1-ethyl-2-methyl-2-propenyl. Preference is also given to alkenyls having 2 to 4 carbon atoms such as, inter alia, 2-propenyl, 2-butenyl or 1-methyl-2-propenyl. The inventive alkenyls may be substituted by one or more identical or different radicals.

The term " C₂-C₆- alkynyl" represents straight-chain or branched hydrocarbons preferably having 2 to 6 carbon atoms and at least one triple bond, for example 2-propynyl, 2-butynyl, 3-butynyl, 1-methyl-2-propynyl, 2-pentynyl, 3-pentynyl, 4-pentynyl, 1-methyl-3-butynyl, 2-methyl-3-butynyl, 1-methyl-2-butynyl, 1,1-dimethyl-2-propynyl, 1-ethyl-2-propynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-4-pentynyl, 4-methyl-2-pentynyl, 1,1-dimethyl-3-butynyl, 1,2-dimethyl-3-butynyl, 2,2-dimethyl-3-butynyl, 1-ethyl-3-butynyl, 2-ethyl-3-butynyl, 1-ethyl-1-methyl-2-propynyl and 2,5-hexadiynyl. Preference is also given to alkynyls having 2 to 4 carbon atoms such as, inter alia, ethynyl, 2-propynyl or 2-butynyl-2-propenyl. The inventive alkynyls may be substituted by one or more identical or different radicals.

The term "aryl" represents a mono-, bi- or polycyclic aromatic system having preferably 6 to 14, especially 6 to 10, ring carbon atoms, for example phenyl, naphthyl, anthryl, phenanthrenyl, preferably phenyl. In addition, aryl also represents polycyclic systems such as tetrahydronaphthyl, indenyl, indanyl, fluorenyl, biphenyl, where the bonding site is on the aromatic system. The inventive aryl groups may be substituted by one or more identical or different radicals.

The term "hetaryl" or "heteroaryl" represents heteroaromatic compounds, i.e. completely unsaturated aromatic heterocyclic compounds having at least one ring in which at least one carbon atom is replaced by a heteroatom, preferably by a heteroatom from the group consisting of N, O, S, P, B, Si, Se, and which may be unsubstituted or substituted, where the bonding site is on a ring atom. Unless defined differently, the heteroaryl ring contains preferably 3 to 9 ring atoms, especially 3 to 6 ring atoms, more preferably 5 to 7 ring atoms, and one or more, preferably 1 to 4, especially 1, 2 or 3, heteroatoms in the heteroaryl ring, preferably from the group consisting of N, O, and S, although no two oxygen atoms should be directly adjacent. The heteroaryl rings usually contain not more than 4 nitrogen atoms and/or not more than 2 oxygen atoms and/or not more than 2 sulphur atoms. Particular preference is given to 5- to 7-membered rings having 1 to 3, preferably 1 or 2, identical or different heteroatoms from the group above. Inventive heteroaryls are, for example, furyl, thienyl, pyrazolyl, imidazolyl, 1,2,3- and 1,2,4-triazolyl, isoxazolyl, thiazolyl, isothiazolyl, 1,2,3-, 1,3,4-, 1,2,4- and 1,2,5-oxadiazolyl, azepinyl, pyrrolyl, pyridyl, pyridazinyl, pyrimidinyl, pyrazinyl, 1,3,5-, 1,2,4- and 1,2,3-triazinyl, 1,2,4-, 1,3,2-, 1,3,6- and 1,2,6-oxazinyl, oxepinyl, thiepinyl, 1,2,4-triazolonyl and 1,2,4-diazepinyl. The inventive heteroaryl groups may also be substituted by one or more identical or different radicals

Preferred substituents of the aryl and heteroaryl groups are selected from hydroxy, halogen, cyano, nitro, amino, C₁-C₃-alkyl, C₁-C₃-alkoxy, hydroxycarbonyl, alkoxycarbonyl, alkylcarbamoyl, cycloalkylcarbamoyl, phenyl and 1-pyrrolidinyl.

Preferably the substituents Y and L have the meaning as defined above in the various aspects of the present invention.

In a particularly preferred aspect of the invention the substituent Y in the compounds of formula (I) as defined anywhere herein is selected from a group (T¹) as defined herein, in particular a group (T1-1) or (T1-2), each as defined anywhere herein. In an alternative aspect, of the invention the substituent Y in the compounds of formula (I) as defined anywhere herein is selected from a group (T²) as defined herein, in particular a group (T1-1) or (T1-2), each as defined anywhere herein

Particularly preferred Examples of the present invention are listed in aspect [24] above and as shown in the Examples below. Preferred compounds according to formula (I) of the present invention are those of Examples 6, 7, 8, 13, 14. More preferred compounds according to formula (I) of the present invention are those of Examples 8 and 13.

Salts of the inventive compounds that are suitable in accordance with the invention, for example salts with bases, are all customary non-toxic salts, preferably agriculturally and/or physiologically acceptable salts. Preference is given to salts with inorganic bases, for example alkali metal salts (e.g. sodium, potassium or caesium salts), alkaline earth metal salts (e.g. calcium or magnesium salts), ammonium salts or salts with organic bases, in particular with organic amines, for example triethylammonium, dicyclohexylammonium, N,N-dibenzylethylenediammonium, pyridinium, picolinium or ethanolammonium salts. Preferably salts of the compounds of the present invention are pharmaceutically acceptable salts.

Furthermore, the present invention includes all possible crystalline forms, or polymorphs, of the compounds of the present invention, either as single polymorph, or as a mixture of more than one polymorph, in any ratio.

The compounds of the present invention and their salts - to the extent applicable -can exist as a hydrate, or as a solvate, wherein the compounds of the present invention contain water or polar solvents (for example methanol or ethanol), respectively, as structural element of the crystal lattice of the compounds. It is possible for the amount of polar solvents, in particular water, to exist in a stoichiometric or non-stoichiometric ratio. In the case of stoichiometric solvates, e.g. a hydrate, hemi-, (semi-), mono-, sesqui-, di-, tri-, tetra-, penta- etc. solvates or hydrates, respectively, are possible. The present invention includes all such hydrates or solvates of the compounds and their salts, to the extent applicable.

Depending on the nature of the substituents, the compounds of the formula (I) may be in the form of geometric and/or optically active isomers or corresponding isomer mixtures in different compositions. These stereoisomers are, for example, enantiomers, diastereomers, atropisomers, tautomers or geometric isomers. Accordingly, the invention encompasses both pure stereoisomers and any mixtures of these isomers.

The novel compounds according to the present invention are particularly suitable for the use as medicaments, in particular for the use as medicaments for the treatment of animals. The novel compounds according to the present invention are particularly suitable for the use as medicaments to act against animal parasites, especially ectoparasites, such as insects and arachnids or else. Ectoparasites are typically and preferably arthropods, especially insects such as flies (biting and licking), parasitic fly larvae, sucking lice, biting lice, fleas and the like; or acari such as ticks, for example hard ticks or soft ticks, or mites such as scab mites, bird mites and the like, and also aquatic ectoparasites such as copepods. The novel compounds of the present invention are particularly suitable to act against ticks, fleas, lice, flies and mites.

The novel compounds of the formula (I) having favourable homeotherm toxicity are suitable for controlling parasites which occur in animal breeding and animal husbandry in livestock including aquaculture, breeding animals, zoo animals, laboratory animals, experimental animals and domestic animals. They are active against all or specific stages of development of the parasites.

Agricultural livestock include, for example, mammals such as sheep, goats, horses, donkeys, camels, buffalo, rabbits, reindeer, fallow deer, and particularly cattle and pigs; poultry such as turkeys, ducks, geese, and particularly chickens; fish and crustaceans, for example in aquaculture.

Domestic animals (also indicated as companion animals) include, for example, mammals, such as hamsters, guinea pigs, rats, mice, chinchillas, ferrets, and particularly dogs, cats, cage birds, reptiles, amphibians and aquarium fish. Preferred companion animals are cats and dogs.

In a preferred embodiment, the compounds of the formula (I) are administered to mammals.

In a preferred embodiment the compounds of formula (I) are administered to cats.

In a preferred embodiment the compounds of formula (I) are administered to dogs.

Use of the compounds of the formula (I) for the control of animal parasites is intended to reduce or prevent illness, cases of deaths and reductions in performance (in the case of meat, milk, wool, hides, eggs, honey and the like), such that more economical and simpler animal keeping is enabled and better animal well-being is achievable.

In relation to the animal health field, the term "control" or "controlling" means that the compounds of the formula (I) are effective in reducing the incidence of the particular parasite in an animal infected with such parasites to an innocuous degree. More specifically, "controlling" in the present context means that the compound of the formula (I) can kill the respective parasite, inhibit its growth, or inhibit its proliferation.

These parasites include:
From the order of the Anoplurida, for example, Haematopinus spp., Linognathus spp., Pediculus spp., Phthirus spp., Solenopotes spp.; specific examples are: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;

From the order of the Mallophagida and the suborders Amblycerina and Ischnocerina, for example, Trimenopon spp., Menopon spp., Trinoton spp., Bovicola spp., Werneckiella spp., Lepikentron spp., Damalina spp., Trichodectes spp., Felicola spp.; specific examples are: Bovicola bovis, Bovicola ovis, Bovicola limbata, Damalina bovis, Trichodectes canis, Felicola subrostratus, Bovicola caprae, Lepikentron ovis, Werneckiella equi;

From the order of the Diptera and the suborders Nematocerina and Brachycerina, for example, Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; specific examples are: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;

From the order of the Siphonapterida, for example, Pulex spp., Ctenocephalides spp., Tunga spp., Xenopsylla spp., Ceratophyllus spp.; specific examples are: Ctenocephalides canis, Ctenocephalides felis, Pulex irritans, Tunga penetrans, Xenopsylla cheopis;

From the order of the Heteropterida, for example, Cimex spp., Triatoma spp., Rhodnius spp., Panstrongylus spp.

From the order of the Blattarida, for example, Blatta orientalis, Periplaneta americana, Blattela germanica and Supella spp. (e.g. Suppella longipalpa);

From the subclass of the Acari (Acarina) and the orders of the Meta- and Mesostigmata, for example, Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (the original genus of multihost ticks), Omithonyssus spp., Pneumonyssus spp., Raillietia spp., Pneumonyssus spp., Stemostoma spp., Varroa spp., Acarapis spp.; specific examples are: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Omithonyssus sylviarum, Varroa jacobsoni;

From the order of the Actinedida (Prostigmata) and Acaridida (Astigmata), for example, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp.; specific examples are: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschongastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

From the subclass of the copepods with the order of the Siphonostomatoida in particular the genera Lepeophtheirus and Caligus; the species Lepeophtheirus salmonis, Caligus elongates and Caligus clemensi may be mentioned by way of example and with particular preference.

According to a preferred embodiment the parasites are selected from the following group of ectoparsite species:
Fleas: Ctenocephalides spp.;
Ticks: Amblyomma spp., Dermacentor spp., Rhipicephalus spp., Ixodes spp., Haemaphysalis spp., Hyalomma spp.;
Mites: Demodex spp., Otodectes spp., Sarcoptes spp.; and
Lice: Linognathus spp,.

More particularly the parasites are selected from:
Fleas: Ctenocephalides felis, Ctenocephalides canis;
Ticks: Ixodes scapularis, Ixodes ricinus, Dermacentor variabilis, Amblyomma americanum, Rhipicephalus sanguineus, Dermacentor reticulatus, Ixodes holocyclus, Ixodes hexagonus, Haemaphysalis longicornis;
Mites: Otodectes cynotis, Sarcoptes scabiei, Demodex canis; and
Lice: Linognathus setosus.

In general, the inventive active ingredients can be employed directly when they are used for the treatment of animals. They are preferably employed (administered) in the form of pharmaceutical compositions which may comprise pharmaceutically acceptable excipients, solvents and/or auxiliaries known in the prior art.

The novel active compounds of the present invention may be administered in a known manner, by enteral administration in the form of, for example, tablets, capsules, potions, drenches, granules, pastes, boluses, the feed-through process and suppositories, by parenteral administration, for example by injection (intramuscular, subcutaneous, intravenous, intraperitoneal inter alia), implants, by nasal administration, by dermal administration in the form, for example, of dipping or bathing, spraying, pouring on and spotting on, washing and powdering, and also with the aid of moulded articles containing the active ingredient, such as collars, earmarks, tailmarks, limb bands, halters, marking devices, etc. Preferably the novel compounds of the present invention are administered by subcutaneous or oral administration, more preferably by subcutaneous administration (injection).

The novel active compounds of the present invention can be formulated in any suitable administration form for oral and subcutaneous (injectable) administration known in the prior art.

Based upon standard laboratory techniques known to evaluate compounds useful for controlling parasites on animals, by standard toxicity tests and by standard pharmacological assays for the determination of treatment of the conditions identified above in animals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the subject treated, and the nature and extent of the condition treated.

The content of the novel active compounds of the present invention in formulations for the use (unit dosage) according to the present invention may vary within wide limits. The active compound concentration of the application forms may be from 0.00000001 to 98% by weight of active compound, preferably from 0.00001 to 98 % by weight, more preferably from 0.001 to 98 % by weight. More preferably, the pharmaceutical compositions of the present invention may comprise the novel compounds of the invention in amounts from 0.01 to 98% by weight of active compound, preferably from 0.1 to 98 % by weight. , more preferably from 0.5 to 90 % by weight. The pharmaceutical compositions of the present invention may comprise the novel compounds of the invention in amounts from 0.001 to 95 % by weight of active compound, preferably from 0.01 to 95% by weight, preferably from 0.1 to 50 % by weight, more preferably from 5 to 30 % by weight.

The total amount of the active ingredient to be administered will generally range from about 0.01 to 200 mg/kg body weight per application, preferably in the range of from 0.1 to 100 mg/kg body weight per application, more preferably of from 0.5 to 75 mg/kg body weight per application, more preferably in the range of from 1.0 to 50 mg/kg body weight and most preferably in the range of from 2.0 to 20 mg/kg body weight per application.

In particular, the average dosage for administration by infusion techniques or by injection, including intravenous, intramuscular, and in particular subcutaneous injections will preferably be within the aforesaid ranges.

The average dosage for oral administration will preferably be within the aforesaid ranges.

Clinically useful dosing or administration intervals will range from one application per month to one application every two years, preferably the treatment interval is from one application every three months to one application every two years, more preferred treatment intervals are from one application every six months to one application every two years. Further preferred dosing or administration intervals will range from one application per month to one application every year, preferably one application every three months to one application every year, more preferably one application every four months to one application every year, in particular one application every six months to one application every year. According to a further embodiment the application interval may be from one application every nine months to one application per year.

In addition, it is possible for "drug holidays", in which a subject is not dosed with a drug for a certain period of time, to be beneficial to the overall balance between pharmacological effect and tolerability.

Of course the specific initial and continuing dosage regimen, administered amount of active compound and the particular dosing interval will vary for each subject according to the nature and severity of the condition as determined by the attending diagnostician, the activity of the specific compound employed, the age and general condition of the subject, time of administration, route of administration, rate of excretion of the drug, drug combinations, and the like. The desired mode of treatment and number of doses of a compound of the present invention or a pharmaceutically acceptable salt or composition thereof can be ascertained by those skilled in the art using conventional treatment tests.

In order to broaden the spectrum of activity, the novel active compounds of the present invention can be used in combination with suitable synergists, repellents or other active ingredients, for example acaricides, insecticides, anthelmintics, anti-protozoal agents.

For example the compounds of the present invention can be used in combination with chloride channel activators or modulators from the class of the macrocylic lactones, in particular avermectins/milbemycins, e.g. abamectin, doramectin, emamectin benzoate, eprinomectin, ivermectin, latidectin, lepimectin, milbemycin oxime, milbemectin, moxidectin and selamectin, particular preference is given here, for applications against ectoparasites, to doramectin, eprinomectin, ivermectin, milbemycin oxime, moxidectin or selamectin.

Further, the the compounds of the present invention can be used in combination with antigens for vaccination purposes. Examples for vaccines which may be combined with the compounds of the present invention are against leptospirosis, infectious tracheobronchitis, leishmaniasis or lyme borreliosis (lyme disease).

### Process for Preparing the Novel Compounds of Formula (I)

The novel compounds (I) according to the present invention can be synthesised in a process comprising the step of reacting a compound (A) 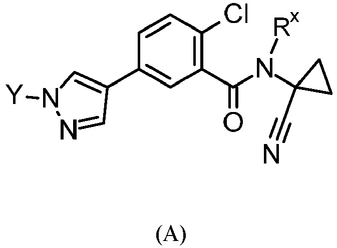 with a group (B) 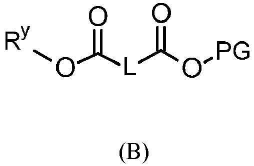 wherein
R^{x} represents hydrogen and R^{y} represents a -CH₂-Cl group or
R^{x} represents a -CH₂-OH group and R^{y} represents hydrogen;
wherein
Y and L have the meaning as defined anywhere herein and wherein PG represents a protecting group or hydrogen to form the compounds according to formula (I),
and wherein in cases wherein PG is not hydrogen, deprotection is carried out to form the compounds (I).

Such process may further comprise one of the following preliminary steps:
in the case that R^{x} represents hydrogen and R^{y} represents a -CH₂-Cl group the preliminary step of preparing the group (B) with R^{y} = -CH₂-Cl (compound (B-2)) from a compound (B-1) 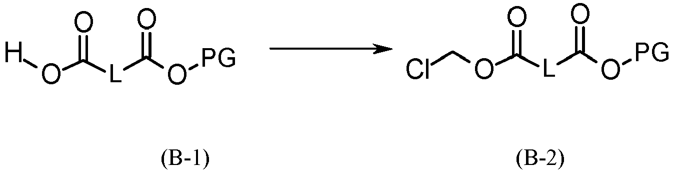 and
in the case that R^{x} represents a -CH₂-OH group and R^{y} represents hydrogen the preliminary step of
preparing the group (A) with R^{x} = -CH₂-OH (compound A-3)) from a compound (A-1) via an intermediate compound (A-2)

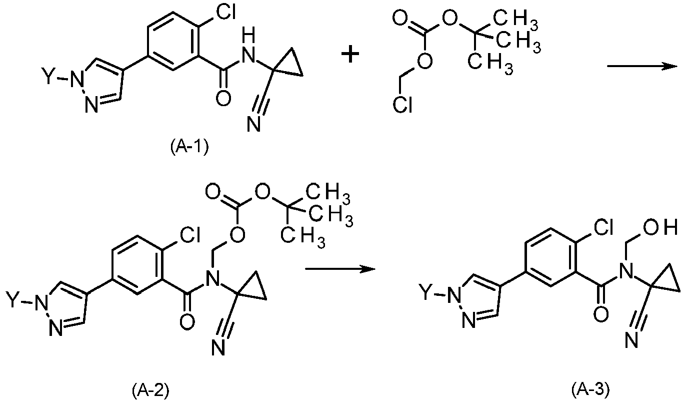

The resulting alternative preparation processes are further illustrated in the general Schemes 1 and 2 as shown below.

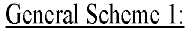

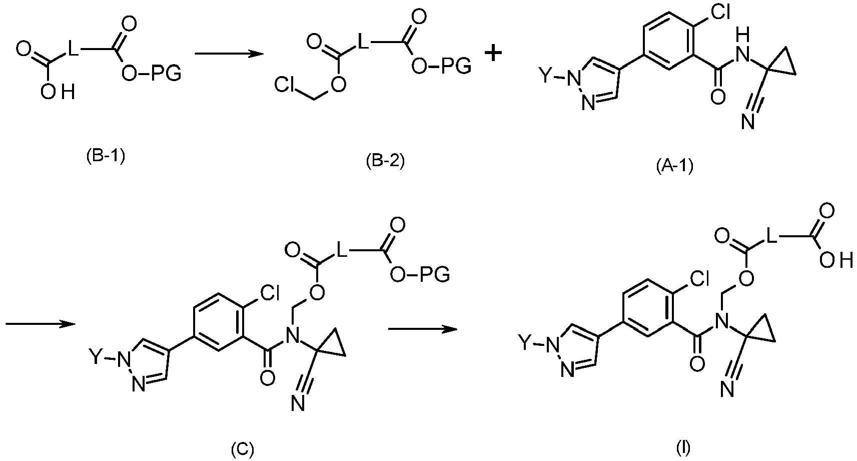

### Preliminary Reaction Step:

The group (B) with R^{y} = -CH₂-Cl (indicated as compound (B-2)) may be prepared from a monoprotected dicarboxylic acid compound (B-1) by treatment with chloromethyl carbonochloridate to form the chloromethyl ester compounds (B-2). The reaction is carried out in a solvent in the presence of a base. Dipolar aprotic solvents such as DMF, THF, acetonitrile, or pyridine can be used. The base may be an amine base like e..g. DIPEA, TEA, DMAP. In a preferred case, pyridine serves both as the solvent and the base. As defined herein, PG may represent hydrogeh, however, it is preferred, that PG represents a suitable protecting group, for instance, a tert-butyl group. Other suitable protecting groups are described in P.G. Wuts, Greene's Protective Groups in Organic Synthesis, Wiley 2014**.**

### Main Reaction Step:

The compound of the formula (I) is formed by treating a compound (A) with R^{x} = hydrogen (indicated as compound (A-1)) with a base followed by reaction with the chloromethyl ester compound of formula (B-2). Strong bases can be used, for instance alkali metal *tert*-butylates, alkali metal hydride bases, metal amide bases, bis(trimethyldisilyl)amide (HMDS) bases, amidine bases or phosphazene bases. In a preferred case, sodium hydride or NaHMDS or KHMDS is used. The reaction is generally carried out in a solvent, usually in a polar aprotic solvent such as THF, diethyl ether, DMF or a mixture of a polar aprotic solvent and other solvents. If PG is hydrogen, this leads directly to the compounds (I) of the present invention. However, in a preferred aspect of the invention PG represents a protecting group, leading to the protected intermediate compounds (C). In such cases, the protecting group PG is cleaved to provide the compound of the invention of structure (I). In a particularly preferred case, PG is a *tert*-butyl group and is removed by treating compound (C) with a solution of hydrochloric acid in 1,4 dioxane or with a solution of TFA in dichloromethane.

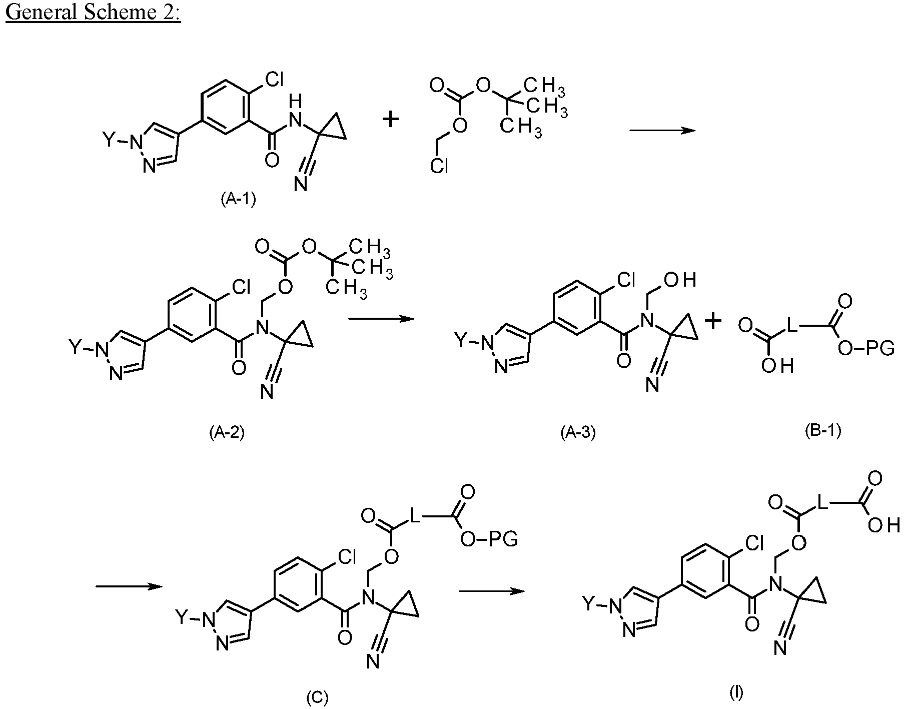

### Preliminary Reaction Step:

The group (A) with R^{x} = -CH₂-OH (indicated as compound (A-3)) may be prepared by coupling an amide compound according to formula (A-1) with *tert*-butyl chloromethyl carbonate (CAS RN 35180-02-0) using a strong base as, for instance an alkali metal *tert*-butylate, an alkali metal hydride, a metal amide base, a bis(trimethyldisilyl)amide (HMDS) base, an amidine base or a phosphazene base. In a preferred case, KHMDS is used. The reaction is generally carried out in a solvent, usually in a polar aprotic solvent such as THF, diethyl ether, DMF or a mixture of a polar aprotic solvent and other solvents. The resulting compound (A-2) is then converted into the hydroxymethyl compound (A-3) by careful removal of the *tert-*butyl carbonate moiety with an acid such as TFA. This reaction is usually carried out in a solvent such as DCM. In case PG is a *tert*.butyl ester, selectivity of carbonate cleavage over the cleavage of PG can be obtained by keeping the reaction time short enough and carrying the reaction out at room temperature or below.

### Main Reaction Step:

The compound of the formula (I) is formed by coupling a compound (A) with R^{x} = -CH₂-OH (indicated as compound (A-3)) with a carboxylic acid compound (B-1). To this end, the compound (B-1) is activated by converting it into the corresponding acid chloride with a method known in the art, preferentially by using oxalic chloride and a catalytic amount of DMF in dichloromethane. The acid chloride is then reacted with compound (A-3) in the presence of a base, preferably an amine base such as DIPEA, TEA or pyridine in an appropriate solvent such as THF, DMF, DCM.

Alternatively, compound (B-1) can be activated by a coupling agent such as HATU, TCTU or other reagents known in the art and then be reacted with compound (A-3) in the presence of a base such as DIPEA, TEA or pyridine in an appropriate solvent such as, for instance, THF, DMF or DCM.

If PG is hydrogen, this leads directly to the compounds (I) of the present invention. However, in a preferred aspect of the invention PG represents a protecting group, leading to the protected intermediate compounds (C). In such cases, the protecting group PG is cleaved to provide the compound of the invention of structure (I). In a particularly preferred case, PG is a *tert*-butyl group and is removed by treating compound (C) with a solution of hydrochloric acid in 1,4 dioxane or with a solution of TFA in dichloromethane.

In the above described Schemes 1 and 2 the groups L, Y and PG have the meanind as defined anywhere herein.

As mentioned above, the invention further relates to the intermediate compounds obtainable in the process according to the present invention, such as in particular intermediate compounds of the to formula (B-1) or (B-2): 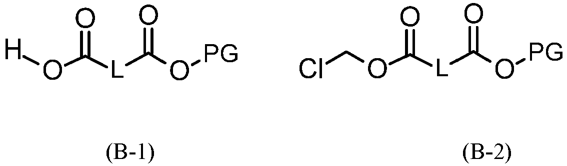 or according to formula (C): 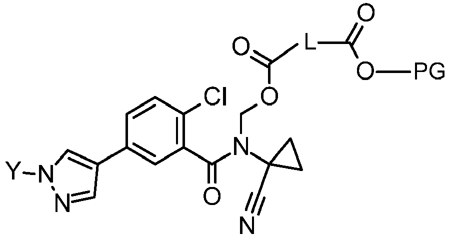 wherein
- L: has the meaning as defined in any one of the preceding aspects and
- PG: represents a tert-butyl group.

Specific examples of intermediate compounds according to the formulae (B-1), (B-2) and (C) are further shown in the following Examples.

### Examples

### List of Abbreviations

- AA: Amblyomma americanus (parasitology)
- abs.: Absolute
- Ac: Acetyl
- aq.: Aqueous, aqueous solution
- AUC: Area under the curve (in Pharmacokinetics)
- cat.: Catalytic
- CF: Ctenocephalides felis (parasitology)
- CI: Chemical ionisation (mass spectroscopy)
- cone: Concentrated
- d: Doublet (NMR)
- d: Day(s)
- dd: Doublet of doublet (NMR)
- DCM: Dichloromethane
- DIPEA: *N,N*,-diisopropylethylamine (Hünig's base)
- DMAP: 4-Dimethylaminopyridine
- DMF: *N,N*-dimethylformamide
- DMSO: Dimethylsulfoxide
- dt: Doublet of triplet (NMR)
- DV: Dermacentor variabilis (parasitology)
- *ent*: Enantiomeric
- eq.: Equivalent(s)
- ESI: Electrospray-ionisation (mass spectroscopy)
- F: Bioavailability (in Pharmacokinetics)
- GC: Gas chromatography
- GC/MS: Gas chromatography coupled to mass spectroscopy
- h: Hour(s)
- HATU: *O*-(7-Azabenzotriazol-1-yl)-*N,N,N,N'*-tetramethyluronium-hexafluorophosphate
- HMDS: Bis(trimethyldisilyl)amide formerly also referred to as hexamethyldisilazide, counterion in strongly basic salts such as LiHMDS, NaHMDS, KHMDS
- HPLC: High pressure liquid chromatography
- iPr: Isopropyl
- IR: Ixodes rhicinus (parasitology)
- iv: Intravenous (PK)
- KHMDS: Potassium bis(trimethyldisilyl)amide
- LC: Liquid chromatography
- LC-MS: Liquid chromatography coupled to mass spectroscopy
- LiHMDS: Lithium bis(trimethyldisilyl)amide
- Lit.: Literature
- m: Multiplet (NMR)
- Me: Methyl
- min: Minute(s)
- MS: Mass spectroscopy
- MTBE: *tert*-Butyl methyl ether
- na: Not analysed
- NaHMDS: *Sodium* bis(trimethyldisilyl)amide
- NMP: *N*-Methyl-2-pyrrolidone
- NMR: Nuclear magnetic resonance spectroscopy
- PBS: Phosphate buffered saline
- PEG: Polyethylene glycol
- PK: Pharmacokinetics
- Pr: Propyl
- q (or quart): Quartet (NMR)
- qd: Quartet of doublet (NMR)
- quant.: quantitative (referring to chemical yield)
- quint: Quintet (NMR)
- *rac*: racemic
- RP: reverse phase (for liquid chromatography)
- RS: Rhipicephalus sanguineus (parasitology)
- Rt: Retention time (chromatography)
- s: Singulet (NMR)
- sc: Subcutaneous (PK and pharmacology)
- SD: Study day (pharmacology)
- sept: Septet (NMR)
- t: Triplet (NMR)
- t: Time point (during an experiment)
- t₀: Time point at beginning of an experiment
- TBTU: *O*-(Benzotriazol-1-yl)-*N,N,N',N*'-tetramethyluronium-tetra-fluoroborate
- tBu: *tert*.-Butyl
- td: Triplett of doublett (NMR)
- TEA: triethylamine
- TFA: Trifluoro acetic acid
- THF: Tetrahydrofuran
- UV: Ultraviolett-spectroscopy

### Analytical Methods

### Method 1 (HPLC-MS)

Instrument: Agilent MS Quad 6150;HPLC: Agilent 1290; Column: Waters Acquity UPLC HSS T3 1.8 µm 50 × 2.1 mm; Eluent A: 1 l water + 0.25 mL 99%ige Formic acid , Eluent B: 1 l Acetonitrile + 0.25 mL 99%ige Formic acid; Gradient: 0.0 min 90 % A → 0.3 min 90 % A → 1.7 min 5% A → 3.0 min 5% A Column temperature: 50°C; Flow: 1,20 mL/min; UV-Detektion: 205 - 305 nm.

### Method 2 (HPLC-MS)

Instrument: Waters ACQUITY SQD UPLC System; Column: Waters Acquity UPLC HSS T3 1.8 µm 50 × 1 mm; eluent A: 1 l water + 0.25 mL 99%ige formic acid , eluent B: 1 L acetonitrile + 0.25 mL 99% formic acid; gradient: 0.0 min 90 % A → 1.2 min 5% A → 2.0 min 5% A column temperature: 50°C; flow: 0.40 mL/min; UV-detection: 210 nm.

### Method 3 (HPLC-MS)

Instrument MS: Thermo Scientific FT-MS; instrument UHPLC+: Thermo Scientific UltiMate 3000; Column: Waters, HSST3, 2.1 × 75 mm, C18 1.8 µm; eluent A: 1 L water + 0.01 % formic acid; eluent B: 1 L acetonitrile + 0.01 % formic acid; gradient: 0.0 min 10 % B → 2.5 min 95% B → 3.5 min 95% B; column temperature: 50°C; flow: 0.90 mL/min; UV-detection: 210 nm/ Optimum Integration Path 210-300 nm

### Method 4 (HPLC-MS)

Instrument: Waters ACQUITY SQD UPLC System; Column: Waters Acquity UPLC HSS T3 1.8 µm 50 × 1 mm; eluent A: 1 L water + 0.25 mL 99% formic acid , eluent B: 1 L acetonitrile+ 0.25 mL 99% formic acid; gradient: 0.0 min 95% A → 6.0 min 5% A → 7.5 min 5% A column temperature: 50°C; flow: 0.35 mL/min; UV-Detection: 210 nm.

### Method 5 (HPLC-MS)

The column used was a Shim-pack XR-ODS, 2.2 µm, 3.0 × 50 mm. A linear gradient was applied, starting at 95% A (A: 0.05% TFA in water) and ending at 100 % B (B: 0.05% TFA in acetonitrile) over 4.70 min with a total run time of 5.00 min. The column temperature was at 45 °C with the flow rate of 1.20 mL/min.

### Method 6 (HPLC-MS)

The column used was a Shim-pack XR-ODS, 2.2 µm, 3.0 × 50 mm. A linear gradient was applied, starting at 95% A (A: 0.05% TFA in water) and ending at 100 % B (B: 0.05% TFA in acetonitrile) over 1.70 min with a total run time of 2.00 min. The column temperature was at 40 °C with the flow rate of 1.20 mL/min.

### Method 7 (HPLC-MS)

The column used was a Kinetex EVO C18 100A, 2.6 µm, 3.0 × 50 mm. A linear gradient was applied, starting at 90 % A (A: 0.03% NH₃H₂O in water) and ending at 95% B (B: acetonitrile) over 1.70 min with a total run time of 2.00 min. The column temperature was at 40 °C with the flow rate of 1.20 mL/min.

### Method 8 (HPLC)

Instrument: HP 1260 Infinity HPLC System with G4212B diode array detector; column: Kromasil 100 C18ec 5 µm 250 × 4 mm; eluent A: 1 L water + 1.0 mL TFA, eluent B: 1 L acetonitrile+ 1.0 mL TFA; gradient: 0.0 min 98% A → 1.0 min 98% A → 8.0 min 30 % A → 16.0 min 30 % A → 19.0 min 2% A → 20.0 min 2% A → 23.0 min 98% A → 25.0 min 30 % A, column temperature: 37°C; flow: 1.5 mL/min; UV-Detection: 214 nm, injection volume 10 µL.

### Method 9 (HPLC-MS)

Instrument: HP 1200 Infinity HPLC System with G1315B diode array detector; Waters MS QuattroMicro (ESI+/ESI-) column: Kromasil 100 C18ec 5 µm 250 × 4 mm; eluent A: 1 L water + 0.5 mL 50 % HCOOH, eluent B: 1 L acetonitrile+ 0.5 mL 50 % HCOOH; gradient: 0.0 min 98% A → 1.0 min 98% A → 8.0 min 30 % A → 16.0 min 30 % A → 19.0 min 2% A - 20.0 min 2% A → 23.0 min 98% A → 25.0 min 30 % A, column temperature: 37°C; flow: 1.5 mL/min; UV-Detection: 214 nm, injection volume 10 µL

### Method 10 (HPLC, short column)

Instrument: HP 1260 Infinity HPLC System with G4212B diode array detector; column: Nucleodur 100 C18ec 3 µm 50 × 2 mm; eluent A: 1 L water + 1.0 mL TFA, eluent B: 1 L acetonitrile+ 1.0 mL TFA; gradient: 0.0 min 98% A (0.75 ml/min) → 1.0 min 98%A (0.75 ml/min) → 15.0 min 5 % A (0.75 ml/min) → 17.5 min 5 % A (0.75 ml/min) → 17.7 min 98% A (1.5 ml/min) → 18.2 min 98% A (1.5 ml/min) → 18.5 min 98% A (1.0 ml/min) → 19.0 min 98% A (0.75 ml/min), column temperature: 37°C; UV-Detection: 214 nm, injection volume 6 µL.

### Synthesis of Starting Materials

2-Chlor-5-{1-[2-chlor-4-(1,1,1,2,3,3,3-heptafluorpropan-2-yl)-6-(trifluormethoxy)phenyl]-1*H*-pyrazol-4-yl}-*N*-(1-cyanocyclopropyl)benzamide (CAS-RN 1771742-44-9) was synthesised as described in WO2015/067646 A1.

2-Chloro-*N-*(1-cyanocyclopropyl)-5-[2'-methyl-5'-(pentafluoroethyl)-4'-(trifluoromethyl)-2'*H*-[1,3'-bipyrazol]-4-yl]benzamide (CAS RN 1621436-41-6) was synthesised as described in WO2016/026789A1.

(2*E*)-4-*tert*-Butoxy-4-oxobut-2-enic acid (CAS RN 135355-96-3) was synthesised according to the method published by P.A. Clarke, R.L. Davie S. Peace, Tetrahedron Lett. 2002, 43, 2753-2756.

2-(*tert*-Butoxycarbonyl)cyclopropane-1-carboxylic acid (mixture of isomers, CAS RN 1378831-04-9 was prepared according to the method described in WO2008/123207A1.

2-Chloro-*N*-(1-cyanocyclopropyl)-5-[2'-methyl-5'-(pentafluoroethyl)-4'-(trifluoromethyl)-2'*H*-[1,3'-bipyrazol]-4-yl]benzamide (CAS-RN 1621436-41-6) was prepared according to procedures described in WO 2014/122083 A1, WO 2015/078846 A1 and WO2015/181139A1.

4-*tert*-Butoxy-3,3-dimethyl-4-oxobutanoic acid (CAS RN 660423-00-7) was prepared according to a method described in WO2004/014881A1.

### Synthesis of Intermediates

### Intermediate 1A

### tert-Butyl chloromethyl cyclopropane-1,2-dicarboxylate (mixture of isomers)

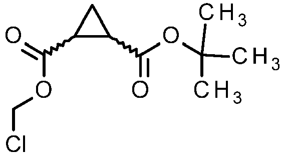

(*tert*-Butoxycarbonyl)cyclopropane-1-carboxylic acid (mixture of isomers, CAS RN 1378831-04-9, 279 mg, 1.50 mmol) and sodium hydrogen carbonate (504 mg, 6.0 mmol) were dissolved in a mixture of DCM (6.0 mL) and water (6.0 mL), tetrabutylammonium hydrogen sulfate (50.9 mg, 150 µmol) was added and the mixture was stirred at 0 °C for 10 min. Then, a solution of chloromethyl sulfurochloridate (200 µl, 2.0 mmol) in dichloromethane (5.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to yield 302 mg (100 % purity, 86 % yield) of the title compound.

¹H-NMR (600 MHz, DMSO-d⁶) δ [ppm]: 1.393 (0.81), 1.411 (16.00), 1.418 (1.02), 5.841 (0.42), 5.852 (1.72), 5.858 (1.72), 5.868 (0.42).

### Intermediate 2A

### tert-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl cyclopropane-1,2-dicarboxylate (mixture of isomers)

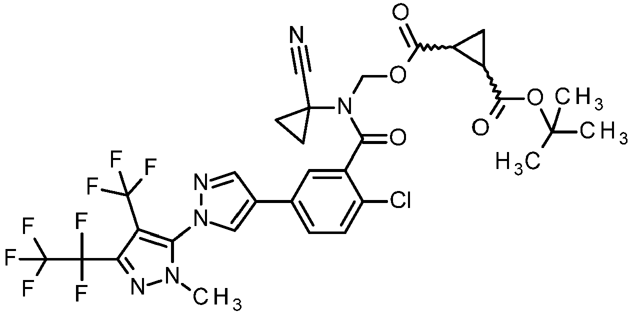

2-Chloro-*N-*(1-cyanocyclopropyl)-5-[2'-methyl-5'-(pentafluoroethyl)-4'-(trifluoromethyl)-2'*H*-[1,3'-bipyrazol]-4-yl]benzamide (138 mg, 250 µmol) was dissolved in dry THF (4.0 mL). This solution was cooled to -45°C on an acetone - dry ice bath. Potassium bis(trimethylsilyl)amide (800 µL, 0.50 M solution in toluene, 400 µmol) was added dropwise and the mixture was stirred 30 min. at -45 °C. Then, sodium iodide (19 mg, 125 µmol) and a solution of *tert*-butyl chloromethyl cyclopropane-1,2-dicarboxylate (intermediate 1A, 88.0 mg, 375 µmol) in THF (4.0 mL) was added, the dry ice bath was removed and the mixture was stirred at room temperature for 60 min. The solvent was distilled and the residue was purified by preparative HPLC (RP C-18 10 µm water-acetonitrile gradient with 0.01 % TFA in both eluents, 90: 10->5:95) to give 132 mg (100 % purity, 70 % yield) of the title compound.

LC-MS Method 4): Rₜ = 4.87 min; MS (ESIpos): m/z = 695 [M+M-C4H8]+⁺

¹H-NMR (600 MHz, DMSO-d⁶) δ [ppm]: 1.383 (16.00), 1.405 (0.43), 1.756 (0.92), 1.958 (0.47), 3.824 (9.34), 5.747 (1.04), 7.847 (0.71), 7.858 (1.42), 8.567 (1.04), 8.832 (0.97).

### Intermediate 3A

### 1-tert-Butyl 4-(chloromethyl) 2,2-dimethylbutanedioate

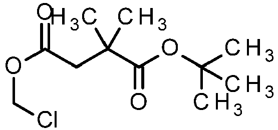

4-*tert*-Butoxy-3,3-dimethyl-4-oxobutanoic acid (CAS RN 660423-00-7, 600 mg, 2.97 mmol) and sodium hydrogen carbonate (997 mg, 11.9 mmol) were dissolved in a mixture of dichloromethane (18 mL) and water (18 mL), tetrabutylammonium hydrogen sulfate (101 mg, 297 µmol) was added and the mixture was stirred at 0 °C for 10 min. Then, a solution of chloromethyl sulfurochloridate (410 µl, 4.0 mmol) in dichloromethane (5.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to yield 488 mg (100 % purity, 66 % yield) of the title compound.

¹H-NMR (400 MHz, DMSO-d⁶) δ [ppm]: 1.161 (9.39), 1.189 (0.68), 1.372 (16.00), 2.624 (3.10), 5.836 (3.78).

### Intermediate 4A

### 1-tert-Butyl 4-{[{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyll 2,2-dimethylbutanedioate

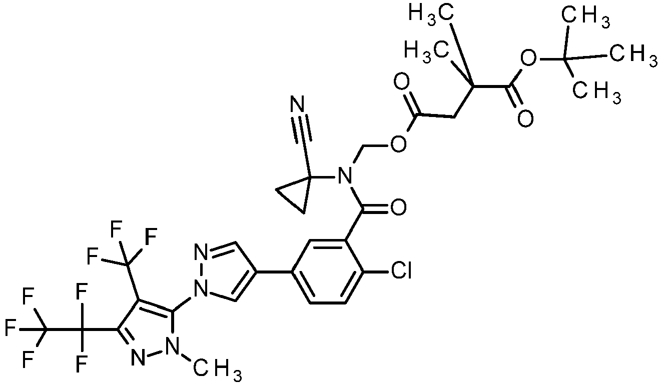

2-Chloro-*N*-(1-cyanocyclopropyl)-5-[2'-methyl-5'-(pentafluoroethyl)-4'-(trifluoromethyl)-2'*H*-[1,3'-bipyrazol]-4-yl]benzamide (138 mg, 250 µmol) was dissolved in dry THF (4.0 mL). This solution was cooled to -45°C on an acetone - dry ice bath. Potassium bis(trimethylsilyl)amide (800 µL, 0.50 M solution in toluene, 400 µmol) was added dropwise and the mixture was stirred 30 min. at -45 °C. Then, sodium iodide (19 mg, 125 µmol) and a solution of 1-*tert*-butyl 4-(chloromethyl) 2,2-dimethylbutanedioate (intermediate 3A, 94.0 mg, 375 µmol) in THF (4.0 mL) was added, the dry ice bath was removed and the mixture was stirred at room temperature for 60 min. The solvent was distilled and the residue was purified by preparative HPLC (RP C-18 10 µm water-acetonitrile gradient with 0.01 % TFA in both eluents, 90: 10->5:95) to give 112 mg (100 % purity, 58 % yield) of the title compound.

LC-MS Method 4): Rₜ = 5.05 min; MS (ESIpos): m/z = 711 [M+H-C4H8]+⁺

### Intermediate 5A

### tert-Butyl chloromethyl benzene-1,4-dicarboxylate

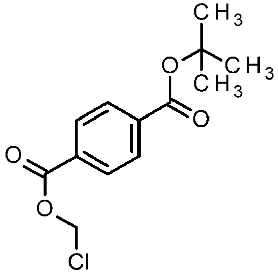

4-(*tert*-Butoxycarbonyl)benzoic acid (CAS RN 20576-82-3, 222 mg, 1.00 mmol) and sodium hydrogen carbonate (336 mg, 4.0 mmol) were dissolved in a mixture of dichloromethane (6.0 mL) and water (6.0 mL), tetrabutylammonium hydrogen sulfate (34.0 mg, 100 µmol) was added and the mixture was stirred at 0 °C for 10 min. Then, a solution of chloromethyl sulfurochloridate (140 µl, 1.4 mmol) in dichloromethane (1.5 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to yield 249 mg (100 % purity, 92 % yield)

¹H-NMR (400 MHz, DMSO-d⁶) δ [ppm]: 1.569 (16.00), 6.131 (3.99), 8.052 (0.78), 8.069 (0.48), 8.074 (1.85), 8.111 (1.75), 8.116 (0.47), 8.133 (0.83).

### Intermediate 6A

### tert-Butyl [(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl benzene-1,4-dicarboxylate

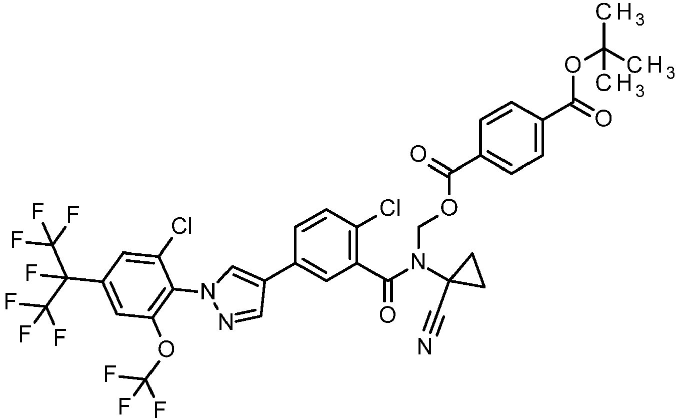

2-Chloro-5-{1-[2-chloro-4-(1,1,1,2,3,3,3-heptafluoropropan-2-yl)-6-(trifluoromethoxy)phenyl]-1*H-*pyrazol-4-yl}-*N*(1-cyanocyclopropyl)benzamide (162 mg, 250 µmol) was dissolved in dry THF (4.0 mL). This solution was cooled to -45°C on an acetone - dry ice bath. Potassium bis(trimethylsilyl)amide (800 µL, 0.50 M solution in toluene, 400 µmol) was added dropwise and the mixture was stirred 30 min. at -45 °C. Then, sodium iodide (19 mg, 125 µmol) and a solution of *tert*-butyl chloromethyl benzene-1,4-dicarboxylate (intermediate 5A, 102 mg, 375 µmol) in THF (4.0 mL) was added, the dry ice bath was removed and the mixture was stirred at room temperature for 60 min. The solvent was distilled and the residue was purified by preparative HPLC (RP C-18 10 µm water-acetonitrile gradient with 0.01 % TFA in both eluents, 90: 10->5:95) to give 167 mg (100 % purity, 76 % yield)

LC-MS Method 4): Rt = 5.54 min; MS (ESIpos): m/z = 883 [M+H]+

¹H-NMR (400 MHz, DMSO-d⁶) δ [ppm]: -0.008 (1.45), 0.008 (1.64), 1.234 (0.61), 1.356 (1.75), 1.537 (16.00), 1.560 (0.94), 1.803 (0.91), 7.592 (0.49), 7.614 (0.61), 7.801 (0.58), 7.821 (0.52), 7.866 (0.89), 7.923 (1.35), 7.966 (0.77), 7.986 (1.33), 8.033 (1.25), 8.053 (0.80), 8.191 (1.52), 8.195 (1.49), 8.384 (2.08), 8.748 (1.68).

### Intermediate 7A

### tert-Butyl chloromethyl pentanedioate

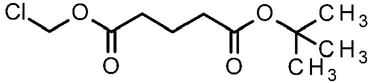

5-tert-butoxy-5-oxopentanoic acid (CAS RN 63128-51-8, 400 mg, 2.13 mmol) and sodium hydrogen carbonate (714 mg, 8.5 mmol) were dissolved in a mixture of dichloromethane (20 mL) and water (20 mL), tetrabutylammonium hydrogen sulfate ((72 mg, 213 µmol) was added and the mixture was stirred at 0 °C for 10 min. Then, a solution of chloromethyl sulfurochloridate (290 µl, 2.9 mmol) in dichloromethane (5.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to give 379 mg (75 % yield) of the title compound.

¹H-NMR (600 MHz, DMSO-d⁶) δ [ppm]: 1.390 (0.50), 1.398 (16.00), 1.739 (0.73), 1.751 (1.08), 1.763 (0.76), 2.238 (0.86), 2.250 (1.56), 2.262 (0.77), 2.430 (0.84), 2.442 (1.56), 2.455 (0.78), 5.839 (3.82).

### Intermediate 8A

### tert-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl pentanedioate

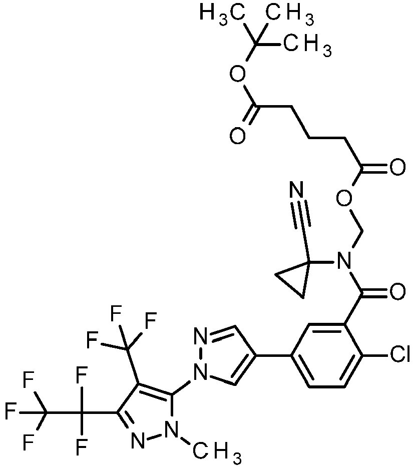

2-Chloro-*N-*(1-cyanocyclopropyl)-5-[2'-methyl-5'-(pentafluoroethyl)-4'-(trifluoromethyl)-2'*H*-[1,3'-bipyrazol]-4-yl]benzamide (187 mg, 338 µmol) was dissolved in dry THF (6.0 mL). This solution was cooled to -45°C on an acetone - dry ice bath. Potassium bis(trimethylsilyl)amide (1.0 ml, 540 µmol, 0.50 M solution in toluene, 507 µmol) was added dropwise and the mixture was stirred 30 min. at -45 °C. Then, sodium iodide (25.3 mg, 169 µmol) and a solution of tert-butyl chloromethyl pentanedioate (intermediate 7A, 120 mg, 507 µmol) in THF (4.0 mL) was added, the dry ice bath was removed and the mixture was stirred at room temperature for 4 h. The solvent was distilled and the residue was purified by preparative HPLC (RP C-18 10 µm water-acetonitrile gradient with 0.01 % TFA in both eluents, 90: 10->5:95) to give 102 mg (40 % yield) of the title compound.

LC-MS Method 4): Rₜ = 4.79 min; MS (ESIneg): m/z = 751 [M-H]⁻

¹H-NMR (400 MHz, DMSO-d⁶) δ [ppm]: 1.235 (0.55), 1.361 (16.00), 1.386 (0.81), 1.651 (0.56), 1.670 (0.76), 1.688 (0.58), 1.750 (0.80), 2.154 (0.53), 2.172 (0.97), 2.190 (0.52), 2.312 (0.48), 2.330 (0.86), 2.348 (0.44), 2.524 (0.47), 3.823 (5.90), 5.754 (0.45), 7.610 (0.50), 7.630 (0.61), 7.829 (0.81), 7.842 (0.65), 7.863 (0.50), 8.571 (1.04), 8.839 (1.02).

### Intermediate 9A

### 1-tert-Butyl 1-(chloromethyl) cyclopropane-1,1-dicarboxylate

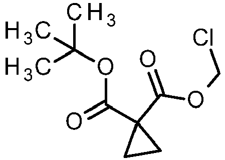

1-(*tert*-Butoxycarbonyl)cyclopropane-1-carboxylic acid (CAS RN 1268842-79-0, 541 mg, 2.90 mmol) and sodium hydrogen carbonate (976 mg, 11.6 mmol) were dissolved in a mixture of dichloromethane (18 mL) and water (18 mL). Tetrabutylammonium hydrogen sulfate (98.6 mg, 290 µmol) was added and the mixture was stirred at 0 °C for 10 min. Then, a solution of chloromethyl sulfurochloridate (400 µl, 3.9 mmol) in dichloromethane (2.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to give 428 mg (63 % yield) of the title compound.

¹H-NMR (600 MHz, DMSO-d⁶) δ [ppm]: 1.172 (0.73), 1.366 (5.60), 1.418 (16.00), 5.893 (3.55).

### Intermediate 10A

### 1-tert-Butyl 1-{[{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl} cyclopropane-1,1-dicarboxylate

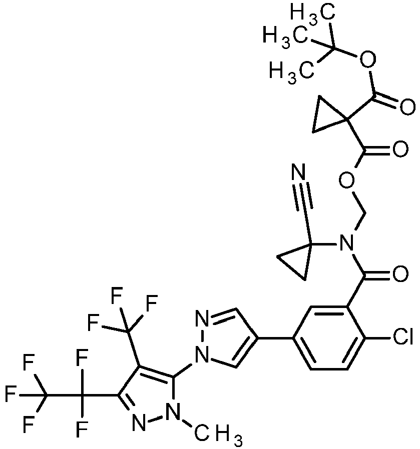

2-Chloro-*N*-(1-cyanocyclopropyl)-5-[2'-methyl-5'-(pentafluoroethyl)-4'-(trifluoromethyl)-2'*H*-[1,3'-bipyrazol]-4-yl]benzamide (188 mg, 341 µmol) was dissolved in dry THF (6.0 mL). This solution was cooled to -45°C on an acetone - dry ice bath. Potassium bis(trimethylsilyl)amide (1.0 ml, 540 µmol, 0.50 M solution in toluene, 507 µmol) was added dropwise and the mixture was stirred 30 min. at -45 °C. Then, sodium iodide (25 mg, 169 µmol) and a solution of 1-tert-butyl 1-(chloromethyl) cyclopropane-1,1-dicarboxylate (intermediate 9A, 120 mg, 511 µmol) in THF (6.0 mL) was added, the dry ice bath was removed and the mixture was stirred at room temperature for 3 h. The solvent was distilled and the residue was purified by preparative HPLC (RP C-18 10 µm water-acetonitrile gradient with 0.01 % TFA in both eluents, 90: 10->5:95) to give 130 mg (51 % yield) of the title compound.

LC-MS Method 4): Rₜ = 4.80 min; MS (ESIneg): m/z = 749 [M-H]⁻

¹H-NMR (400 MHz, DMSO-d⁶) δ [ppm]: -0.023 (0.52), -0.008 (0.66), 0.008 (0.66), 1.235 (1.13), 1.283 (3.27), 1.290 (3.17), 1.339 (16.00), 1.356 (2.76), 1.416 (1.63), 1.758 (1.35), 2.073 (7.32), 3.820 (12.13), 7.622 (0.89), 7.643 (1.02), 7.809 (1.28), 7.842 (0.94), 7.864 (0.81), 8.546 (1.95), 8.818 (1.83).

### Intermediate 11A

### tert-Butyl chloromethyl butanedioate

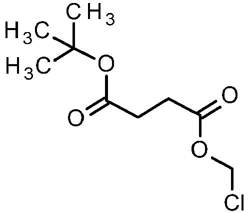

4-*tert*-Butoxy-4-oxobutanoic acid (CAS RN 15026-17-2, 697 mg, 4.00 mmol) and sodium hydrogen carbonate (1.34 g, 16.0 mmol) were dissolved in a mixture of dichloromethane (31 mL) and water (31 mL). Tetrabutylammonium hydrogen sulfate (136 mg, 400 µmol) was added and the mixture was stirred at 0 °C for 10 min. Then, a solution of chloromethyl sulfurochloridate (550 µl, 5.4 mmol) in dichloromethane (4.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to give 750 mg (100 % purity, 84 % yield) of the title compound.

¹H-NMR (600 MHz, DMSO-d⁶) δ [ppm]: 1.389 (16.00), 2.483 (0.59), 2.504 (1.23), 2.597 (0.91), 2.605 (0.57), 2.609 (0.96), 2.619 (0.56), 5.844 (3.89).

### Intermediate 12A

### tert-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl butanedioate

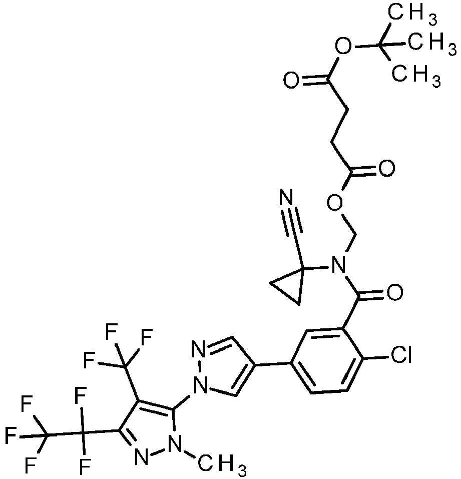

2-Chloro-*N*-(1-cyanocyclopropyl)-5-[2'-methyl-5'-(pentafluoroethyl)-4'-(trifluoromethyl)-2'*H*-[1,3'-bipyrazol]-4-yl]benzamide (227 mg, 410 µmol) was dissolved in dry THF (7.0 mL). This solution was cooled to -45°C on an acetone - dry ice bath. Potassium bis(trimethylsilyl)amide (1.3 ml, 0.50 M solution in toluene, 660 µmol) was added dropwise and the mixture was stirred 30 min. at -45 °C. Then, sodium iodide (31 mg, 205 µmol) and a solution of *tert*-Butyl chloromethyl butanedioate (intermediate 11A, 147 mg, 615 µmol) in THF (7.0 mL) was added, the dry ice bath was removed and the mixture was stirred at room temperature for 2 h. The solvent was distilled and the residue was purified by preparative HPLC (RP C-18 10 µm water-acetonitrile gradient with 0.01 % TFA in both eluents, 90:10->5:95) to give 189 mg (100 % purity, 62 % yield) of the title compound.

LC-MS Method 4): Rₜ = 4.70 min; MS (ESIpos): m/z = 683 [M+H]⁺

¹H-NMR (400 MHz, DMSO-d⁶) δ [ppm]: -0.008 (0.79), 0.008 (0.84), 1.235 (0.45), 1.330 (16.00), 1.367 (1.07), 1.380 (0.87), 1.418 (0.87), 1.748 (1.06), 2.073 (2.95), 2.411 (1.32), 2.427 (1.06), 3.823 (9.89), 7.611 (0.68), 7.632 (0.83), 7.842 (2.41), 7.863 (0.83), 8.575 (1.77), 8.838 (1.68).

### Intermediate 13A

### tert-Butyl chloromethyl (2E)-but-2-enedioate

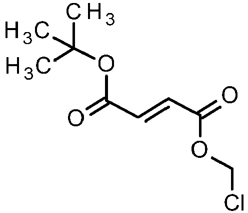

(2*E*)-4-*tert*-butoxy-4-oxobut-2-enoic acid (CAS RN 135355-96-3, 1.72 g, 10.0 mmol) and sodium hydrogen carbonate (3.36 g, 40.0 mmol) were dissolved in a mixture of dichloromethane (62 mL) and water (62 mL). Tetrabutylammonium hydrogen sulfate (340 mg, 1000 µmol) was added and the mixture was stirred at 0 °C for 10 min. Then, a solution of chloromethyl sulfurochloridate (1.4 ml, 13 mmol) in dichloromethane (7.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to give 2.05 g (100 % purity, 93 % yield) of the title compound.

LC-MS Method 1): Rₜ = 1.34 min; MS (ESIpos): m/z = 165 [M+H-C4H8]⁺

¹H-NMR (600 MHz, DMSO-d⁶) δ [ppm]: 1.469 (16.00), 5.970 (4.04), 6.723 (0.51), 6.750 (1.50), 6.775 (1.54), 6.801 (0.50).

### Intermediate 14A

### tert-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl (2E)-but-2-enedioate

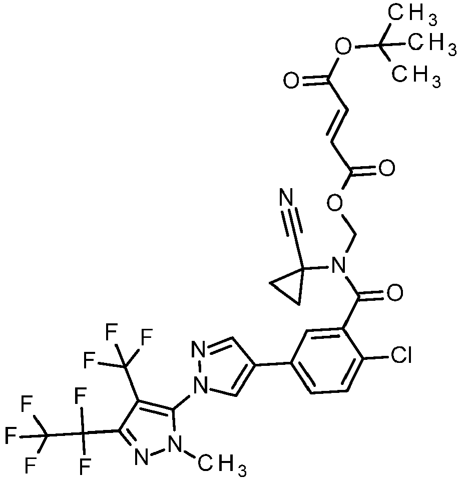

2-Chloro-*N*-(1-cyanocyclopropyl)-5-[2'-methyl-5'-(pentafluoroethyl)-4'-(trifluoromethyl)-2'H-[1,3'-bipyrazol]-4-yl]benzamide (224 mg, 405 µmol) was dissolved in dry THF (7.0 mL). This solution was cooled to -45°C on an acetone - dry ice bath. Potassium bis(trimethylsilyl)amide (1.3 ml, 0.50 M solution in toluene, 660 µmol) was added dropwise and the mixture was stirred 30 min. at -45 °C. Then, sodium iodide (31 mg, 205 µmol) and a solution of *tert*-butyl chloromethyl (2*E*)-but-2-enedioate (intermediate 13A, 134 mg, 608 µmol) in THF (7.0 mL) was added, the dry ice bath was removed and the mixture was stirred at room temperature for 2 h. The solvent was distilled and the residue was purified by preparative HPLC (RP C-18 10 µm water-acetonitrile gradient with 0.01 % TFA in both eluents, 90: 10->5:95) to give 180 mg (100 % purity, 60 % yield) of the title compound.

LC-MS Method 4): Rₜ = 4.84 min; MS (ESIpos): m/z = 736 [M+H]⁺

¹H-NMR (400 MHz, DMSO-d⁶) δ [ppm]: -0.008 (1.21), 0.008 (1.33), 1.235 (0.61), 1.433 (16.00), 1.445 (4.03), 1.456 (1.86), 1.777 (1.03), 2.524 (0.63), 3.820 (10.03), 6.612 (0.90), 6.675 (0.92), 6.714 (0.46), 7.619 (0.72), 7.640 (0.80), 7.852 (0.78), 7.873 (0.70), 7.901 (1.24), 8.558 (2.06), 8.828 (1.62).

### Intermediate 15A

### tert-Butyl chloromethyl butanedioate

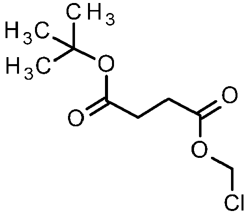

4-*tert-*Butoxy-4-oxobutanoic acid (CAS RN 15026-17-2, 697 mg, 4.00 mmol) and sodium hydrogen carbonate (1.34 g, 16.0 mmol) were dissolved in a mixture of dichloromethane (31 mL) and water (31 mL). Tetrabutylammonium hydrogen sulfate (136 mg, 400 µmol) was added and the mixture was stirred at 0 °C for 10 min. Then, a solution of chloromethyl sulfurochloridate (550 µl, 5.4 mmol) in dichloromethane (4.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to give 750 mg (100 % purity, 84 % yield) of the title compound.

¹H-NMR (600 MHz, DMSO-d⁶) δ [ppm]: 1.389 (16.00), 2.483 (0.59), 2.504 (1.23), 2.597 (0.91), 2.605 (0.57), 2.609 (0.96), 2.619 (0.56), 5.844 (3.89).

### Intermediate 16A

### tert-Butyl [(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl butanedioate

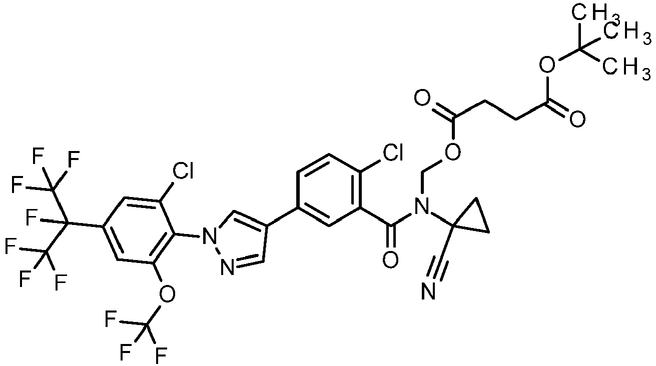

2-Chloro-5-{1-[2-chloro-4-(1,1,1,2,3,3,3-heptafluoropropan-2-yl)-6-(trifluoromethoxy)phenyl]-1*H-*pyrazol-4-yl}-*N*-(1-cyanocyclopropyl)benzamide (649 mg, 1.00 mmol) was dissolved in dry THF (16 mL). This solution was cooled to -45°C on an acetone - dry ice bath. Potassium bis(trimethylsilyl)amide (3.2 ml, 0.50 M solution in toluene, 1.6 mmol) was added dropwise and the mixture was stirred 30 min. at -45 °C. Then, sodium iodide (74.9 mg, 500 µmol) and a solution of tert-butyl chloromethyl butanedioate (intermediate 15A, 334 mg, 1.50 mmol) in THF (16 mL) was added, the dry ice bath was removed and the mixture was stirred at room temperature for 40 min. The solvent was distilled and the residue was purified by preparative HPLC (RP C-18 10 µm water-acetonitrile gradient with 0.01 % TFA in both eluents, 90: 10->5:95) to give 520 mg (62 % yield) of the title compound.

LC-MS Method 4): Rₜ = 5.13 min; MS (ESIpos): m/z = 835 [M+H]⁺

¹H-NMR (500 MHz, DMSO-d⁶) δ [ppm]: -0.007 (1.44), 0.007 (0.95), 1.327 (16.00), 1.357 (4.14), 1.377 (1.34), 1.746 (0.90), 2.185 (0.45), 2.412 (1.39), 2.425 (1.02), 3.352 (11.10), 3.361 (8.28), 7.593 (0.76), 7.609 (0.87), 7.806 (2.19), 7.824 (0.76), 7.946 (1.67), 8.219 (1.90), 8.222 (1.86), 8.464 (1.81), 8.783 (1.67).

### Intermediate 17A

### tert-Butyl [(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl pentanedioate

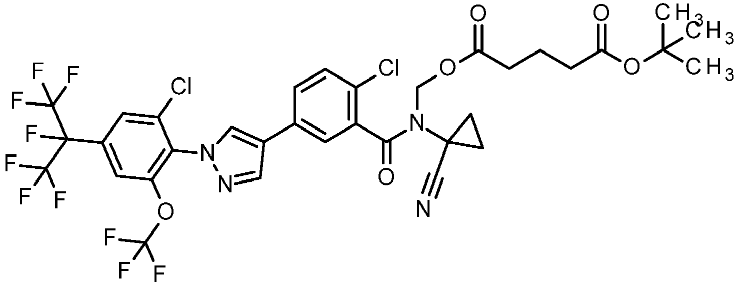

2-Chloro-5-{1-[2-chloro-4-(1,1,1,2,3,3,3-heptafluoropropan-2-yl)-6-(trifluoromethoxy)phenyl]-1H-pyrazol-4-yl}-N-(1-cyanocyclopropyl)benzamide (162 mg, 250 µmol) was dissolved in dry THF (4.0 mL). This solution was cooled to -45°C on an acetone - dry ice bath. Potassium bis(trimethylsilyl)amide (800 µl 0.50 M solution in toluene, 400 µmol) was added dropwise and the mixture was stirred 30 min. at -45 °C. Then, sodium iodide (19 mg, 125 µmol) and a solution of *tert*-butyl chloromethyl pentanedioate (intermediate 7A, 88.8 mg, 375 µmol) in THF (4.0 mL) was added, the dry ice bath was removed and the mixture was stirred at room temperature for 40 min. The solvent was distilled and the residue was purified by preparative HPLC (RP C-18 10 µm water-acetonitrile gradient with 0.01 % TFA in both eluents, 90: 10->5:95) to give 171 mg (81 % yield) of the title compound.

LC-MS Method 4): Rₜ = 5.21 min; MS (ESIpos): m/z = 849 [M+H]⁺

¹H-NMR (400 MHz, DMSO-d⁶) δ [ppm]: 1.358 (16.00), 1.378 (0.50), 1.676 (0.44), 1.744 (0.47), 2.170 (0.55), 2.328 (0.45), 7.787 (0.45), 7.938 (0.63), 8.210 (0.72), 8.214 (0.70), 8.454 (0.57), 8.775 (0.54).

### Intermediate 18A

### tert-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl benzene-1,4-dicarboxylate

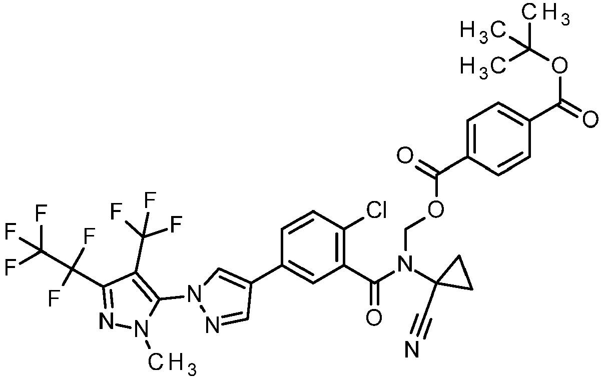

2-Chloro-*N*-(1-cyanocyclopropyl)-5-[2'-methyl-5'-(pentafluoroethyl)-4'-(trifluoromethyl)-2'*H*-[1,3'-bipyrazol]-4-yl]benzamide (138 mg, 250 µmol) was dissolved in dry THF (4.0 mL). This solution was cooled to -45°C on an acetone - dry ice bath. Potassium bis(trimethylsilyl)amide (800 µl 0.50 M solution in toluene, 400 µmol) was added dropwise and the mixture was stirred 30 min. at -45 °C. Then, sodium iodide (19 mg, 125 µmol) and a solution of *tert*-butyl chloromethyl benzene-1,4-dicarboxylate (intermediate 5A, 102 mg, 375 µmol) in THF (4.0 mL) was added, the dry ice bath was removed and the mixture was stirred at room temperature for 2 h. The solvent was distilled and the residue was purified by preparative HPLC (RP C-18 10 µm water-acetonitrile gradient with 0.01 % TFA in both eluents, 90:10->5:95) to give 157 mg (100 % purity, 80 % yield) of the title compound.

LC-MS (Method 4): Rₜ = 5.02 min; MS (ESIpos): m/z = 787 [M+H]⁺

¹H-NMR (600 MHz, DMSO-d⁶) δ [ppm]: 1.359 (2.27), 1.545 (16.00), 1.562 (0.77), 1.812 (0.96), 2.072 (7.74), 3.788 (4.47), 7.617 (0.48), 7.631 (0.55), 7.840 (0.53), 7.854 (0.50), 7.883 (0.85), 7.979 (0.79), 7.992 (1.23), 8.033 (1.15), 8.046 (0.82), 8.488 (1.60), 8.785 (3.02).

### Intermediate 19A

### tert-Butyl methyl (trans)-cyclohexane-1,4-dicarboxylate

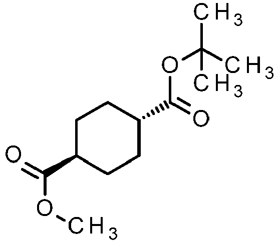

(*trans*)-4-(methoxycarbonyl)cyclohexane-1-carboxylic acid (CAS RN 15177-67-0, 1.12 g, 6.00 mmol) was dissolved in toluene (30 mL) and 1,1-di-*tert*-butoxy-*N*,*N*-dimethylmethanamine (5.8 mL, 24 mmol) was slowly added over a period of 7 h at 80 °C. Stirring at 80 °C was maintained for 18 h, the mixture was diluted with ethyl acetate, washed with water and brine, dried over anhydrous sodium sulfate, filtered and evaporated. The crude product was purified by chromatography on silica gel (cyclohexane / ethyl acetate 5: 1) to give 1.23 g (100 % purity, 85 % yield) of the title compound.

¹H-NMR (600 MHz, DMSO-d⁶) δ [ppm]: 1.310 (0.95), 1.329 (0.97), 1.382 (16.00), 1.877 (0.52), 1.890 (0.54), 3.342 (5.51).

### Intermediate 20A

### (trans)-4-(tert-Butoxycarbonyl)cyclohexane-1-carboxylic acid

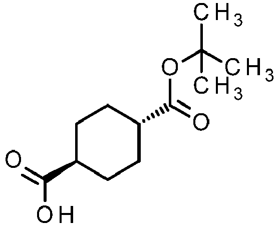

*tert*-Butyl methyl (*trans*)-cyclohexane-1,4-dicarboxylate (intermediate 19A, 1.29 g, 5.32 mmol) was dissolved in a mixture of methanol (25 mL) and water (15 mL), potassium hydroxide (1.51 g, 27.0 mmol) was added and the mixture was stirred at room temperature for 1 h. Then, pH was adjusted to 4 by adding 1M hydrochloric acid., the mixture was diluted with water and extracted with three portions of MTBE. The combined organic extracts were dried over anhydrous sodium sulfate, filtered and evaporated. The title compound (1.15 g, 100 % purity, 95 % yield) was obtained as a colourless solid.

¹H-NMR (500 MHz, DMSO-d⁶) δ [ppm]: 1.286 (0.48), 1.296 (0.61), 1.306 (0.48), 1.380 (16.00), 1.872 (0.43), 1.884 (0.46).

### Intermediate 21A

### tert-Butyl chloromethyl (trans)-cyclohexane-1,4-dicarboxylate

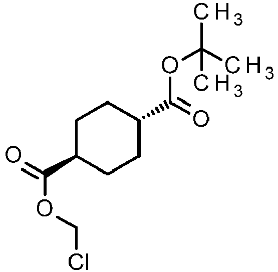

(*trans*)-4-(*tert*-Butoxycarbonyl)cyclohexane-1-carboxylic acid (intermediate 20A, 530 mg, 2.32 mmol) and sodium hydrogen carbonate (780 mg, 9.29 mmol) were dissolved in a mixture of dichloromethane (14 mL) and water (14 mL). Tetrabutylammonium hydrogen sulfate (79 mg, 232 µmol) was added and the mixture was stirred at 0 °C for 10 min. Then, a solution of chloromethyl sulfurochloridate (320 µl, 3.1 mmol) in dichloromethane (4.0 mL) was added dropwise. The ice bath was removed and stirring was maintained for 16 h. Dichloromethane was added and the phases were separated. The organic phase was washed with brine, dried over anhydrous sodium sulfate and the solvent was distilled. The crude product was purified by chromatography on silica gel with a gradient of cyclohexane - ethyl acetate to give 490 mg (100 % purity, 76 % yield) of the title compound.

¹H-NMR (500 MHz, DMSO-d⁶) δ [ppm]: 1.313 (0.40), 1.337 (1.12), 1.357 (1.20), 1.385 (16.00), 1.876 (0.46), 1.886 (0.43), 1.896 (0.64), 1.913 (0.67), 1.922 (0.41), 1.933 (0.42), 5.847 (3.70).

### Intermediate 22A

### tert-Butyl [(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl (1r,4r)-cyclohexane-1,4-dicarboxylate

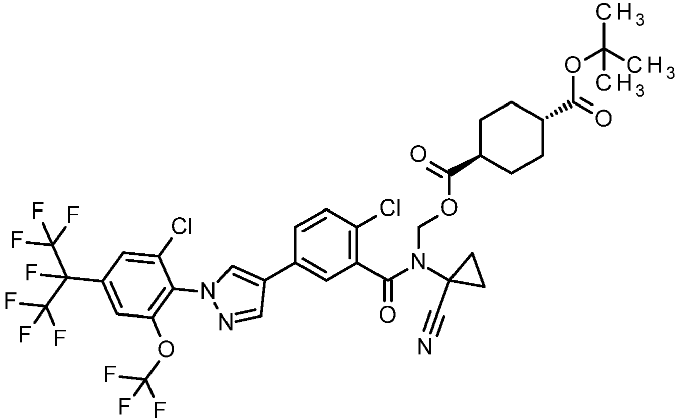

2-Chloro-5-{1-[2-chloro-4-(1,1,1,2,3,3,3-heptafluoropropan-2-yl)-6-(trifluoromethoxy)phenyl]-1H-pyrazol-4-yl}-*N*-(1-cyanocyclopropyl)benzamide (162 mg, 250 µmol) was dissolved in dry THF (4.0 mL). This solution was cooled to -45°C on an acetone - dry ice bath. Potassium bis(trimethylsilyl)amide (800 µl 0.50 M solution in toluene, 400 µmol) was added dropwise and the mixture was stirred 30 min. at -45 °C. Then, sodium iodide (19 mg, 125 µmol) and a solution of tert-butyl chloromethyl (*trans*)-cyclohexane-1,4-dicarboxylate (intermediate 21A, , 104 mg, 375 µmol) in THF (4.0 mL) was added, the dry ice bath was removed and the mixture was stirred at room temperature for 40 min. The solvent was distilled and the residue was purified by preparative HPLC (RP C-18 10 µm water-acetonitrile gradient with 0.01 % TFA in both eluents, 90:10->5:95) to give 204 mg (100 % purity, 92 % yield) of the title compound.

LC-MS Method 4): Rₜ = 5.47 min; MS (ESIneg): m/z = 887 [M-H]⁻

¹H-NMR (400 MHz, DMSO-d⁶) δ [ppm]: 1.235 (1.31), 1.248 (1.16), 1.355 (16.00), 1.750 (0.83), 1.812 (0.66), 1.858 (0.76), 2.524 (0.46), 3.476 (0.99), 7.591 (0.57), 7.612 (0.69), 7.784 (1.02), 7.798 (0.81), 7.819 (0.59), 7.927 (1.28), 8.199 (1.42), 8.203 (1.38), 8.478 (1.21), 8.797 (1.17).

### Intermediate 23A

### tert-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl (trans)-cyclohexane-1,4-dicarboxylate

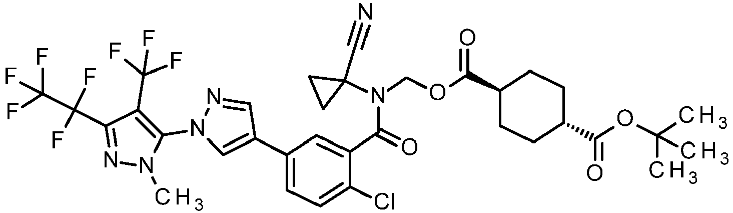

2-Chloro-*N*-(1-cyanocyclopropyl)-5-[2'-methyl-5'-(pentafluoroethyl)-4'-(trifluoromethyl)-2'*H*-[1,3'-bipyrazol]-4-yl]benzamide (138 mg, 250 µmol) was dissolved in dry THF (4.0 mL). This solution was cooled to -45°C on an acetone - dry ice bath. Potassium bis(trimethylsilyl)amide (800 µl 0.50 M solution in toluene, 400 µmol) was added dropwise and the mixture was stirred 30 min. at -45 °C. Then, sodium iodide (19 mg, 125 µmol) and a solution of tert-butyl chloromethyl (*trans*)-cyclohexane-1,4-dicarboxylate (intermediate 21A, 104 mg, 375 µmol) in THF (4.0 mL) was added, the dry ice bath was removed and the mixture was stirred at room temperature for 40 min. The solvent was distilled and the residue was purified by preparative HPLC (RP C-18 10 µm water-acetonitrile gradient with 0.01 % TFA in both eluents, 90: 10->5:95) to give 157 mg (79 % yield) of the title compound.

LC-MS Method 4): Rₜ = 5.11 min; MS (ESIpos): m/z = 737 [M+H-C2H8]+⁺

¹H-NMR (600 MHz, DMSO-d⁶) δ [ppm]: 1.236 (0.83), 1.251 (1.11), 1.360 (16.00), 1.383 (0.62), 1.457 (0.43), 1.753 (0.69), 1.806 (0.57), 1.857 (0.67), 2.070 (1.13), 2.183 (0.52), 3.361 (0.44), 3.819 (9.21), 7.618 (0.59), 7.632 (0.69), 7.810 (0.92), 7.840 (0.61), 7.854 (0.58), 8.587 (1.20), 8.849 (1.16).

### Intermediate 24A

### tert-Butyl [(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl (2E)-but-2-enedioate

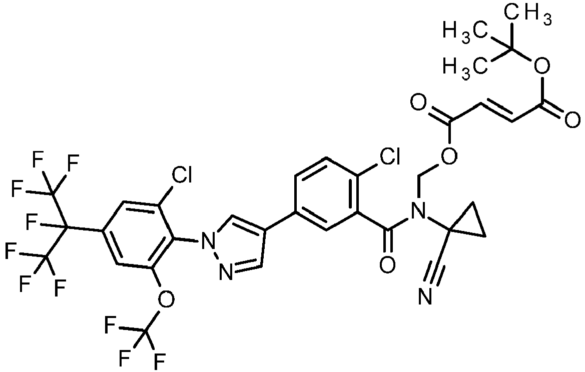

2-Chloro-5-{1-[2-chloro-4-(1,1,1,2,3,3,3-heptafluoropropan-2-yl)-6-(trifluoromethoxy)phenyl]-1H-pyrazol-4-yl}-*N*-(1-cyanocyclopropyl)benzamide (162 mg, 250 µmol) was dissolved in dry THF (4.0 mL). This solution was cooled to -45°C on an acetone - dry ice bath. Potassium bis(trimethylsilyl)amide (800 µl 0.50 M solution in toluene, 400 µmol) was added dropwise and the mixture was stirred 30 min. at -45 °C. Then, sodium iodide (19 mg, 125 µmol) and a solution of tert-butyl chloromethyl (2E)-but-2-enedioate (intermediate 13A, 83 mg, 375 µmol) in THF (4.0 mL) was added, the dry ice bath was removed and the mixture was stirred at room temperature for 40 min. The solvent was distilled and the residue was purified by preparative HPLC (RP C-18 10 µm water-acetonitrile gradient with 0.01 % TFA in both eluents, 90:10->5:95) to give 165 mg (100 % purity, 79 % yield) of the title compound.

LC-MS Method 4): Rₜ = 5.30 min; MS (ESIpos): m/z = 833 [M+H]⁺

¹H-NMR (400 MHz, DMSO-d⁶) δ [ppm]: 1.356 (3.45), 1.427 (16.00), 1.769 (1.02), 2.184 (0.43), 6.631 (0.87), 6.687 (0.86), 6.726 (0.42), 7.595 (0.64), 7.616 (0.73), 7.811 (0.80), 7.831 (0.69), 7.871 (1.18), 7.927 (1.75), 8.200 (1.97), 8.204 (1.94), 8.437 (2.38), 8.765 (1.44).

### Intermediate 25A

### 1-tert-Butyl 4-1[(2-chloro-5-11-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl} 2,2-dimethylbutanedioate

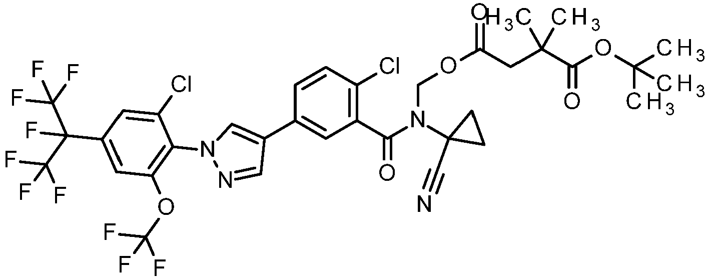

2-Chloro-5-{1-[2-chloro-4-(1,1,1,2,3,3,3-heptafluoropropan-2-yl)-6-(trifluoromethoxy)phenyl]-1H-pyrazol-4-yl}-*N*-(1-cyanocyclopropyl)benzamide (162 mg, 250 µmol) was dissolved in dry THF (4.0 mL). This solution was cooled to -45°C on an acetone - dry ice bath. Potassium bis(trimethylsilyl)amide (800 µl 0.50 M solution in toluene, 400 µmol) was added dropwise and the mixture was stirred 30 min. at -45 °C. Then, sodium iodide (19 mg, 125 µmol) and a solution of 1-*tert*-butyl 4-(chloromethyl) 2,2-dimethylbutanedioate (intermediate 3A, 94.0 mg, 375 µmol) in THF (4.0 mL) was added, the dry ice bath was removed and the mixture was stirred at room temperature for 40 min. The solvent was distilled and the residue was purified by preparative HPLC (RP C-18 10 µm water-acetonitrile gradient with 0.01 % TFA in both eluents, 90:10->5:95) to give 156 mg (100 % purity, 72 % yield) of the title compound.

LC-MS Method 4): Rₜ = 5.42 min; MS (ESIpos): m/z = 863 [M+H]⁺

¹H-NMR (400 MHz, DMSO-d⁶) δ [ppm]: 0.996 (0.44), 1.058 (5.32), 1.186 (0.43), 1.235 (0.77), 1.280 (16.00), 1.364 (0.71), 1.737 (1.21), 2.523 (1.78), 7.582 (0.80), 7.603 (1.00), 7.776 (1.40), 7.796 (1.07), 7.817 (0.90), 7.937 (2.08), 8.213 (2.35), 8.217 (2.24), 8.448 (1.91), 8.763 (1.75).

### Intermediate 26A

### tert-butyl [(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methyl carbonate

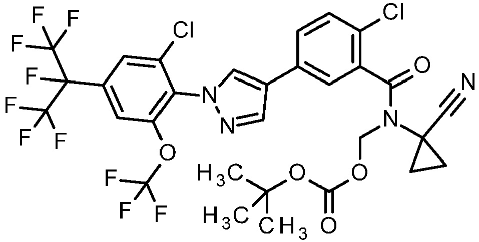

2-Chloro-5-{1-[2-chloro-4-(1,1,1,2,3,3,3-heptafluoropropan-2-yl)-6-(trifluoromethoxy)phenyl]-1*H-*pyrazol-4-yl}-*N*-(1-cyanocyclopropyl)benzamide (325 mg, 500 µmol) was dissolved in THF (16 mL) and the mixture was cooled to -45 °C on an acetone - dry ice bath. Potassium bis(trimethylsilyl)amide (1.4 mL, 0.50 M, 700 µmol) was added dropwise and the mixture was stirred 30 min. at -45 °C. Then, sodium iodide (38 mg, 250 µmol) and a solution of *tert*-butyl chloromethyl carbonate (CAS RN 35180-02-0, 125 mg, 750 µmol) in THF 8.0 mL) was added, the dry ice bath was removed and the mixture was stirred at room temperature over night. The mixture was poured into conc. aqueous ammonium chloride and extracted with ethyl acetate. The organic extract was dried over anhydrous sodium sulfate, filtered and evaporated. The residue was purified by preparative HPLC (RP C-18 10 µm water-acetonitrile gradient with 0.01 % TFA in both eluents, 90:10->5:95) to give 220 mg (100 % purity, 56 % yield) of the title compound.

LC-MS Method 4): Rₜ = 5.06 min; MS (ESIpos): m/z = 779 [M+H]⁺

¹H-NMR (400 MHz, DMSO-d⁶) δ [ppm]: -0.008 (1.99), 0.008 (2.71), 1.157 (0.81), 1.175 (1.56), 1.193 (0.77), 1.235 (0.98), 1.320 (16.00), 1.356 (3.15), 1.476 (1.03), 1.750 (1.42), 1.988 (2.62), 3.477 (3.98), 4.021 (0.62), 4.039 (0.61), 7.588 (0.94), 7.609 (1.14), 7.773 (1.54), 7.798 (1.17), 7.819 (0.99), 7.939 (2.24), 8.212 (2.57), 8.447 (1.99), 8.776 (1.94).

### Intermediate 27A

### 2-Chloro-5-{1-[2-chloro-4-(1,1,1,2,3,3,3-heptafluoropropan-2-yl)-6-(trifluoromethoxy)phenyl]-1H-pyrazol-4-yl}-N-(1-cyanocyclopropyl)-N-(hydroxymethyl)benzamide

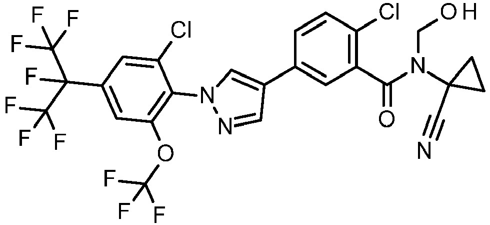

*tert*-Butyl [(2-chloro-5-{1-[2-chloro-4-(1,1,1,2,3,3,3-heptafluoropropan-2-yl)-6-(trifluoromethoxy) phenyl]-1*H*-pyrazol-4-yl}benzoyl)(1-cyanocyclopropyl)amino]methyl carbonate (intermediate 26A, 220 mg, 282 µmol) was dissolved in 30 % TFA in DCM (200 µL) and the mixture was stirred at room temperature for 90 seconds..The solvents were then distilled under vacuum and the residue was purified by preparative (RP C-18 10 µm acetonitrile-water gradient with 0.01 % TFA in both eluents, 10:90->95:5). 53.0 mg (92 % purity, 25 % yield) of the title compound were obtained.

LC-MS Method 4): Rₜ = 4.20 min; MS (ESIpos): m/z = 679 [M+H]⁺

¹H-NMR (600 MHz, DMSO-d⁶) δ [ppm]: -0.021 (0.45), 0.005 (0.82), 0.999 (1.09), 1.005 (1.03), 1.012 (0.49), 1.235 (1.03), 1.242 (2.99), 1.246 (3.56), 1.259 (0.79), 1.287 (5.91), 1.296 (16.00), 1.307 (0.84), 1.314 (1.50), 1.320 (1.40), 1.326 (0.79), 1.502 (2.04), 1.686 (5.48), 2.075 (2.75), 2.387 (0.47), 2.426 (0.82), 2.655 (0.69), 3.385 (8.66), 4.597 (2.36), 6.336 (1.07), 6.699 (1.03), 7.563 (6.91), 7.577 (7.78), 7.663 (0.54), 7.696 (0.47), 7.710 (0.51), 7.742 (1.07), 7.745 (1.46), 7.761 (10.40), 7.774 (6.34), 7.777 (5.13), 7.788 (5.09), 7.791 (4.49), 7.883 (0.75), 7.939 (9.99), 8.086 (0.43), 8.213 (11.43), 8.390 (0.75), 8.455 (14.52), 8.481 (0.41), 8.703 (0.77), 8.759 (1.98), 8.777 (13.74), 8.808 (0.64), 9.049 (1.18).

### Intermediate 28A

### tert-Butyl 4-(2-{[(2-chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxyl-2-oxoethyl)benzoate

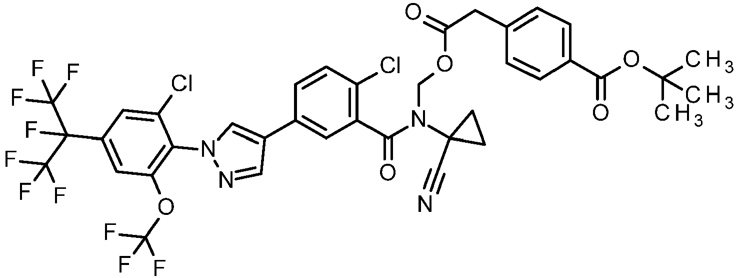

*tert*-Butyl 4-(2-chloro-2-oxoethyl)benzoic acid (CAS RN 732308-82-6, 118 mg, 0.5 mmol) was dissolved in DCM (2.0 mL), the mixture was cooled to 0 °C. Then, oxalic chloride (87 µL, 1.0 mmol) and DMF (2.5 µL) were added. The mixture was stirred for 30 min, then the solvent was evaporated at room temperature under vacuum. The resulting *tert*-butyl 4-(2-chloro-2-oxoethyl)benzoate (127 mg, 500 µmol) was taken up into THF (1.0 mL). and this mixture was added to a solution of 2-chloro-5-{1-[2-chloro-4-(1,1,1,2,3,3,3-heptafluoropropan-2-yl)-6-(trifluoromethoxy)phenyl]-1H-pyrazol-4-yl}-*N*-(1-cyanocyclopropyl)-*N*-(hydroxymethyl)benzamide (intermediate 27A, 170 mg, 250 µmol) and DIPEA (87 µl, 500 µmol) in THF (3.0 mL) at room temperature. Stirring was maintained over night. The solvents were distilled, the residue was purified by preparative HPLC (RP C-18 10 µm acetonitrile-water gradient with 0.01 % TFA in both eluents, 10:90->95:5) and eventually, 115 mg (94 % purity, 48 % yield) of the title compound were obtained.

LC-MS Method 4): Rₜ = 5.45 min; MS (ESIpos): m/z = 897 [M+H]⁺

¹H-NMR (600 MHz, DMSO-d⁶) δ [ppm]: 1.236 (0.43), 1.358 (0.82), 1.522 (16.00), 1.534 (1.43), 1.539 (1.43), 1.542 (0.62), 1.544 (0.49), 1.551 (1.16), 1.562 (1.09), 7.778 (0.42), 7.797 (0.56), 7.919 (0.60), 8.191 (0.67), 8.194 (0.66).

### Synthesis of Example Compounds according to the Present Invention

### Example Compound 1

### 2-({[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}carbonyl)cyclopropane-1-carboxylic acid (mixture of isomers)

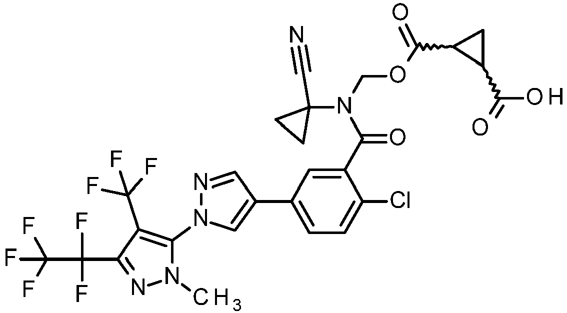

*tert*-Butyl [{2-chloro-5-[2'-methyl-5'-(pentafluoroethyl)-4'-(trifluoromethyl)-2'*H*-[1,3'-bipyrazol]-4-yl] benzoyl}(1-cyanocyclopropyl)amino]methylcyclopropane-1,2-dicarboxylate (intermediate 2A, 125 mg, 166 µmol) was dissolved in 30 % TFA in DCM (13.0 mL) and stirred at ambient temperature for 20 min. The volatiles were evaporated and the residue was purified by preparative HPLC (RP C-18 10 µm acetonitrile-water gradient with 0.01 % TFA in both eluents, 10:90->95:5). 88.0 mg (100 % purity, 76 % yield) of the title compound were obtained.

LC-MS Method 4): Rₜ = 3.93 min; MS (ESIpos): m/z = 695 [M+H]⁺

¹H-NMR (600 MHz, DMSO-d⁶) δ [ppm]: 1.275 (0.45), 1.757 (1.79), 1.977 (0.87), 3.820 (16.00), 7.611 (0.72), 7.626 (0.84), 7.843 (2.66), 7.854 (1.28), 8.563 (1.63), 8.832 (1.88).

### Example Compound 2

### 4-{[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}-2,2-dimethyl-4-oxobutanoic acid

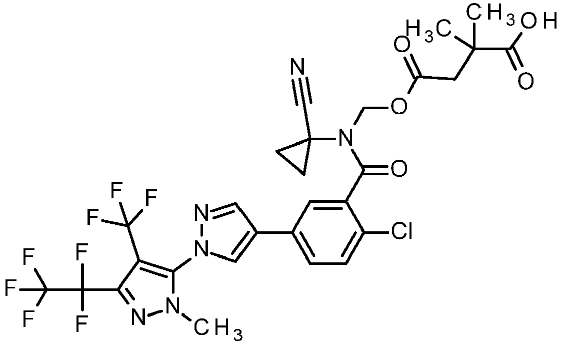

1-*tert*-Butyl 4-{[{2-chloro-5-[2'-methyl-5'-(pentafluoroethyl)-4'-(trifluoromethyl)-2'*H*-[1,3'-bipyrazol]-4-yl]benzoyl}(1-cyanocyclopropyl)amino]methyl} 2,2-dimethylbutanedioate (intermediate 4A, 108 mg, 141 µmol) was dissolved in 30 % TFA in DCM (11.0 mL) and stirred at ambient temperature for 20 min. The volatiles were evaporated and the residue was purified by preparative HPLC (RP C-18 10 µm acetonitrile-water gradient with 0.01 % TFA in both eluents, 10:90->95:5). 92.0 mg (100 % purity, 92 % yield) of the title compound were obtained.

LC-MS Method 4): Rₜ = 4.12 min; MS (ESIneg): m/z = 711 [M+H]⁻

¹H-NMR (600 MHz, DMSO-d⁶) δ [ppm]: 1.029 (0.88), 1.084 (5.58), 1.220 (0.52), 1.740 (2.04), 3.817 (16.00), 7.608 (1.01), 7.622 (1.21), 7.794 (1.65), 7.836 (1.09), 7.850 (1.11), 8.560 (2.17), 8.824 (2.13).

### Example 3

### 4-({ [(2-Chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methoxy}carbonyl)benzoic acid

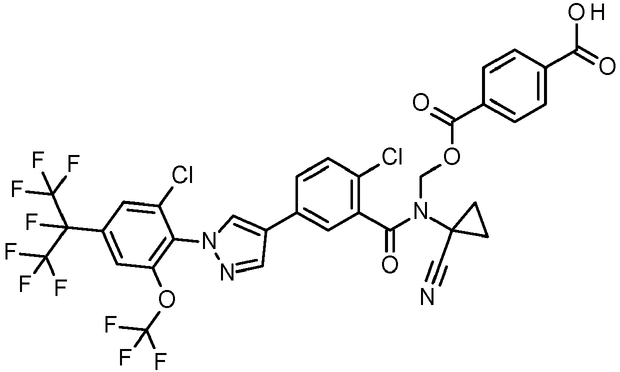

*tert*-Butyl [(2-chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methyl benzene-1,4-dicarboxylate (intermediate 6A, 160 mg, 181 µmol) was dissolved in 30 % TFA in DCM (8.0 mL) and stirred at ambient temperature for 20 min. The volatiles were evaporated and the residue was purified by preparative HPLC (RP C-18 10 µm acetonitrile-water gradient with 0.01 % TFA in both eluents, 10:90->95:5). 126 mg (100 % purity, 84 % yield) of the title compound were obtained.

LC-MS Method 4): Rₜ = 4.51 min; MS (ESIpos): m/z = 827 [M+H]⁺

¹H-NMR (600 MHz, DMSO-d⁶) δ [ppm]: -0.020 (0.42), 1.037 (0.70), 1.235 (1.05), 1.279 (1.52), 1.299 (1.43), 1.550 (0.86), 1.598 (1.32), 1.727 (1.27), 1.806 (6.13), 2.086 (3.27), 2.387 (0.73), 2.426 (0.92), 2.521 (1.49), 2.524 (1.27), 2.578 (0.44), 2.615 (0.70), 2.655 (0.84), 3.820 (1.49), 5.461 (1.01), 5.655 (1.03), 6.994 (0.92), 7.079 (0.97), 7.164 (0.86), 7.598 (2.90), 7.611 (3.38), 7.807 (3.69), 7.820 (3.38), 7.877 (5.30), 7.910 (10.37), 8.016 (4.26), 8.029 (8.62), 8.047 (8.33), 8.182 (11.52), 8.185 (11.52), 8.387 (15.08), 8.725 (16.00), 8.768 (0.42).

### Example 4

### 5-{[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}-5-oxopentanoic acid

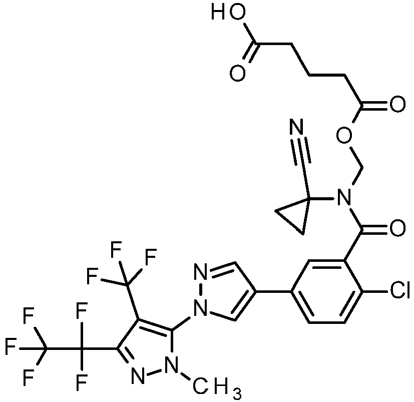

*tert*-Butyl [{2-chloro-5-[2'-methyl-5'-(pentafluoroethyl)-4'-(trifluoromethyl)-2'*H*-[1,3'-bipyrazol]-4-yl] benzoyl}(1-cyanocyclopropyl)amino]methyl pentanedioate (intermediate 8A, 100 mg, 133 µmol) was dissolved in 30 % TFA in DCM (8.0 mL) and stirred at ambient temperature for 5 min. The volatiles were evaporated and the residue was purified by preparative HPLC (RP C-18 10 µm acetonitrile-water gradient with 0.01 % TFA in both eluents, 10:90->95:5). 68.2 mg (100 % purity, 74 % yield) of the title compound were obtained.

LC-MS Method 4): Rₜ = 3.85 min; MS (ESIpos): m/z = 697 [M+H]⁺

¹H-NMR (600 MHz, DMSO-d⁶) δ [ppm]: 1.237 (0.59), 1.399 (2.97), 1.504 (0.42), 1.671 (1.32), 1.683 (1.75), 1.695 (1.34), 1.748 (2.12), 2.085 (0.73), 2.182 (1.29), 2.194 (2.22), 2.206 (1.22), 2.343 (1.88), 2.384 (0.47), 2.423 (0.62), 2.652 (0.47), 3.334 (1.31), 3.818 (16.00), 7.610 (1.11), 7.624 (1.27), 7.821 (1.88), 7.841 (1.36), 7.854 (1.22), 8.564 (2.37), 8.831 (2.29).

### Example 5

### 1-({[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}carbonyl)cyclopropane-1-carboxylic acid

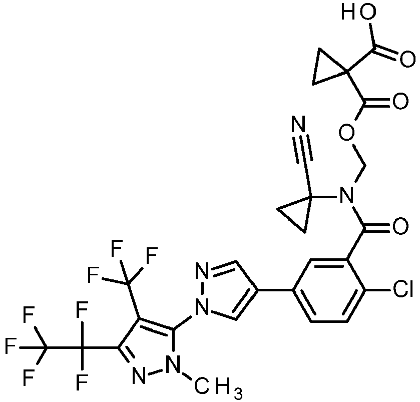

1-*tert*-Butyl 1-{[{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl} cyclopropane-1,1-dicarboxylate (intermediate 10A, 130 mg, 173 µmol) was dissolved in 30 % TFA in DCM (10.0 mL) and stirred at ambient temperature for 5 min. The volatiles were evaporated and the residue was purified by preparative HPLC (RP C-18 10 µm acetonitrile-water gradient with 0.01 % TFA in both eluents, 10:90->95:5). 20.0 mg (100 % purity, 17 % yield) of the title compound were obtained.

LC-MS Method 4): Rₜ = 3.95 min; MS (ESIneg): m/z = 693 [M-H]⁻

¹H-NMR (600 MHz, DMSO-d⁶) δ [ppm]: 1.314 (3.72), 1.749 (1.69), 3.818 (16.00), 7.620 (0.85), 7.634 (0.99), 7.799 (1.37), 7.841 (1.02), 7.854 (0.96), 8.534 (2.70), 8.798 (3.09).

### Example 6

### 4-{[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}-4-oxobutanoic acid

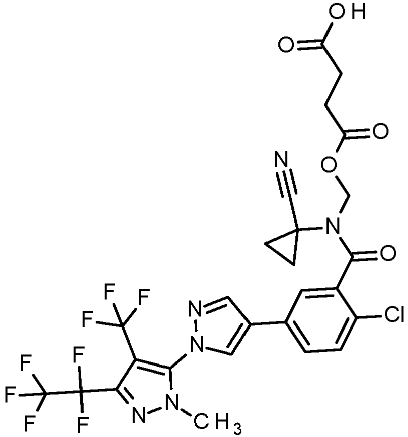

*tert*-Butyl [{2-chloro-5-[2'-methyl-5'-(pentafluoroethyl)-4'-(trifluoromethyl)-2'*H*-[1,3'-bipyrazol]-4-yl] benzoyl}(1-cyanocyclopropyl)amino]methyl butanedioate (intermediate 12A, 180 mg, 244 µmol) was dissolved in 30 % TFA in DCM (15.0 mL) and stirred at ambient temperature for 5 min. The volatiles were evaporated and the residue was purified by preparative HPLC (RP C-18 10 µm acetonitrile-water gradient with 0.01 % TFA in both eluents, 10:90->95:5). 125 mg (100 % purity, 75 % yield) of the title compound were obtained.

LC-MS Method 4): Rₜ = 3.80 min; MS (ESIneg): m/z = 681 [M-H]⁻

¹H-NMR (600 MHz, DMSO-d⁶) δ [ppm]: 1.747 (1.80), 2.432 (1.91), 2.442 (1.45), 3.819 (16.00), 7.611 (0.98), 7.625 (1.12), 7.823 (1.70), 7.840 (1.16), 7.854 (1.06), 8.563 (2.19), 8.828 (2.20).

### Example 7

### (2E)-4-{[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}-4-oxobut-2-enoic acid

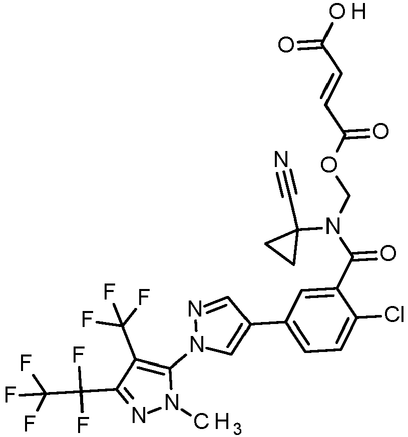

*tert*-Butyl [{2-chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methyl (2E)-but-2-enedioate (intermediate 14A, 175 mg, 237 µmol) was dissolved in 30 % TFA in DCM (15.0 mL) and stirred at ambient temperature for 5 min. The volatiles were evaporated and the residue was purified by preparative HPLC (RP C-18 10 µm acetonitrile-water gradient with 0.01 % TFA in both eluents, 10:90->95:5). 101 mg (97 % purity, 61 % yield) of the title compound were obtained.

LC-MS Method 4): Rₜ = 3.87 min; MS (ESIpos): m/z = 681 [M+H]⁺

¹H-NMR (400 MHz, DMSO-d⁶) δ [ppm]: -0.008 (0.59), 0.008 (0.54), 1.533 (0.46), 1.672 (0.45), 1.776 (1.79), 2.367 (0.43), 2.670 (0.40), 3.815 (16.00), 5.472 (0.41), 6.605 (0.58), 6.644 (1.35), 6.706 (1.40), 6.745 (0.67), 7.619 (1.01), 7.639 (1.23), 7.844 (1.22), 7.872 (2.13), 8.551 (3.72), 8.830 (2.87).

### Example 8

### 4-{[(2-Chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-4-oxobutanoic acid

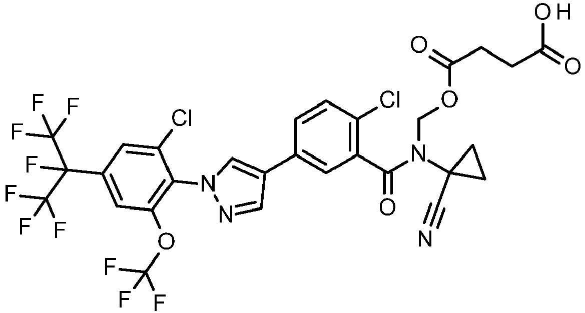

*tert*-Butyl [(2-chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methyl butanedioate (intermediate 16A, 525 mg, 628 µmol) was dissolved in 30 % TFA in DCM (25.0 mL) and stirred at ambient temperature for 15 min. The volatiles were evaporated and the residue was purified by preparative HPLC (RP C-18 10 µm acetonitrile-water gradient with 0.01 % TFA in both eluents, 10:90->95:5). 320 mg (100 % purity, 65 % yield) of the title compound were obtained.

LC-MS (Method 4): Rₜ = 4.12 min; MS (ESIpos): m/z = 779 [M+H]⁺

¹H-NMR (400 MHz, DMSO-d⁶) δ [ppm]: -0.008 (2.35), 0.008 (2.63), 1.138 (1.03), 1.185 (0.66), 1.235 (0.47), 1.510 (2.35), 1.614 (2.21), 1.742 (10.18), 2.073 (2.49), 2.328 (1.08), 2.366 (1.17), 2.430 (9.99), 2.446 (8.68), 2.670 (1.45), 2.710 (1.17), 5.234 (2.16), 5.319 (2.25), 7.587 (5.21), 7.607 (6.48), 7.790 (11.45), 7.817 (6.10), 7.938 (14.22), 8.209 (16.00), 8.213 (15.72), 8.454 (13.09), 8.774 (12.06), 12.240 (11.92).

### Example 9

### 5-{[(2-Chloro-5-{1-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-5-oxopentanoic acid

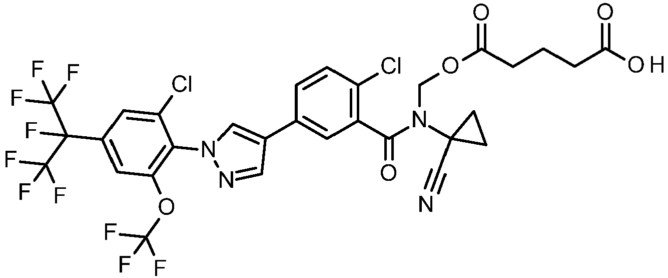

*tert*-Butyl [(2-chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methyl pentanedioate (intermediate 17A, 180 mg, 212 µmol) was dissolved in 30 % TFA in DCM (18 mL) and stirred at ambient temperature for 15 min. The volatiles were evaporated and the residue was purified by preparative HPLC (RP C-18 10 µm acetonitrile-water gradient with 0.01 % TFA in both eluents, 10:90->95:5). 140 mg (100 % purity, 83 % yield) of the title compound were obtained.

LC-MS Method 4): Rₜ = 4.32 min; MS (ESIpos): m/z = 793 [M+H]⁺

¹H-NMR (400 MHz, DMSO-d⁶) δ [ppm]: -0.149 (0.89), -0.008 (6.59), 0.008 (8.73), 0.146 (0.81), 1.045 (0.44), 1.185 (0.52), 1.235 (0.52), 1.508 (2.41), 1.668 (6.72), 1.686 (9.54), 1.704 (7.92), 1.746 (10.88), 2.132 (0.78), 2.180 (6.64), 2.198 (11.82), 2.216 (6.35), 2.328 (6.85), 2.343 (9.83), 2.360 (5.62), 2.671 (1.23), 2.711 (0.97), 3.503 (4.13), 5.200 (1.91), 5.338 (1.93), 5.642 (0.47), 7.587 (6.07), 7.607 (7.32), 7.787 (10.30), 7.799 (8.78), 7.821 (6.35), 7.937 (14.12), 8.209 (16.00), 8.454 (13.39), 8.777 (12.58), 12.068 (0.73).

### Example 10

### 4-({[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}carbonyl)benzoic acid

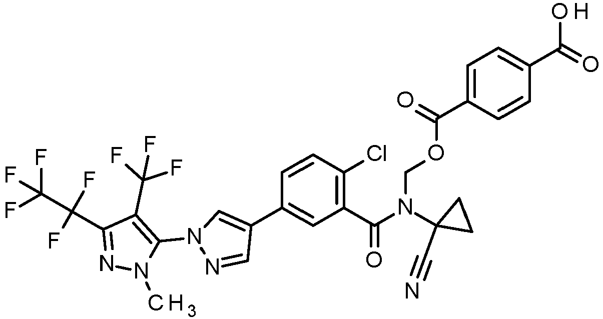

*tert*-Butyl [{2-chloro-5-[2'-methyl-5'-(pentafluoroethyl)-4'-(trifluoromethyl)-2'*H*-[1,3'-bipyrazol]-4-yl] benzoyl}(1-cyanocyclopropyl)amino]methyl benzene-1,4-dicarboxylate (intermediate 18A, 150 mg, 191 µmol) was dissolved in 30 % TFA in DCM (15 mL) and stirred at ambient temperature for 30 min. The volatiles were evaporated and the residue was purified by preparative HPLC (RP C-18 10 µm acetonitrile-water gradient with 0.01 % TFA in both eluents, 10:90->95:5). 101 mg (100 % purity, 73 % yield) of the title compound were obtained.

LC-MS Method 4): Rₜ = 3.98 min; MS (ESIpos): m/z = 731 [M+H]⁺

¹H-NMR (600 MHz, DMSO-d⁶) δ [ppm]: 0.005 (1.03), 1.594 (0.68), 1.739 (0.71), 1.811 (3.35), 2.385 (0.64), 2.423 (0.71), 2.464 (0.97), 2.515 (1.43), 2.518 (1.49), 2.522 (1.28), 2.571 (0.67), 2.613 (0.59), 2.652 (0.58), 3.787 (16.00), 5.463 (0.59), 5.636 (0.61), 7.618 (1.64), 7.632 (1.84), 7.838 (1.81), 7.850 (1.67), 7.899 (2.84), 8.032 (6.15), 8.039 (6.28), 8.493 (6.62), 8.781 (12.17).

### Example 11

### (trans)-4-({[(2-Chloro-5-{1-[2-chloro-4-(1,1,1,2,3,3,3-heptafluoropropan-2-yl)-6-(trifluoromethoxy) phenyl]-1H pyrazol-4-yl}benzoyl)(1-cyanocyclopropyl)amino]methoxy}carbonyl)cyclohexane-1-carboxylic acid

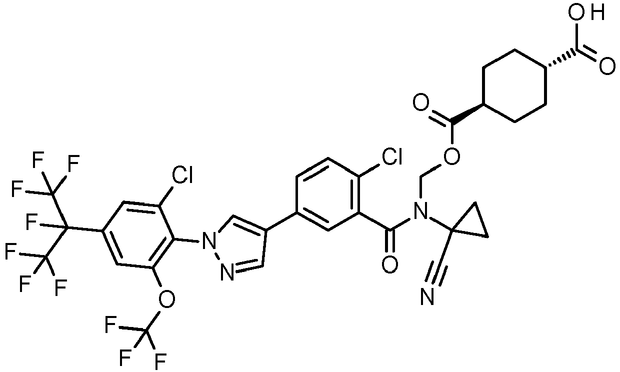

*tert*-Butyl [(2-chloro-5-{1-[2-chloro-4-(1,1,1,2,3,3,3-heptafluoropropan-2-yl)-6-(trifluoromethoxy) phenyl]-1*H*-pyrazol-4-yl}benzoyl)(1-cyanocyclopropyl)amino]methyl (*trans*)-cyclohexane-1,4-dicarboxylate (intermediate 22A, 200 mg, 225 µmol) was dissolved in 30 % TFA in DCM (20 mL) and stirred at ambient temperature for 20 min. The volatiles were evaporated and the residue was purified by preparative HPLC (RP C-18 10 µm acetonitrile-water gradient with 0.01 % TFA in both eluents, 10:90->95:5). 137 mg (100 % purity, 73 % yield) of the title compound were obtained.

LC-MS (Method 4): Rₜ = 4.53 min; MS (ESIpos): m/z = 833 [M+H]⁺

¹H-NMR (600 MHz, DMSO-d⁶) δ [ppm]: -0.022 (0.46), 1.186 (0.66), 1.235 (2.53), 1.275 (11.76), 1.490 (2.27), 1.646 (1.98), 1.755 (7.94), 1.855 (7.10), 1.878 (8.46), 2.083 (3.29), 2.257 (3.19), 2.388 (0.58), 2.427 (0.69), 2.656 (0.50), 3.516 (3.90), 5.167 (1.92), 5.347 (1.90), 7.598 (6.53), 7.612 (7.29), 7.796 (12.66), 7.815 (5.93), 7.934 (14.16), 8.207 (16.00), 8.480 (13.20), 8.791 (13.11).

### Example 12

### (trans)-4-({[{2-Chloro-5-[2'-methyl-5'-(pentafluoroethyl)-4'-(trifluoromethyl)-2'H-[1,3'-bipyrazol]-4-yl]benzoyl}(1-cyanocyclopropyl)amino]methoxy}carbonyl)cyclohexane-1-carboxylic acid

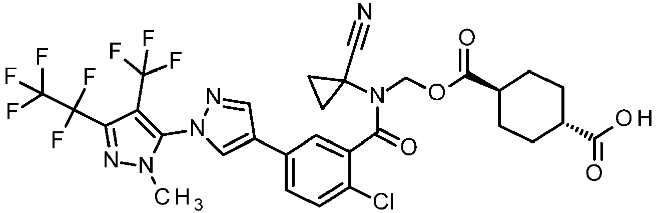

*tert*-Butyl [{2-chloro-5-[2'-methyl-5'-(pentafluoroethyl)-4'-(trifluoromethyl)-2'*H*-[1,3'-bipyrazol]-4-yl] benzoyl}(1-cyanocyclopropyl)amino]methyl (*trans*)-cyclohexane-1,4-dicarboxylate (intermediate 23A, 152 mg, 192 µmol) was dissolved in 30 % TFA in DCM (15 mL) and stirred at ambient temperature for 15 min. The volatiles were evaporated and the residue was purified by preparative HPLC (RP C-18 10 µm acetonitrile-water gradient with 0.01 % TFA in both eluents, 10:90->95:5). 131 mg (98 % purity, 91 % yield) of the title compound were obtained.

LC-MS Method 4): Rₜ = 4.05 min; MS (ESIpos): m/z = 737 [M+H]⁺

¹H-NMR (600 MHz, DMSO-d⁶) δ [ppm]: 1.237 (0.83), 1.275 (2.13), 1.322 (0.41), 1.754 (1.62), 1.871 (1.47), 2.086 (0.62), 2.255 (0.56), 3.817 (16.00), 7.619 (1.06), 7.632 (1.23), 7.822 (1.75), 7.837 (1.20), 7.852 (1.09), 8.585 (2.12), 8.840 (2.12).

### Example 13

### (2E)-4-{[(2-Chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methoxy}-4-oxobut-2-enoic acid

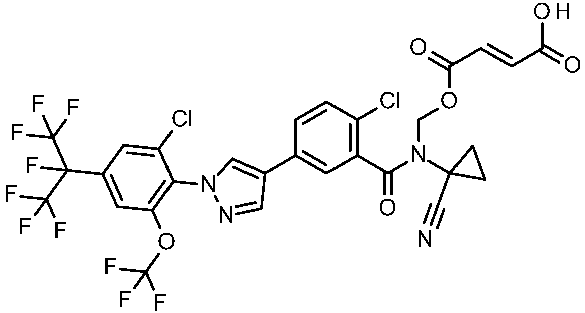

tert-Butyl [(2-chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methyl (2E)-but-2-enedioate (intermediate 24A, 141 mg, 169 µmol) was dissolved in 30 % TFA in DCM (15 mL) and stirred at ambient temperature for 15 min. The volatiles were evaporated and the residue was purified by preparative HPLC (RP C-18 10 µm acetonitrile-water gradient with 0.01 % TFA in both eluents, 10:90->95:5). 118 mg (100 % purity, 90 % yield) of the title compound were obtained.

LC-MS (Method 4): Rₜ = 4.20 min; MS (ESIpos): m/z = 777 [M+H]⁺

¹H-NMR (500 MHz, DMSO-d⁶) δ [ppm]: -0.022 (0.47), 0.007 (0.67), 1.236 (1.09), 1.446 (1.07), 1.547 (1.73), 1.661 (1.69), 1.770 (7.51), 2.363 (0.75), 2.637 (0.56), 4.316 (0.51), 5.354 (1.42), 5.469 (1.47), 6.620 (2.51), 6.652 (4.53), 6.679 (0.98), 6.725 (4.75), 6.757 (2.85), 7.596 (3.84), 7.613 (4.42), 7.806 (5.07), 7.823 (5.15), 7.837 (6.95), 7.926 (12.85), 8.197 (14.82), 8.201 (14.26), 8.433 (16.00), 8.716 (0.64), 8.761 (9.91), 13.231 (0.44).

### Example 14

### 4-1[(2-Chloro-5-11-[2-chloro-4-(1,1,1,2,3,3,3-heptafluoropropan-2-yl)-6-(trifluoromethoxy)phenyl]-1H-pyrazol-4-yl}benzoyl)(1-cyanocyclopropyl)amino]methoxy}-2,2-dimethyl-4-oxobutanoic acid

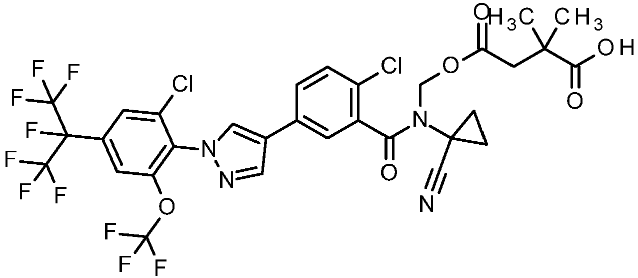

1-tert-butyl 4-{[(2-chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methyl} 2,2-dimethylbutanedioate (152 mg, 176 µmol) was dissolved in 30 % TFA in DCM (15 mL) and stirred at ambient temperature for 20 min. The volatiles were evaporated and the residue was purified by preparative HPLC (RP C-18 10 µm acetonitrile-water gradient with 0.01 % TFA in both eluents, 10:90->95:5). 128 mg (100 % purity, 90 % yield) of the title compound were obtained.

LC-MS Method 4): Rₜ = 4.53 min; MS (ESIpos): m/z = 807 [M+H]⁺

¹H-NMR (400 MHz, DMSO-d⁶) δ [ppm]: -0.008 (2.23), 0.008 (2.59), 1.026 (1.30), 1.082 (16.00), 1.214 (1.38), 1.234 (1.16), 1.515 (1.07), 1.613 (1.03), 1.736 (4.66), 2.073 (1.52), 2.328 (0.74), 2.367 (0.52), 2.670 (1.19), 2.710 (0.61), 5.183 (0.78), 5.342 (0.76), 7.583 (2.35), 7.604 (2.91), 7.765 (3.95), 7.793 (2.94), 7.813 (2.55), 7.937 (6.21), 8.212 (7.04), 8.215 (6.76), 8.451 (5.46), 8.770 (5.16), 12.186 (1.60

### Example 15

### 4-(2-{[(2-Chloro-5-{1-[2-chloro-4-(1,1,1,2,3,3,3-heptafluoropropan-2-yl)-6-(trifluoromethoxy) phenyl]-1H-pyrazol-4-yl}benzoyl)(1-cyanocyclopropyl)amino]methoxy}-2-oxoethyl)benzoic acid

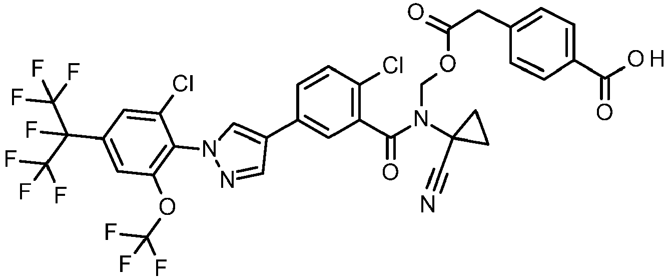

*tert*-Butyl 4-(2-{[(2-chloro-5-{1-[2-chloro-4-(1,1,1,2,3,3,3-heptafluoropropan-2-yl)-6-(trifluoro-methoxy)phenyl]-1*H*-pyrazol-4-yl}benzoyl)(1-cyanocyclopropyl)amino]methoxy}-2-oxoethyl) benzoate (intermediate 28A, 114 mg, 127 µmol) was dissolved in 30 % TFA in DCM (15 mL) and stirred at ambient temperature for 20 min. The volatiles were evaporated and the residue was purified by preparative HPLC (RP C-18 10 µm acetonitrile-water gradient with 0.01 % TFA in both eluents, 10:90->95:5). 85.0 mg (98 % purity, 78 % yield) of the title compound were obtained.

LC-MS Method 4): Rₜ = 4.49 min; MS (ESIpos): m/z = 841 [M+H]⁺

¹H-NMR (600 MHz, DMSO-d⁶) δ [ppm]: 1.236 (0.41), 1.489 (1.51), 1.624 (1.47), 1.746 (7.08), 2.086 (0.45), 2.386 (0.73), 2.424 (1.55), 2.520 (1.63), 2.523 (1.83), 2.573 (0.49), 2.577 (0.61), 2.613 (0.69), 2.653 (1.47), 3.561 (0.73), 3.774 (6.31), 3.907 (0.69), 5.241 (1.22), 5.368 (1.22), 7.306 (7.86), 7.319 (8.39), 7.452 (0.61), 7.580 (3.91), 7.594 (4.44), 7.805 (13.96), 7.826 (11.11), 7.839 (9.61), 7.922 (13.84), 8.194 (16.00), 8.197 (15.35), 8.462 (8.71), 8.771 (8.59), 12.860 (0.61).

### Physicochemical and Biological Examples

### Assessment of Prodrug Stability and Release of Parent Drug

Preparation of the PBS buffer solution pH 7.4: 90g sodium chloride, 13.61 g potassium dihydrogen phosphate, 83.35 g 1M aqueous sodium hydroxide were dissolved in water. More water was added up to a total volume of 1L. This solution was diluted 1: 10 with water. Eventually, pH was adjusted to pH 7.4 by the addition of phosphoric acid.

Citric acid buffer solution pH 3.0: commercially available citric acid buffer (pH 3) from Fluka (art. No 31046) was used containing 8.47 g of citric acid, 3.49 g of sodium chloride and 0.82 g of sodium hydroxide.

### Biological Example B1

### Measurement of Solubility in Buffer at pH 6.5

2 - 4 mg of the test compound are dissolved in DMSO to reach a concentration of 50 g/L (solution A). To 10 µL of this solution 960 µL PBS buffer pH 6.5 are added (final concentration: 515 µg/l); the mixture is shaken for 24h at rt in a 96 well plate. An aliquot is centrifuged at 42000 rpm für 30 min. The supernatant is diluted with acetonitrile/water (8:2) 1:10 and 1:1000 resp. This diluted samples are analyzed by LC-MSMS.

Calibration: 10 µL of solution A are diluted with 823 µL DMSO (final concentration: 600 µg/mL), which is further diluted with acetonitrile/water 8:2 by a factor of 100 (provides solution B).

The calibration curve is obtained from solution B by further diluting with acetonitrile/water 8:2 with target concentrations of 1.2 -12 - 60 - 600 ng/mL and injecting these four solutions for MS measurement.

MS method optimization: Solution B is utilized for MS method optimization.

PBS-Puffer: 6.18g sodium chloride and 3.96 g sodium dihydrogen phosphate are dissolved in 1L aqua dist., the pH is adjusted to 6.5 with 1N sodium hydroxide.

### LC and MS conditions

LC-MSMS optimization: The following configurations were used for optimization
AB Sciex TRIPLE QUAD 4500, Agilent 1260 Infinity (G1312B), degasser (G4225A), column oven (G1316C and G1316A), CTC Analytics PAL injection system HTS-xt
Eluent A: 0.5 mL formic acid (50 % strength)/ L water, Eluent B: 0.5 mL formic acid (50 % strength) / L acetonitrile

| **time [min]** | **flow [µL/min]** | **%B** |
|---|---|---|
| 0.00 | 200 | 70 |
| 0.08 | 200 | 70 |
| 0.09 | 25 | 70 |
| 0.60 | 25 | 70 |
| 0.65 | 200 | 70 |
| 1.10 | 200 | 70 |

Autosampler: without auto inject ahead setting; column: stainless steel capillary, oven temperature: 22°C; flow rate: flow gradient, injected volume: 2 µL.
Waters Quattro Micro MS, Agilent 1100 (G1312A), degasser (G1322A), column oven (G1316A), CTC Analytics PAL injection system HTS, eluents as above

| **time [min]** | **flow [µL/min]** | **%B** |
|---|---|---|
| 0.00 | 250 | 70 |
| 1.50 | 250 | 70 |
| 0.09 | 25 | 70 |

Autosampler with auto inject ahead setting; column: stainless steel capillary, oven temperature: 22°C, flow rate: flow gradient, injected volume: 5 µL.
MS method: Flow Injection Analysis (FIA) for optimization ("MS-OPTI"); ionization mode ABSciex-MS: ESI-pos/neg, Waters-MS: ESI-pos

### HPLC method for MSMS quantification

The following conditions were used for quantification:
Eluent A, B as above
ABSciex-MS

| **time [min]** | **%A** | **%B** |
|---|---|---|
| 0 | 90 | 10 |
| 0.5 | 5 | 95 |
| 0.84 | 5 | 95 |
| 0.85 | 90 | 10 |
| 1.22 | 90 | 10 |

Autosampler without auto inject ahead setting, column: Waters OASIS HLB, 2.1 x 20 mm, 25 µ, column temperature: 30°C, flow rate: 2.5 mL/min, injected volume: 2 µL, Splitter (before MS) 1:20.

### Waters-MS

### Gradient as above

Autosampler: with auto inject ahead setting, column: stainless steel capillary, column: Waters OASIS HLB, 2,1 x 20 mm, 25 µ, column temperature: 30°C, flow rate: 2,5 mL/min, injected volume: 5 µL, Splitter (before MS) 1:20, MS method: Multiple Reaction Monitoring (MRM).

**Table 0: Solubility of example compounds at pH 6.5**

| **Example No** | **mg/mL** |
|---|---|
| 1 | 331 |
| 2 | 71 |
| 3 | 3 |
| 4 | 179 |
| 5 | 171 |
| 6 | 200 |
| 8 | 12 |
| 9 | 10 |
| 10 | 31 |
| 11 | 5 |
| 12 | 46 |
| 13 | 18 |
| 14 | 5 |
| 15 | 5 |

### Biological Example B2

### Measurement of Stability of Prodrugs in Buffer at pH 7.4

0.15 mg of the test compound were dissolved in 0.1 mL dimethylsulfoxide and 0.4 mL acetonitrile. For complete dissolution, the HPLC vial with the sample solution was shaken and treated with ultrasound. Then, 1.0 mL of PBS buffer solution pH 7.4 was added and the sample was vortexed.The sample solution was analysed by HPLC (method 8) to determine the amount of the test compound at a particular time over a period of 24 h at 37 °C. The peak areas, given as percentage of total area, are used for quantification. In addition, the reaction mixture was analysed by method 9 (HPLC-MS) at the final timepoint.

**Table 1: Stability of example compounds at pH 7.4**

| **Example No** | **% Recovery after 24 h** |
|---|---|
| 1 | 100 |
| 2 | 55 |
| 3 | 99 |
| 4 | 100 |
| 5 | 71 |
| 6 | 90 |
| 7 | 96 |
| 8 | 95 |
| 9 | 100 |
| 10 | 100 |
| 11 | 100 |
| 12 | 100 |
| 13 | 97 |
| 14 | 63 |
| 15 | 100 |

### Biological Example B3

### Measurement of Release of Parent Drug in Rat Plasma

1 mg of the test compound was dissolved in 0.5 mL acetonitrile/dimethylsulfoxide 9:1. For complete dissolution the HPLC vial was shaken and treated with ultrasound. 20 µL of this solution were added to 1 mL of rat plasma (Li-heparin plasma, Hannover-Janvier rat, RjHan male) with vortexing at a temperature of 37 °C. Aliquots (100 µL each) were taken at 0.17, 0.5, 1, 1.5, 2 and 4 hours. Each aliquot was transferred to a vial containing 300 µL of acetonitrile/ citric acid buffer pH 3 8:2. These solutions were centrifuged at 5000 rpm for 10 minutes. The supernatant was analysed by HPLC (Method 8) to determine the amount of the test compound. In addition, the reaction mixture was analysed by method 9 (HPLC-MS) at the final timepoint. Both decrease of the prodrug concentration and increase of parent drug concentration were monitored. All data is given as percent area of the prodrug at t₀.

Parent Drug A means 2-Chlor-5-{1-[2-chlor-4-(1,1,1,2,3,3,3-heptafluorpropan-2-yl)-6-(trifluormethoxy)phenyl]-1*H*-pyrazol-4-yl}-*N*-(1-cyanocyclopropyl)benzamide (CAS-RN 1771742-44-9).

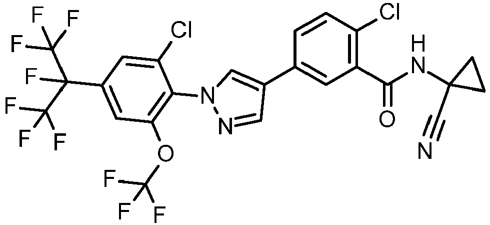

### Parent Drug B means 2-chloro-N-(1-cyanocyclopropyl)-5-[2'-methyl-5'-(pentafluoroethyl)-4'-(trifluoromethyl)-2'H-[1,3'-bipyrazol]-4-yl]benzamide (CAS-RN 1621436-41-6)

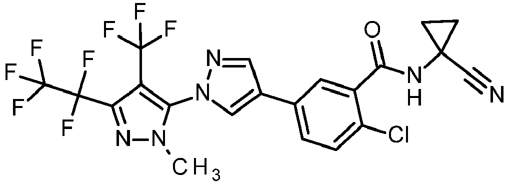

**Table 2: Degradation of prodrugs in rat plasma: and release of parent drug**

| | **% Recovery of prodrug at different time points** | | | | | | **Parent Drug** | **% Parent Drug** |
|---|---|---|---|---|---|---|---|---|
| **Example No** | **0.17 h** | **0.5 h** | **1h** | **1.5 h** | **2h** | **4h** | | **4h** |
| **1** | 93 | 88 | 80 | 74 | 66 | 49 | B | 53 |
| 3 | 100 | 100 | 100 | 100 | 100 | 100 | A | 0 |
| 4 | 73 | 42 | 18 | 9 | 0 | 0 | B | 109 |
| 7 | 84 | 58 | 26 | 10 | 4 | 0 | B | 78 |
| 8 | 96 | 95 | 93 | 92 | 90 | 86 | A | 14 |
| 9 | 89 | 77 | 66 | 56 | 48 | 29 | A | 71 |
| 10 | 99 | 98 | 96 | 94 | 92 | 85 | B | 16 |
| 11 | 98 | 96 | 92 | 89 | 86 | 74 | A | 26 |
| 12 (^{∗}) | 91 | 70 | 49 | 33 | 22 | 0 | B | 104 |
| 13 | 96 | 85 | 63 | 49 | 35 | 14 | A | 65 |
| 15 | 94 | 81 | 62 | 47 | 36 | 10 | A | 91 |

| | | | | | | | | |
|---|---|---|---|---|---|---|---|---|
| (*): HPLC conditions: Method 10 | | | | | | | | |

### Biological Example B4

### Measurement of Release of Parent Drug in Dog Plasma

1 mg of the test compound was dissolved in 0.5 mL acetonitrile/dimethylsulfoxide 9:1. For complete dissolution the HPLC vial was shaken and treated with ultrasound. 20 µL of this solution were added to 1 mL of dog plasma (Beagle, male; EDTA stabilized) under vortexing at a temperature of 37 °C. Aliquots (100 µL each) were taken at 0.17, 0.5, 1, 1.5, 2 and 4 hours. Each aliquot was transferred to a vial containing 300 µL of acetonitrile/ citric acid buffer pH 3 80:20. These solutions were centrifuged at 5000 rpm for 10 minutes. The supernatant was analysed by HPLC (Method 8) to determine the amount of the test compound. In addition, the reaction mixture was analysed by method 9 (HPLC-MS) at the final timepoint.

Both decrease of the prodrug concentration and increase of parent drug concentration were monitored. All data is given as percent area of the prodrug at t₀.

**Table 3: Degradation of prodrugs in dog plasma**

| | **% Recovery at indicated time points** | | | | | | **Parent Drug** | **% Parent Drug** |
|---|---|---|---|---|---|---|---|---|
| **Example No** | **0.17 h** | **0.5 h** | **1h** | **1.5 h** | **2h** | **4h** | | **4h** |
| **13** | 95 | 79 | 51 | 33 | 18 | 0 | A | 79 |

### Biological Example B5

### Evaluation of Pharmacokinetics (in vivo)

To evaluate the pharmacokinetics of test substances in vivo, these test substances are dissolved in appropriate formulation vehicles (ethanol, dimethyl sulfoxide, PEG400, glycerol formal etc.), or mixtures thereof. The test substances are then administered to rats or dogs intravenously, orally or subcutaneously. The intravenous application is performed as bolus. Administered doses range usually between 0.1 to 5 mg/kg, however, doses for subcutaneous and oral administration can exceed this range up to doses of 30 mg/kg. Blood samples are retrieved via a catheter, exsanguination or venous puncture in vials containing appropriate anticoagulants, such as lithium heparinate or potassium EDTA. Plasma is generated from the blood via centrifugation. Blood samples are taken over an appropriate time interval, usually lasting up to 144h after administration. If necessary, samples from later time points can also be taken. If possible, time points are chosen so that the initial absorption phase, the maximum plasma concentration (Cmax), and exposure within a certain time interval (AUC(0-t))aer described. Furthermore, it is possible to also retrieve organ-, tissue- and urine samples . The quantitative measurement of the test substances in the samples is performed using calibration curves in the respective matrices. The protein content of the samples is precipitated using acetonitrile or methanol. Thereafter, the samples are separated using HPLC in combination with reversed phase chromatography columns. The HPLC system is coupled to a triple quadrupole mass spectrometer via an electrospray interface. The evaluation of the plasma concentration / time profiles are afterwards evaluated using a validated pharmacokinetics evaluation program.

Exposure of parent drug A within a certain time interval displayed as AUC(0-t) in tables 4-6 was used to assess conversion of prodrug to drug and absorption of prodrug compared to parent drug *in vivo,* respectively. Comparison of exposures after intravenous administration of parent drug A and prodrugs showed the amount of prodrug which was converted into parent. Comparison of exposures after subcutaneous or oral administration of parent drug and prodrugs additionally showed altered absorption characteristics of prodrugs compared to parent. Relative bioavailability (Fᵣₑₗ) was used to describe the exposure of parent drug after prodrug administration compared to the exposure of parent drug after direct administration of compound A which was normalised to 100 %.

**Table 4: AUC of parent drug A after intravenous administration of example prodrugs and after intravenous administration of parent drug A**

| | **Units** | **Concentration of Parent Drug A** | | | |
|---|---|---|---|---|---|
| **after admin. of** | | **Parent Drug A** | **Example 13** | **Example 8** | **Parent Drug A** |
| **Admin Route** | | iv bolus | | | |
| **Species** | | Rat | | | Dog |
| **AUC(0-144 h)ₙₒᵣₘ** | kg·h/L | 11 | 7.3 | 12 | |
| **AUC(0-72 h)ₙₒᵣₘ** | kg·h/L | | | | 6.0 |

**Table 5: AUC of parent drug A after subcutaneous administration of example prodrugs and after subcutaneous administration of parent drug A**

| | **Units** | **Concentration of Parent Drug A** | | | |
|---|---|---|---|---|---|
| **after admin. of** | | **Parent Drug A** | **Example 13** | **Example 8** | **Parent Drug A** |
| **Admin Route** | | sc | | | |
| **Species** | | Rat | | | Dog |
| **AUC(0-144 h)ₙₒᵣₘ** | kg h/L | 1.7 | 5.9 | 6.7 | |
| **AUC(0-384 h)ₙₒᵣₘ** | kg h/L | | | | 1.0 |
| **Fᵣₑₗ*** | % | 100 | 347 | 394 | 100 |

| | | | | | |
|---|---|---|---|---|---|
| ^{∗}Fᵣₑₗ calculated based on AUC(0-t)norm | | | | | |

**Table 6: AUC of parent drug A after oral administration of example prodrugs and after oral administration of parent drug A**

| | **Units** | **Concentration of Parent Drug A** | |
|---|---|---|---|
| **after admin. of** | | **Example 1** | **Parent Drug A** |
| **Admin Route** | | po | |
| **Species** | | Rat | |
| **Dose Admin** | | | |
| **Unit Dose Admin** | | mg/kg | |
| **AUC(0-24)norm** | kg·h/L | | |
| **F*** | % | | |

| | | | |
|---|---|---|---|
| *F calculated based on AUC(0-24)ₙₒᵣₘ | | | |

### Biological Example B6

### Measurement of the Antiparasitic Activity after Administration of a Prodrug to Rats

Compounds were dissolved in the appropriate volume of glycerol formal (p.a.) just before application to the animals (dose volume was adjusted to 0.03 mL/kg bodyweight). 5 rats per group were either injected intraperitoneally or subcutaneously.

Rats were infested with 30 Dermacentor variabilis nymphs on study days -2, 7, 14, 21, 28, 35 and 30 adult Ctenocephalides fleas on study days -1, 8, 15, 22, 29, 36.

Parasite counts were performed on study days 2, 9, 16, 23, 30, 37.

Percent efficacy was calculated as arithmetic mean parasite numbers as life attached tick numbers and live flea numbers of the respective study group in comparison to parasite counts on a placebo-treated control group. Infestation of groups with less than 75% efficacy on two consecutive counting occasions will terminated for the respective parasite.

Both SC subcutaneous (sc) prodrug treatments (example 1 and example 4) are superior in their flea and tick efficacy on rats over the parent drug also injected subcutaneously.

**Table 7: In-vivo Efficacy of parent drug A and prodrug examples formulations applied i.p. or sc in rats**

| **Example** | **Dose [mg/kg]** | **Applicat. type** | **Parasite** | **SD2** | **SD9** | **SD16** | **SD23** | **SD30** | **SD37** | **SD42** |
|---|---|---|---|---|---|---|---|---|---|---|
| **Parent Drug A** | 10 | ip | Ctenocephalides felis adult | 84 | 68 | 71 | na | na | na | na |
| | | | Dermacentor variabilis nymph | 96 | 87 | 83 | 93 | 53 | 32 | na |
| | | sc | Ctenocephalides felis adult | 53 | 44 | na | na | na | na | na |
| | | | Dermacentor variabilis nymph | 72 | 61 | na | na | na | na | na |

**Table 8: In-vivo Efficacy of novel prodrug examples formulations applied sc in rats**

| **Example** | **Dose [mg/kg]** | **Applicat. type** | **Parasite** | **SD2** | **SD9** | **SD16** | **SD23** | **SD30** | **SD37** | **SD42** |
|---|---|---|---|---|---|---|---|---|---|---|
| **Example Compound 13** | 11.97 | sc | Ctenocephalides felis adult | 100 | 97 | 96 | 79 | 57 | 51 | na |
| | | | Dermacentor variabilis nymph | 100 | 100 | 99 | 77 | 59 | 49 | na |
| **Example Compound 9** | 12.22 | sc | Ctenocephalides felis adult | 100 | 97 | 96 | 97 | 91 | 46 | na |
| | | | Dermacentor variabilis nymph | 99 | 100 | 96 | 98 | 85 | 49 | na |
| **Example Compound 11** | 12.84 | sc | Ctenocephalides felis adult | 100 | 100 | 100 | 100 | 97 | 76 | na |
| | | | Dermacentor variabilis nymph | 100 | 100 | 100 | 100 | 96 | 71 | na |
| **Example Compound 3** | 12.74 | sc | Ctenocephalides felis adult | 100 | 100 | 100 | 96 | 80 | 55 | na |
| | | | Dermacentor variabilis nymph | 99 | 98 | 93 | 96 | 85 | 48 | na |
| **Example Compound 8** | 12.00 | sc | Ctenocephalides felis adult | 100 | 99 | 100 | 100 | 97 | 88 | 78 |
| | | | Dermacentor variabilis nymph | 100 | 100 | 99 | 100 | 100 | 100 | 92 |
| **Example Compound 15** | 12.96 | sc | Ctenocephalides felis adult | 100 | 100 | 100 | 99 | 96 | 81 | 75 |
| | | | Dermacentor variabilis nymph | 100 | 100 | 100 | 100 | 100 | 100 | 96 |

### Biological Example B7

### Method for Measurement of the Antiparasitic Activity after Administration of a Prodrug to Dogs

Compounds are dissolved in the appropriate volume of glycerol formal (p.a.) just before application to the animals (dose volume is adjusted to 0.1 mL/kg bodyweight). Dogs are injected intravenously respective subcutaneously (see table 8 for details).

Dogs are infested with ectoparasites according to table on study 1 day prior to counting for fleas, 2 days prior to counting for RS, IR, DV ticks and 3 days prior to counting for AA ticks. For this purpose, dogs are placed in an individually labeled box with grid windows. The ticks are released onto the back of the dogs and are allowed to disperse and move into the hair without disturbance. The dog remains in the box for approximately 120 minutes with the lid closed and the light switched off in the room. For flea infestations, flea containers are opened in the animal's cage and fleas are applied on the dog's back between the shoulders.

Tick counts including removal of I. ricinus, R. sanguineus and D. variabilis are conducted 48(±4) hours after the infestation. Tick counts including removal of A. americanum are conducted 72(±4) hours after the infestation. Flea counts are conducted 24(±4) hours after the infestation.

On each parasite assessment day percent efficacy is calculated as arithmetic mean parasite numbers as life attached and live free tick numbers and live flea numbers of the respective study group in comparison to parasite counts on a placebo non-treated control group.

## Claims

1. Compounds of formula (I) 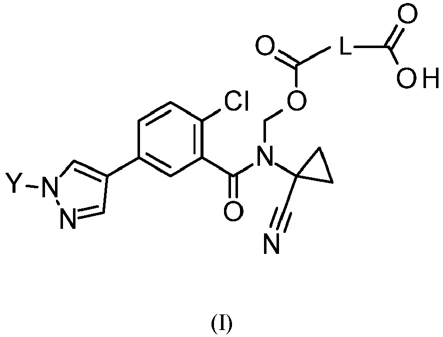 wherein
L is linear C₁-C₄ alkanediyl, C₂-C₄ alkenediyl or C₂-C₄ alkynediyl, each of which may be substituted with one or more groups independently selected from halogen, C₁-C₄ alkyl and C₃-C₆-cycloalkyl, wherein two C₁-C₄-alkyl substituents may form a ring together with the carbon atom to which they are bonded; or
a moiety (CH₂)ₙ-X-(CH₂)ₘ wherein
n and m are independently 0, 1 or 2 and
X is selected from a group X¹, X², X³ or X⁴, wherein
X¹ is C₃-C₇-cycloalkanediyl, which is optionally substituted with 1 to 3 substituents selected from halogen, cyano, and C₁-C₄ alkyl, wherein (CH₂)ₙ- and (CH₂)ₘ- may be attached either to the same or to different carbon atoms of X¹; or
X² is phenylene, which is optionally substituted with 1 to 4 substituents selected from halogen, cyano, and C₁-C₄ alkyl; or
X³ is C₅-C₆-heteroarenediyl, which is optionally substituted with 1, 2 or 3 substituents selected from halogen, cyano, and C₁-C₄ alkyl; or
X⁴ is C₅-C₈ bicycloalkanediyl, which is optionally substituted with 1 to 4 substituents selected from halogen, cyano, and C₁-C₄ alkyl wherein (CH₂)ₙ- and (CH₂)ₘ- may be attached either to the same or to different carbon atoms of X⁴; and
Y is selected from a group (T¹) 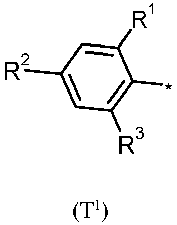
wherein
R¹, R² and R³ are each independently selected from hydrogen, halogen, cyano, nitro, linear or branched C₁-C₆-alkyl, C₃-C₆-cycloalkyl, C₁-C₆-alkoxy, halogen-substituted linear or branched C₁-C₆-alkyl, halogen-substituted C₁-C₆-alkoxy, halogen substituted C₃-C₆-cycloalkyl, C₁-C₆-alkylsulphanyl, Ci-Ce-alkylsulphinyl, C₁-C₆-alkylsulphonyl, *N-*C₁-C₆-alkylamino, *N,N*-di-C₁-C₆-alkylamino, *N*-C₁-C₃-alkoxy-C₁-C₄-alkylamino and 1-pyrrolidinyl
or from a group (T²) 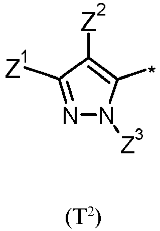
wherein
Z¹ and Z² are each independently selected from hydrogen, halogen, cyano, nitro, linear or branched Ci-Ce-alkyl, C₃-C₆-cycloalkyl, C₁-C₆-alkoxy, C₁-C₆-alkylcarbonyl, halogen-substituted linear or branched C₁-C₆-alkyl, halogen-substituted Ci-Ce-alkoxy, halogen substituted C₃-C₆-cycloalkyl, C₁-C₆-alkylsulphanyl, C₁-C₆-alkylsulphinyl, C₁-C₆-alkylsulphonyl, *N*-C₁-C₆-alkylamino, *N,N*-di-C₁-C₆-alkylamino, *N*-C₁-C₃-alkoxy-C₁-C₄-alkylamino and 1-pyrrolidinyl; and
Z³ represents hydrogen, linear or branched C₁-C₆-alkyl, C₃-C₆-cycloalkyl, C₂-C₆-alkenyl, C₂-C₆-alkynyl, aryl or hetaryl, each of which may be substituted with 1 to 5 substituents selected from hydroxy, halogen, cyano, nitro, amino, C₁-C₃-alkyl, C₁-C₃-alkoxy, hydroxycarbonyl, alkoxycarbonyl, alkylcarbamoyl, cycloalkylcarbamoyl and phenyl;
and the salts thereof.

2. Compounds according to claim 1, wherein
L is linear C₁-C₄ alkanediyl or C₂-C₄ alkenediyl, each of which may be substituted with one or more groups independently selected from halogen, C₁-C₄ alkyl and C₃-C₆ cycloalkyl, wherein two C₁-C₄-alkyl substituents may form a ring together with the carbon atom to which they are bonded; or
a moiety (CH₂)ₙ-X-(CH₂)ₘ wherein
n and m are independently 0, 1 or 2 and
X is selected from a group X¹, X², X³ or X⁴ as defined in claim 1; and
Y is selected from a group (T¹) or (T²) as defined in claim 1;
and the salts thereof.

3. Compounds according to claim 1 or 2, wherein
L is linear C₁-C₄ alkanediyl or C₂-C₄ alkenediyl, each of which may be substituted with one or more groups independently selected from halogen, C₁-C₄ alkyl and C₃-C₆ cycloalkyl, wherein two C₁-C₄-alkyl substituents may form a ring together with the carbon atom to which they are bonded; or
a moiety (CH₂)ₙ-X-(CH₂)ₘ wherein
n and m are independently 0, 1 or 2 and
X is selected from a group X¹ or X² as defined in claim 1; and
Y is selected from a group (T¹) or (T²) as defined in claim 1;
and the salts thereof.

4. Compounds according to claim 1, 2 or 3, wherein
Y is selected from a group (T¹) 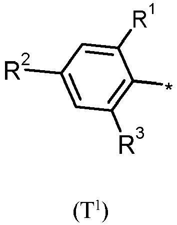 wherein
R¹, R² and R³ are each independently selected from hydrogen, halogen, linear or branched C₁-C₃-alkyl, C₃-C₆-cycloalkyl, C₁-C₃-alkoxy, linear or branched halogen-substituted C₁-C₃-alkyl, halogen-substituted C₁-C₃-alkoxy, halogen substituted C₃-C₆-cycloalkyl, and 1-pyrrolidinyl;
or from a group (T²) 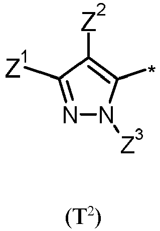
wherein
Z¹ represents linear or branched C₁-C₃-alkyl, C₃-C₆-cycloalkyl, C₁-C₃-alkoxy,
which may independently of one another be substituted with 1 to 5 substituents selected from hydroxy, halogen, cyano, nitro, C₁-C₃-alkyl, and C₁-C₃-alkoxy;
Z² represents halogen, cyano, nitro, amino, or linear or branched C₁-C₆-alkyl, C₁-C₆-alkylcarbonyl, C₁-C₆-alkylsulphanyl, C₁-C₆-alkylsulphinyl, C₁-C₆-alkylsulphonyl, which may independently of one another be substituted with 1 to 5 substituents selected from hydroxy, halogen, cyano, nitro, C₁-C₃-alkyl, and C₁-C₃-alkoxy;
and
Z³ represents hydrogen or linear or branched C₁-C₆-alkyl, C₃-C₆-cycloalkyl, C₂-C₆-alkenyl, C₂-C₆-alkynyl, aryl or hetaryl, which may independently of one another be substituted with 1 to 5 substituents selected from hydroxy, halogen, cyano, nitro, C₁-C₃-alkyl, and C₁-C₃-alkoxy;
and the salts thereof.

5. Compounds according to claim 1, 2, 3 or 4, wherein
Y is selected from a group (T¹) 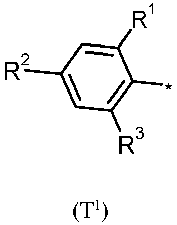
wherein
R¹ is halogen;
R² is linear or branched C₁-C₃-alkyl substituted with 1 to 7 halogen; and
R³ is C₁-C₃-alkoxy substituted with 1 to 3 halogen;
or from a group (T²) 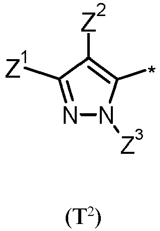
wherein
Z¹ represents linear or branched C₁-C₃-alkyl or C₃-C₆-cycloalkyl, substituted with 1 to 5 halogen substituents;
Z² represents linear or branched C₁-C₃-alkyl substituted with 1 to 3 halogen substituents, nitro, methylsulphanyl, methylsulphinyl, methylsulphonyl, fluorine, chlorine, bromine, or iodine;
and
Z³ represents hydrogen or linear or branched C₁-C₆-alkyl;
and the salts thereof.

6. Compounds according to any one of the preceding claims, wherein the group (T¹) is represented by one of the following groups (T1-1), (T1-2) or (T2-1): 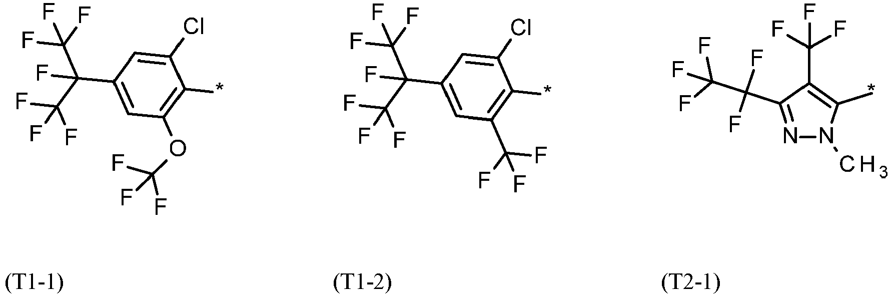 and the salts thereof.

7. Compounds according to any one of the preceding claims, wherein
L is linear C₂-C₄ alkanediyl or C₂-C₄ alkenediyl, each of which may be substituted with one or more groups independently selected from halogen and C₁-C₄ alkyl;
and the salts thereof.

8. Compounds according to any one of the preceding claims, wherein
L is a moiety (CH₂)ₙ-X¹-(CH₂)ₘ wherein
n and m are independently 0, 1 or 2 and
X¹ is C₃-C₆-cycloalkanediyl, which is optionally substituted with 1 to 3 substituents selected from halogen, cyano, and C₁-C₄ alkyl, wherein (CH₂)ₙ- and (CH₂)ₘ- may be attached either to the same or to different carbon atoms of X¹;
and the salts thereof.

9. Compounds according to any one of the preceding claims, wherein
L is a moiety (CH₂)ₙ-X²-(CH₂)ₘ wherein
n and m are independently 0, 1 or 2 and
X² is phenylene, which is optionally substituted with 1 to 4 substituents selected from halogen, cyano, and C₁-C₄ alkyl;
and the salts thereof.

10. Compounds according to any one of the preceding claims for use as medicaments.

11. Pharmaceutical composition comprising at least one compound according to any one of the preceding claims, which optionally comprises at least one further component selected from auxiliaries, excipients, solvents and/or at least one additional pharmaceutically active agent.

12. Pharmaceutical composition according to claim 11 for use as a medicament for subcutaneous or oral application.

13. The compounds according to any one of the claims 1 to 9 or the pharmaceutical composition according to claims 11 or 12 for use in controlling parasites on animals.

14. Process for preparing the compounds according to any of the claims 1 to 9, comprising the step of reacting a compound (A) 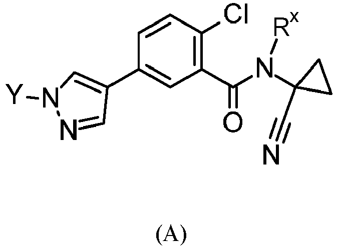
with a group (B) 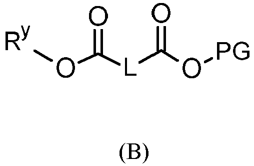
wherein
R^{x} represents hydrogen and R^{y} represents a -CH₂-C1 group or
R^{x} represents a -CH₂-OH group and R^{y} represents hydrogen;
wherein
Y and L have the meaning as defined in any one of the preceding claims and wherein PG represents a protecting group or hydrogen to form the compounds according to formula (I),
and wherein in cases wherein PG is not hydrogen, deprotection is carried out to form the compounds (I).

15. Process according to claim 14, further comprising
in the case that R^{x} represents hydrogen and R^{y} represents a -CH₂-C1 group the preliminary step of preparing the group (B) with R^{y} = -CH₂-C1 (compound (B-2)) from a compound (B-1) 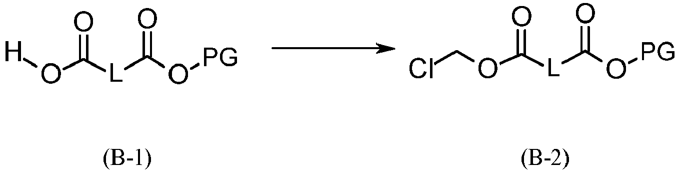 or
in the case that R^{x} represents a -CH₂-OH group and R^{y} represents hydrogen the preliminary step of
preparing the group (A) with R^{x} = -CH₂-OH (compound A-3)) from a compound (A-1) via an intermediate compound (A-2)

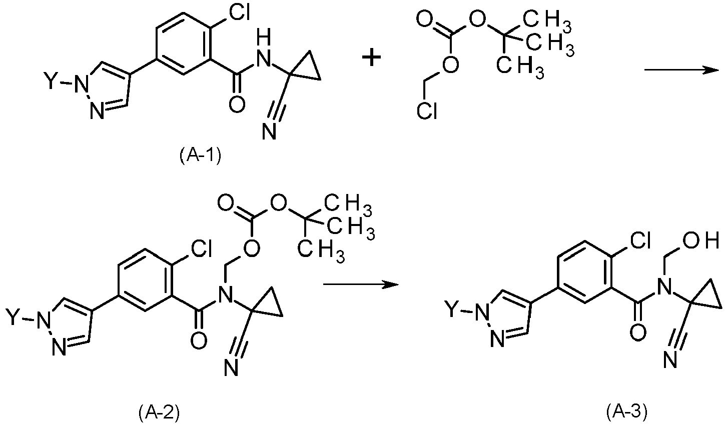

16. Intermediate compounds according to formula (C) 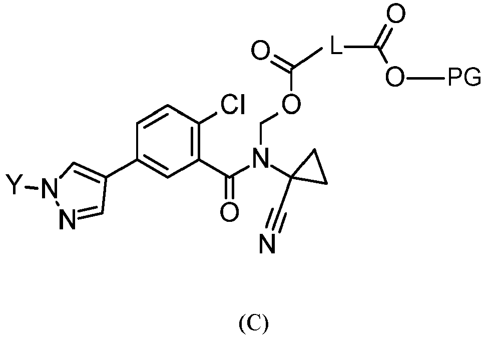
wherein Y and L have the meaning as defined in any one of the preceding claims and wherein PG represents a tert-butyl group;
or according to formula (A-3) 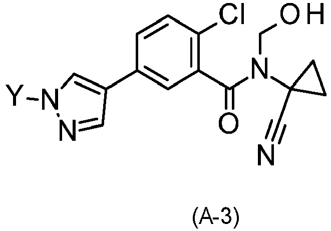
wherein Y has the meaning as defined in any one of the preceding claims.

## Patentansprüche

1. Verbindungen mit der Formel (I) 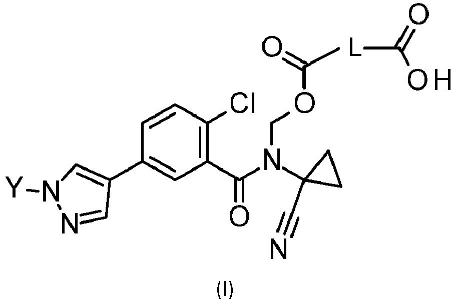 wobei
L lineares C₁-C₄-Alkandiyl, C₂-C₄-Alkendiyl oder C₂-C₄-Alkindiyl ist, wobei jedes davon mit einer oder mehreren Gruppen substituiert sein kann, die unabhängig voneinander aus Halogen, C₁-C₄-Alkyl und C₃-C₆-Cycloalkyl ausgewählt sind, wobei zwei
C₁-C₄-Alkylsubstituenten zusammen mit dem Kohlenstoffatom, an welches sie gebunden sind, einen Ring bilden können; oder
eine Grundeinheit (CH₂)ₙ-X-(CH₂)ₘ ist, worin
n und m unabhängig voneinander 0, 1 oder 2 sind und
X aus einer Gruppe X¹, X², X³ oder X⁴ ausgewählt ist; wobei
X¹ C₃-C₇-Cycloalkandiyl bedeutet, das wahlweise mit 1 bis 3 Substituenten substituiert ist, die aus Halogen, Cyano und C₁-C₄-Alkyl ausgewählt sind, wobei (CH₂)ₙ- und (CH₂)ₘ- an entweder dasselbe Kohlenstoffatom oder an verschiedene Kohlenstoffatome von X¹ gebunden sein können; oder
X² Phenylen bedeutet, das wahlweise mit 1 bis 4 Substituenten substituiert ist, die aus Halogen, Cyano und C₁-C₄-Alkyl ausgewählt sind, oder
X³ C₅-C₆-Heteroarendiyl bedeutet, das wahlweise mit 1, 2 oder 3 Substituenten substituiert ist, die aus Halogen, Cyano und C₁-C₄-Alkyl ausgewählt sind; oder
X⁴ C₅-C₈-Bicycloalkandiyl bedeutet, das wahlweise mit 1 bis 4 Substituenten substituiert ist, die aus Halogen, Cyano und C₁-C₄-Alkyl ausgewählt sind, wobei (CH₂)ₙ- und (CH₂)ₘ- an entweder dasselbe Kohlenstoffatom oder an verschiedene Kohlenstoffatome von X⁴ gebunden sein können; und
Y aus einer Gruppe (T¹) ausgewählt ist 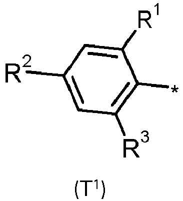 worin
R¹, R² und R³ jeweils unabhängig voneinander aus Wasserstoff, Halogen, Cyano, Nitro, linearem oder verzweigtem C₁-C₆-Alkyl, C₃-C₆-Cycloalkyl, C₁-C₆-Alkoxy, halogensubstituiertem linearem oder verzweigtem C₁-C₆-Alkyl, halogensubstituiertem C₁-C₆-Alkoxy, halogensubstituiertem C₃-C₆-Cycloalkyl, C₁-C₆-Alkylsulfanyl, C₁-C₆-Alkylsulfinyl, C₁-C₆-Alkylsulfonyl, *N*-C₁-C₆-Alkylamino, *N*,*N*-Di-C₁-C₆-Alkylamino, *N*-C₁-C₃-Alkoxy-C₁-C₄-alkylamino und 1-Pyrrolidinyl ausgewählt sind oder
aus einer Gruppe (T²)ausgewählt ist 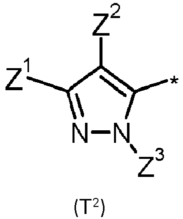
worin
Z¹ und Z² jeweils unabhängig voneinander aus Wasserstoff, Halogen, Cyano, Nitro, linearem oder verzweigtem C₁-C₆-Alkyl, C₃-C₆-Cycloalkyl, C₁-C₆-Alkoxy, C₁-C₆-Alkylcarbonyl, halogensubstituiertem linearem oder verzweigtem C₁-C₆-Alkyl, halogensubstituiertem C₁-C₆-Alkoxy, halogensubstituiertem C₃-C₆-Cycloalkyl, C,-Cs-Alkylsulfanyl, C₁-C₆-Alkylsulfinyl, C₁-C₆-Alkylsulfonyl, *N*-C₁-C₆-Alkylamino, *N,N*-Di-C₁-C₆-Alkylamino, *N*-C₁-C₃-Alkoxy-C₁-C₄-alkylamino und 1-Pyrrolidinyl ausgewählt sind; und
Z³ Wasserstoff, lineares oder verzweigtes
C₁-C₆-Alkyl, C₃-C₆-Cycloalkyl, C₂-C₆-Alkenyl, C₂-C₆-Alkinyl, Aryl oder Heteroaryl bedeutet, wobei jedes davon mit 1 bis 5 Substituenten substituiert sein kann, die aus Hydroxy, Halogen, Cyano, Nitro, Amino, C₁-C₃-Alkyl, C₁-C₃-Alkoxy, Hydroxycarbonyl, Alkoxycarbonyl, Alkylcarbamoyl, Cycloalkylcarbamoyl und Phenyl ausgewählt sind;
und die Salze davon.

2. Verbindungen nach Anspruch 1, wobei
L lineares C₁-C₄-Alkandiyl oder C₂-C₄-Alkendiyl ist, wobei jedes davon mit einer oder mehreren Gruppen substituiert sein kann, die unabhängig voneinander aus Halogen, C₁-C₄-Alkyl und C₃-C₆-Cycloalkyl ausgewählt sind, wobei zwei C₁-C₄-Alkylsubstituenten zusammen mit dem Kohlenstoffatom, an welches sie gebunden sind, einen Ring bilden können; oder
eine Grundeinheit (CH₂)ₙ-X-(CH₂)ₘ ist, worin
n und m unabhängig voneinander 0, 1 oder 2 sind und
X aus einer wie in Anspruch 1 definierten Gruppe X¹, X², X³ oder X⁴ ausgewählt ist; und
Y aus einer wie in Anspruch 1 definierten Gruppe (T¹) oder (T²) ausgewählt ist;
und die Salze davon.

3. Verbindungen nach Anspruch 1 oder 2, wobei
L lineares C₁-C₄-Alkandiyl oder C₂-C₄-Alkendiyl ist, wobei jedes davon mit einer oder mehreren Gruppen substituiert sein kann, die unabhängig voneinander aus Halogen, C₁-C₄-Alkyl und C₃-C₆-Cycloalkyl ausgewählt sind, wobei zwei C₁-C₄-Alkylsubstituenten zusammen mit dem Kohlenstoffatom, an welches sie gebunden sind, einen Ring bilden können; oder
eine Grundeinheit (CH₂)ₙ-X-(CH₂)ₘ ist, worin
n und m unabhängig voneinander 0, 1 oder 2 sind und
X aus einer wie in Anspruch 1 definierten Gruppe X¹ oder X² ausgewählt ist; und
Y aus einer wie in Anspruch 1 definierten Gruppe (T¹) oder (T²) ausgewählt ist;
und die Salze davon.

4. Verbindungen nach Anspruch 1, 2 oder 3, wobei
Y aus einer Gruppe (T¹) ausgewählt ist 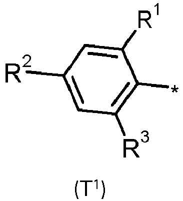
worin
R¹, R² und R³ jeweils unabhängig voneinander aus Wasserstoff, Halogen, linearem oder verzweigtem C₁-C₃-Alkyl, C₃-C₆-Cycloalkyl, C₁-C₃-Alkoxy, linearem oder verzweigtem halogensubstituiertem C₁-C₃-Alkyl, halogensubstituiertem C₁-C₃-Alkoxy, halogensubstituiertem C₃-C₆-Cycloalkyl und
1-Pyrrolidinyl ausgewählt sind; oder
aus einer Gruppe (T²) ausgewählt ist 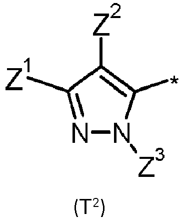
worin
Z¹ lineares oder verzweigtes C₁-C₃-Alkyl,
C₃-C₆-Cycloalkyl, C₁-C₃-Alkoxy bedeutet, das unabhängig voneinander mit 1 bis 5 Substituenten substituiert sein kann, die aus Hydroxy, Halogen, Cyano, Nitro, C₁-C₃-Alkyl und C₁-C₃-Alkoxy ausgewählt sind;
Z² Halogen, Cyano, Nitro, Amino oder lineares oder verzweigtes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl bedeutet, das unabhängig voneinander mit 1 bis 5 Substituenten substituiert sein kann, die aus Hydroxy, Halogen, Cyano, Nitro, C₁-C₃-Alkyl und C₁-C₃-Alkoxy ausgewählt sind; und
Z³ Wasserstoff oder lineares oder verzweigtes C₁-C₆-Alkyl, C₃-C₆-Cycloalkyl, C₂-C₆-Alkenyl, C₂-C₆-Alkinyl, Aryl oder Heteroaryl bedeutet, das unabhängig voneinander mit 1 bis 5 Substituenten substituiert sein kann, die aus Hydroxy, Halogen, Cyano, Nitro, C₁-C₃-Alkyl und C₁-C₃-Alkoxy ausgewählt sind;
und die Salze davon.

5. Verbindungen nach Anspruch 1, 2, 3 oder 4, wobei
Y aus einer Gruppe (T¹)ausgewählt ist 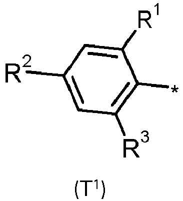
worin
R¹ Halogen bedeutet;
R² lineares oder verzweigtes C₁-C₃-Alkyl, das mit 1 bis 7 Halogenen substituiert ist, bedeutet; und
R³ C₁-C₃-Alkoxy, das mit 1 bis 3 Halogenen substituiert ist, bedeutet; oder
aus einer Gruppe (T²) ausgewählt ist 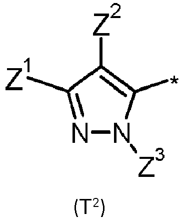
worin
Z¹ lineares oder verzweigtes C₁-C₃-Alkyl oder C₃-C₆-Cycloalkyl, das mit 1 bis 5 Halogensubstituenten substituiert ist;
Z² lineares oder verzweigtes C₁-C₃-Alkyl, das mit 1 bis 3 Halogensubstituenten, Nitro, Methylsulfanyl, Methylsulfinyl, Methylsulfonyl, Fluor, Chlor, Brom oder Iod substituiert ist; und
Z³ Wasserstoff oder lineares oder verzweigtes C₁-C₆-Alkyl ist;
und die Salze davon.

6. Verbindungen nach einem der vorhergehenden Ansprüche, wobei die Gruppe (T¹) von einer der folgenden Gruppen (T1-1), (T1-2) oder (T2-1) repräsentiert wird: 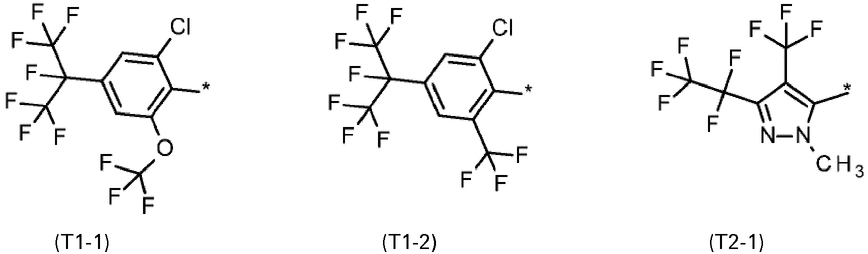 und die Salze davon.

7. Verbindungen nach einem der vorhergehenden Ansprüche, wobei
L lineares C₂-C₄-Alkandiyl oder C₂-C₄-Alkendiyl bedeutet, wobei jedes davon mit einer oder mehreren Gruppen substituiert sein kann, die unabhängig voneinander aus Halogen und C₁-C₄-Alkyl ausgewählt sind;
und die Salze davon.

8. Verbindungen nach einem der vorhergehenden Ansprüche, wobei
L eine Grundeinheit (CH₂)ₙ-X¹-(CH₂)ₘ bedeutet, worin
n und m unabhängig voneinander 0, 1 oder 2 sind und
X¹ C₃-C₆-Cycloalkandiyl bedeutet, das wahlweise mit 1 bis 3 Substituenten substituiert ist, die aus Halogen, Cyano und C₁-C₄-Alkyl ausgewählt sind, wobei (CH₂)ₙ- und (CH₂)ₘ- an entweder dasselbe Kohlenstoffatom oder an verschiedene Kohlenstoffatome von X¹ gebunden sein können;
und die Salze davon.

9. Verbindungen nach einem der vorhergehenden Ansprüche, wobei
L eine Grundeinheit (CH₂)ₙ-X²-(CH₂)ₘ bedeutet, worin
n und m unabhängig voneinander 0, 1 oder 2 sind und
X² Phenylen bedeutet, das wahlweise mit 1 bis 4 Substituenten substituiert ist, die aus Halogen, Cyano und C₁-C₄-Alkyl ausgewählt sind;
und die Salze davon.

10. Verbindungen nach einem der vorhergehenden Ansprüche für eine Verwendung als Arzneimittel.

11. Pharmazeutische Zubereitung, die mindestens eine Verbindung nach einem der vorhergehenden Ansprüche umfasst und welche wahlweise mindestens eine weitere Komponente umfasst, die aus Arzneimittelhilfsstoffen, Arzneimittelgrundlagen, Lösungsmitteln und/oder mindestens einem zusätzlichen pharmazeutischen Wirkstoff ausgewählt ist.

12. Pharmazeutische Zubereitung nach Anspruch 11 zur Verwendung als Arzneimittel für eine subkutane oder orale Verabreichung.

13. Die Verbindungen nach einem der Ansprüche 1 bis 9 oder die pharmazeutische Zubereitung nach Anspruch 11 oder 12 für eine Verwendung zur Bekämpfung von Parasiten bei Tieren.

14. Verfahren zur Herstellung der Verbindungen nach einem der Ansprüche 1 bis 9, welches den Schritt der Umsetzung einer Verbindung (A) 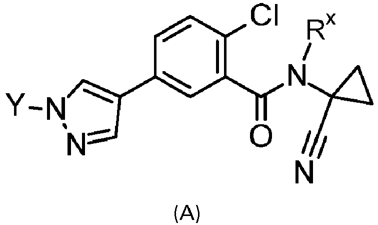 mit einer Gruppe (B) 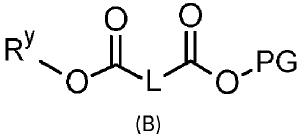
umfasst, worin
R^{x} Wasserstoff und R^{y} eine -CH₂-Cl-Gruppe oder
R^{x} eine -CH₂-OH-Gruppe und R^{y} Wasserstoff bedeutet;
wobei
Y und L die in einem der vorhergehenden Ansprüche definierte Bedeutung besitzen und wobei PG eine Schutzgruppe oder Wasserstoff bedeutet, um die Verbindungen mit der Formel (I) zu bilden, und wobei
in den Fällen, in welchen PG keinen Wasserstoff bedeutet, eine Entschützung durchgeführt wird, um die Verbindungen (I) zu bilden.

15. Verfahren nach Anspruch 14, das ferner in dem Fall, dass R^{x} Wasserstoff und R^{y} eine - CH₂-Cl-Gruppe bedeutet, den vorhergehenden Schritt der Herstellung der Gruppe (B) mit R^{y} = -CH₂-Cl (Verbindung (B-2)) aus einer Verbindung (B-1) 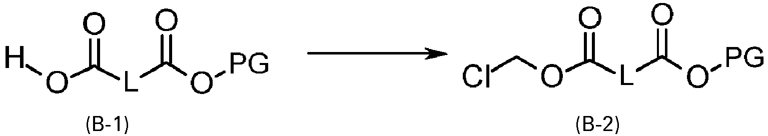 oder
in dem Fall, dass R^{x} eine -CH₂-OH-Gruppe und R^{y} Wasserstoff bedeutet, den vorhergehenden Schritt der Herstellung der Gruppe (A) mit R^{x} = -CH₂-OH (Verbindung (A-3)) aus einer Verbindung (A-1) über das Zwischenprodukt (A-2) 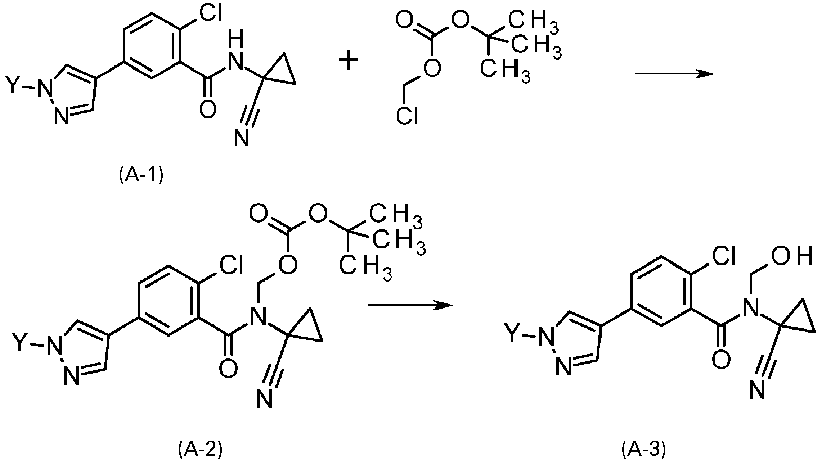 umfasst.

16. Zwischenprodukte mit der Formel (C) 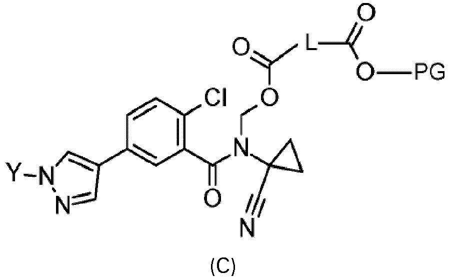
worin Y und L die in einem der vorhergehenden Ansprüche definierte Bedeutung besitzen und worin PG eine *tert*.-Butylgruppe bedeutet; oder
mit der Formel (A-3) 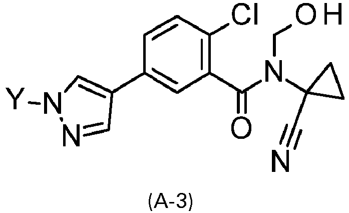
worin Y die in einem der vorhergehenden Ansprüche definierte Bedeutung besitzt.

## Revendications

1. Composés de formule (I) 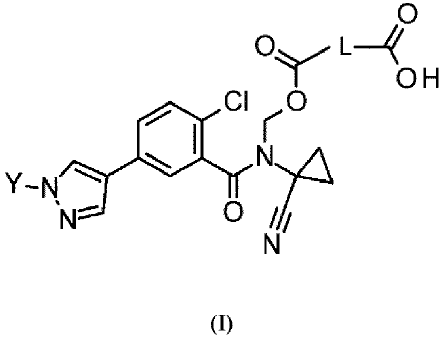 dans lesquels
L est alcanediyle en C₁ à C₄, alcanediyle en C₂ à C₄ ou alcynediyle en C₂ à C₄, inéaire, dont chacun peut être substitué par un ou plusieurs groupes indépendamment choisis parmi halogène, alkyle en C₁ à C₄ et cycloalkyle en C₃ à C₆, dans lequel deux substituants alkyle en C₁ à C₄ pouvant former un cycle conjointement avec l'atome de carbone auquel ils sont liés ; ou
un fragment (CH₂)ₙ-X-(CH₂)ₘ dans lequel
n et m valent indépendamment 0, 1 ou 2 et
X est choisi dans un groupe X¹, X², X³ ou X⁴, dans lequel
X¹ est cycloalcanediyle en C₃ à C₇, qui est facultativement substitué par 1 à 3 substituants choisis parmi halogène, cyano et alkyle en C₁ à C₄, dans lequel (CH₂)ₙ- et (CH₂)ₘ- pouvant être relié soit au même atome de carbone soit à des atomes de carbone différents de X¹ ; ou
X² est phénylène, qui est facultativement substitué par 1 à 4 substituants choisis parmi halogène, cyano et alkyle en C₁ à C₄ ; ou
X³ est hétéroarènediyle en C₅ à C₆, qui est facultativement substitué par 1, 2 ou 3 substituants choisis parmi halogène, cyano et alkyle en C₁ à C₄ ; ou
X⁴ est bicycloalcanediyle en C₅ à C₈, qui est facultativement substitué par 1 à 4 substituants choisis parmi halogène, cyano et alkyle en C₁ à C₄, dans lequel (CH₂)ₙ- et (CH₂)ₘ- pouvant être reliés soit au même atome de carbone soit à des atomes de carbone différents de X⁴ ; et
Y est choisi parmi un groupe (T¹) 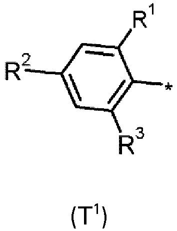
dans lequel
R¹, R² et R³ sont chacun indépendamment choisis parmi hydrogène, halogène, cyano, nitro, alkyle en C₁ à C₆ linéaire ou ramifié, cycloalkyle en C₃ à C₆, alcoxy en C₁ à C₆, alkyle en C₁ à C₆ linéaire ou ramifié à substitution halogène, alcoxy en C₁ à C₆ à substitution halogène, cycloalkyle en C₃ à C₆ à substitution halogène, alkylsulfanyle en C₁ à C₆, alkylsulfinyle en C₁ à C₆, alkylsulfonyle en C₁ à C₆, *N*-alkylamino en C₁ à C₆, *N,N*-di-alkylamino en C₁ à C₆, *N*-alcoxy en C₁ à C₃-alkylamino en C₁ à C₄ et 1-pyrrolidinyle
ou parmi un groupe (T²) 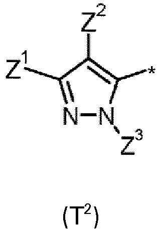
dans lequel
Z¹ et Z² sont chacun indépendamment choisis parmi hydrogène, halogène, cyano, nitro, alkyle en C₁ à C₆ linéaire ou ramifié, cycloalkyle en C₃ à C₆, alcoxy en C₁ à C₆, alkylcarbonyle en C₁ à C₆, alkyle en C₁ à C₆ linéaire ou ramifié à substitution halogène, alcoxy en C₁ à C₆ à substitution halogène, cycloalkyle en C₃ à C₆ à substitution halogène, alkylsulfanyle en C₁ à C₆, alkylsulfinyle en C₁ à C₆, alkylsulfonyle en C₁ à C₆, *N*-alkylamino en C₁ à C₆, *N,N*-di-alkylamino en C₁ à C₆, *N-*alcoxy en C₁ à C₃-alkylamino en C₁ à C₄ et 1-pyrrolidinyle ; et
Z³ représente hydrogène, alkyle en C₁ à C₆ linéaire ou ramifié, cycloalkyle en C₃ à C₆, alcényle en C₂ à C₆, alcynyle en C₂ à C₆, aryle ou hétaryle, dont chacun peut être substitué par 1 à 5 substituants choisis parmi hydroxy, halogène, cyano, nitro, amino, alkyle en C₁ à C₃, alcoxy en C₁ à C₃, hydroxycarbonyle, alcoxycarbonyle, alkylcarbamoyle, cycloalkylcarbamoyle et phényle ;
et les sels de ceux-ci.

2. Composés selon la revendication 1, dans lesquels
L est alcanediyle en C₁ à C₄ ou alcènediyle en C₂ à C₄, linéaire, dont chacun peut être substitué par un ou plusieurs groupes indépendamment choisis parmi halogène, alkyle en C₁ à C₄ et cycloalkyle en C₃ à C₆, dans lequel deux substituants alkyle en C₁ à C₄ pouvant former un cycle conjointement avec l'atome de carbone auquel ils sont liés ; ou
un fragment (CH₂)ₙ-X-(CH₂)ₘ dans lequel
n et m valent indépendamment 0, 1 ou 2 et
X est choisi parmi un groupe X¹, X², X³ ou X⁴ tel que défini dans la revendication 1 ; et
Y est choisi parmi un groupe (T¹) ou (T²) tel que défini dans la revendication 1 ;
et les sels de ceux-ci.

3. Composés selon la revendication 1 ou 2, dans lesquels
L est alcanediyle en C₁ à C₄ ou alcènediyle en C₂ à C₄, linéaire, dont chacun peut être substitué par un ou plusieurs groupes indépendamment choisis parmi halogène, alkyle en C₁ à C₄ et cycloalkyle en C₃ à C₆, dans lequel deux substituants alkyle en C₁ à C₄ pouvant former un cycle conjointement avec l'atome de carbone auquel ils sont liés ; ou
un fragment (CH₂)ₙ-X-(CH₂)ₘ dans lequel
n et m valent indépendamment 0, 1 ou 2 et
X est choisi parmi un groupe X¹ ou X² tel que défini dans la revendication 1 ; et
Y est choisi parmi un groupe (T¹) ou (T²) tel que défini dans la revendication 1 ;
et les sels de ceux-ci.

4. Composés selon la revendication 1, 2 ou 3, dans lesquels
Y est choisi parmi un groupe (T¹) 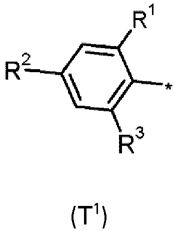
dans lequel
R¹, R² et R³ sont chacun indépendamment choisis parmi hydrogène, halogène, alkyle en C₁ à C₃ linéaire ou ramifié, cycloalkyle en C₃ à C₆, alcoxy en C₁ à C₃, alkyle en C₁ à C₃ à substitution halogène linéaire ou ramifié, alcoxy en C₁ à C₃ à substitution halogène, cycloalkyle en C₃ à C₆ à substitution halogène, et 1-pyrrolidinyle ;
ou parmi un groupe (T²) 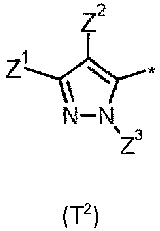
dans lequel
Z¹ représente alkyle en C₁ à C₃ linéaire ou ramifié, cycloalkyle en C₃ à C₆, alcoxy en C₁ à C₃, lesquels peuvent indépendamment l'un de l'autre être substitués par 1 à 5 substituants choisis parmi hydroxy, halogène, cyano, nitro, alkyle en C₁ à C₃ et alcoxy en C₁, à C₃ ;
Z² représente halogène, cyano, nitro, amino, ou alkyle en C₁ à C₆ linéaire ou ramifié, alkylcarbonyle en C₁ à C₆, alkylsulfanyle en C₁ à C₆, alkylsulfinyle en C₁ à C₆, alkylsulfonyle en C₁ à C₆, lesquels peuvent indépendamment l'un de l'autre être substitués par 1 à 5 substituants choisis parmi hydroxy, halogène, cyano, nitro, alkyle en C₁ à C₃, et alcoxy en C₁ à C₃ ;
et
Z³ représente hydrogène ou alkyle en C₁ à C₆ linéaire ou ramifié, cycloalkyle en C₃ à C₆, alcényle en C₂ à C₆, alcynyle en C₂ à C₆, aryle ou hétaryle, lesquels peuvent indépendamment l'un de l'autre être substitués par 1 à 5 substituants choisis parmi hydroxy, halogène, cyano, nitro, alkyle en C₁ à C₃ et alcoxy en C₁ à C₃ ;
et les sels de ceux-ci.

5. Composés selon la revendication 1, 2, 3 ou 4, dans lesquels
Y est choisi parmi un groupe (T¹) 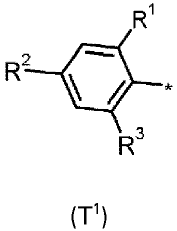
dans lequel
R¹ est halogène ;
R² est alkyle en C₁ à C₃ linéaire ou ramifié substitué par 1 à 7 halogènes ; et
R³ est alcoxy en C₁ à C₃ substitué par 1 à 3 halogènes ;
ou parmi un groupe (T²) 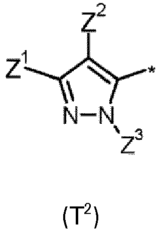
dans lequel
Z¹ représente alkyle en C₁ à C₃ linéaire ou ramifié ou cycloalkyle en C₃ à C₆, substitué par 1 à 5 substituants halogène ;
Z² représente alkyle en C₁ à C₃-linéaire ou ramifié substitué par 1 à 3 substituants halogène, nitro, méthylsulfanyle, méthylsulfinyle, méthylsulfonyle, fluor, chlore, brome ou iode ;
et
Z³ représente hydrogène ou alkyle en C₁ à C₆ linéaire ou ramifié ;
et les sels de ceux-ci.

6. Composés selon l'une quelconque des revendications précédentes, dans lesquels le groupe (T¹) est représenté par l'un des groupes (T1-1), (T1-2) ou (T2-1) suivants : 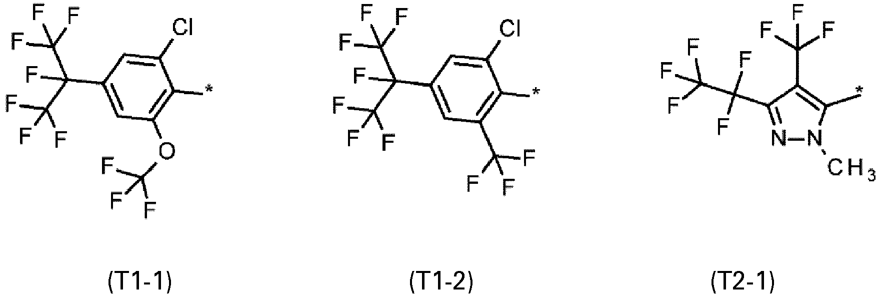 et les sels de ceux-ci.

7. Composés selon l'une quelconque des revendications précédentes, dans lesquels
L est alcanediyle en C₂ à C₄ ou alcènediyle en C₂ à C₄, linéaire, dont chacun peut être substitué par un ou plusieurs groupes indépendamment choisis parmi halogène et alkyle en C₁ à C₄ ;
et les sels de ceux-ci.

8. Composés selon l'une quelconque des revendications précédentes, dans lesquels
L est un fragment (CH₂)ₙ-X¹-(CH₂)ₘ dans lequel
n et m valent indépendamment 0, 1 ou 2 et
X¹ est cycloalcanediyle en C₃ à C₆, qui est facultativement substitué par 1 à 3 substituants choisis parmi halogène, cyano et alkyle en C₁ à C₄, (CH₂)ₙ- et (CH₂)ₘ- pouvant être relié soit au même atome de carbone soit à des atomes de carbone différents de X¹ ;
et les sels de ceux-ci.

9. Composés selon l'une quelconque des revendications précédentes, dans lesquels
L est un fragment (CH₂)ₙ-X²-(CH₂)ₘ dans lequel
n et m valent indépendamment 0, 1 ou 2 et
X² est phénylène, lequel est facultativement substitué par 1 à 4 substituants choisis parmi halogène, cyano et alkyle en C₁ à C₄ ;
et les sels de ceux-ci.

10. Composés selon l'une quelconque des revendications précédentes pour utilisation en tant que médicaments.

11. Composition pharmaceutique comprenant au moins un composé selon l'une quelconque des revendications précédentes, laquelle comprend facultativement au moins un autre composant choisi parmi agents auxiliaires, excipients, solvants et/ou au moins un agent actif sur le plan pharmaceutique supplémentaire.

12. Composition pharmaceutique selon la revendication 11 pour utilisation en tant que médicament pour application sous-cutanée ou orale.

13. Composés selon l'une quelconque des revendications 1 à 9 ou composition pharmaceutique selon les revendications 11 ou 12 pour utilisation dans la lutte contre des parasites chez des animaux.

14. Procédé permettant de préparer les composés selon l'une quelconque des revendications 1 à 9, comprenant l'étape de réaction d'un composé (A) 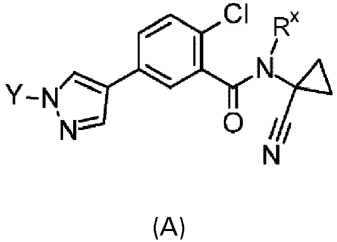
avec un groupe (B) 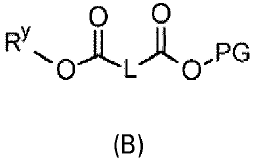
dans lequel
R^{x} représente hydrogène et R^{y} représente un groupe -CH₂-C1 ou
R^{x} représente un groupe -CH₂-OH et R^{y} représente hydrogène ;
dans lequel
Y et L ont la signification telle que définie dans l'une quelconque des revendications précédentes et PG représente un groupe de protection ou hydrogène pour former les composés selon la formule (I),
et dans lequel dans des cas où PG n'est pas hydrogène, une déprotection est effectuée pour former les composés (I).

15. Procédé selon la revendication 14, comprenant en outre
dans le cas où R^{x} représente hydrogène et R^{y} représente un groupe - CH₂-Cl, l'étape préliminaire de préparation du groupe (B) avec R^{y} = -CH₂-Cl (composé (B-2)) à partir d'un composé (B-1) 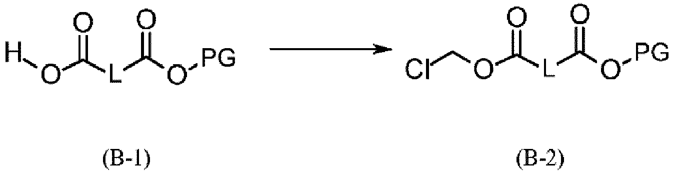 ou
dans le cas où R^{x} représente un groupe -CH₂-OH et R^{y} représente hydrogène, l'étape préliminaire de préparation du groupe (A) avec R^{x} = -CH₂-OH (composé A-3)) à partir d'un composé (A-1) par le biais d'un composé intermédiaire (A-2)

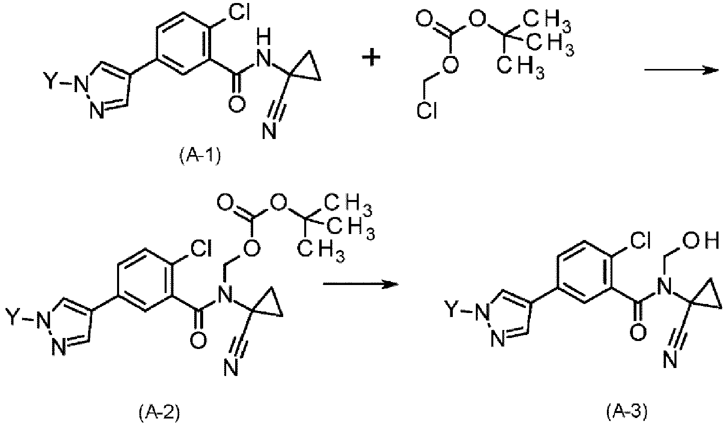

16. Composés intermédiaires selon la formule (C) 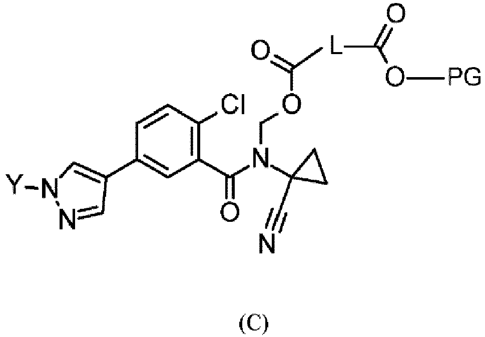
dans laquelle Y et L ont la signification telle que définie dans l'une quelconque des revendications précédentes et dans laquelle PG représente un groupe tert-butyle ;
ou selon la formule (A-3) 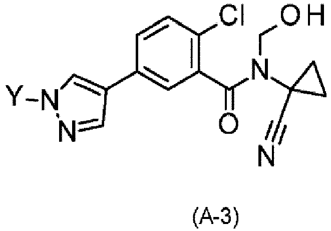
dans laquelle Y a la signification telle que définie dans l'une quelconque des revendications précédentes.
